# EUROPEAN PATENT APPLICATION

(11) **EP 2 765 227 A2**
(43) Date of publication of application: **13.08.2014**
(21) Application number: 14000228.8
(22) Date of filing: 06.04.2011
(51) Int. Cl.: C40B 30/04, C12Q 1/68

(54) **Compositions and methods for identifying autism spectrum disorders**

(30) Priority: 06.04.2010 US 321172 P
(62) Divisional of application: 11766672.7
(71) Applicant: The George Washington University, Washington, DC 20037 (US)
(72) Inventor: Wu, Valerie, Wailin, Rockville, MD 20855 (US)
(74) Representative: Schüssler, Andrea

(57) **Abstract**

The invention provides methods of identifying DNA methylation profiles for neurological and psychiatric conditions including autism spectrum disorders, methods of treating such conditions, and methods of identifying therapeutics for the treatment of such neurological and psychiatric conditions.

## Description

### FIELD OF THE INVENTION

This invention relates to DNA methylation microarray technology, and more specifically to methods and kits for identifying autism and autism spectrum disorders in humans.

### BACKGROUND OF THE INVENTION

Autism spectrum disorders (ASD) collectively represent a heterogeneous range of pervasive neurodevelopmental disorders that are characterized by deficits in social interactions and understanding, aberrant communication and/or language development, and restricted interests and stereotyped behaviors (Volkmar, F. R. et al (1994) Am J Psychiatry 151, 1361-71; Siegel, B. (1991) Psychiatr Clin North Am 14, 53-68). It is believed that multiple etiologies contribute to the heterogeneity in clinical presentation of ASD and further complicate efforts to identify genetic/epigenetic or molecular markers of the disease.

While autism is largely considered genetic in origin due to its high heritability observed in twin and family studies (Bailey, A. et al. (1995) Psychol Med 25, 63-77; Folstein, S., and Rutter, M. (1977) J Child Psychol Psychiatry 18, 297-321), recent evidence supports the involvement of epigenetic regulatory mechanisms in the pathogenesis of ASD (Schanen, N. C. (2006) Hum Mol Genet 15 Spec No 2, R138-50; Beaudet, A. L. (2007) Nature Medicine 13, 534-536; Nakayama, A. et al (2006) Nihon Shinkei Seishin Yakurigaku Zasshi 26, 209-12). These regulatory mechanisms include DNA methylation at CpG sites, genomic imprinting, chromatin modifications, and non-coding RNA (Mehler, M. F., and Mattick, J. S. (2006) J Physiol 575, 333-41; Champagne, F. A. (2008) Frontiers in Neuroendocrinology 29, 386-397; Chuang, J. C., and Jones, P. A. (2007) Pediatr Res 61, 24R-29R; van Vliet, J., Oates, N. A., and Whitelaw, E. (2007) Cell Mol Life Sci 64, 1531-8; Kaminsky, Z. et al. (2006) Ann Med 38, 530-44; Lopez-Rangel, E., and Lewis, M. E. (2006) Clin Genet 69, 21-2). Two single-gene disorders that may be associated with autistic symptoms, Fragile X Syndrome and Rett's Syndrome, arise from epigenetic dysregulation. Fragile X syndrome results from an expansion of the trinucleotide CGG repeat in the 5'UTR region of the FMR1 gene, leading to increased susceptibility to methylation and epigenetic silencing of FMR1 (Feng, Y. et al. (1995) Science 268, 731-4; Li, Z. et al., (2001) Nucleic Acids Res 29, 2276-83), and Rett's Syndrome arises from a mutation in the methyl CpG binding protein 2 (MeCP2) gene, whose protein product is responsible for recognizing methylated genes and is one of the key mediators of epigenetic regulation (Van Den Veyver, I. B., and Zoghbi, H. Y. (2001) Brain and Development 23, S147-S151; Amir, R. E. et al. (1999) Nat Genet 23, 185-8).

Hu *et al*. recently demonstrated differential gene expression in lymphoblastoid cell lines (LCL) from monozygotic twins discordant for diagnosis of autism (Hu, V. et al. (2006) BMC Genomics 7, 118), which strongly suggests that epigenetic factors are also involved in *idiopathic* autism. Other studies have suggested that "epigenetic hotspots" or regions susceptible to genomic imprinting are located in chromosomal regions (e.g., 15q and 7q) identified in genetic linkage analysis of autism (Schanen, N. C. (2006) Hum Mol Genet 15 Spec No 2, R138-50; Davies, W. et al. (2001) Ann Med 33, 428-36). Hogart et al. (Hogart, A. et al. (2007) Hum Mol Genet 16, 691-703) argues that genes located close to these hotspots (like genes encoding for GABAA-receptor subunits, GABRB3, GABRA5 and GABRG3), while not necessarily subject to imprinting, can still convey an ASD risk upon disrupted epigenetic regulation.

Methylation of cytosine residues of CpG sites is the most characterized of the epigenetic mechanisms and involves the addition of a methyl group onto the 5' position of a cytosine residue. While 60-90% of CpGs are found methylated throughout the genome (Klose and Bird, A. P. (2006) Trends in Biochemical Sciences 31, 89-97) unmethylated CpG sites can be found clustered in CpG islands most often associated with 5' promoter regions of genes. Methylation of CpG islands is most often associated with transcriptional silencing and has been found to be a significant contributor to altered gene expression. The mechanism of methylation as a silencing signal is thought to occur by either recruitment of repressive transcriptional silencing machinery, or by steric hindrance preventing the binding of transcription factors necessary for transcriptional activation.

Thus, there is a need for compositions and methods that will provide an increased understanding of the pathophysiology of Autism spectrum disorders, such as autism, pervasive developmental disorders not otherwise specified (PDD-NOS), and Asperger's syndrome, and their treatment.

The present invention satisfied these and other needs by demonstrating herein that one of the regulatory mechanisms responsible for altered gene expression in ASD is altered methylation status of genes, that is, differential methylation of genes relative to that of control samples from non-autistic individuals. The present invention provides compositions and methods for DNA methylation differential gene expression profiling and reveals significantly differentially DNA-methylated genes that are associated with neurological diseases, nervous system development and function, as well as other co-morbid disorders associated with ASD, such as gastrointestinal, muscular, and inflammatory disorders.

### SUMMARY OF THE INVENTION

One aspect of the invention provides an array, e.g., a gene chip array or focussed array, having a plurality of different oligonucleotides with specificity for differentially methylated DNA gene promoter regions, or CpG islands (regions of DNA containing relatively high content of CpG dinucleotides), of genes that are associated with at least one autism spectrum disorder, wherein the autism spectrum disorder comprises autistic disorder, pervasive developmental disorder-not otherwise specified (PDD-NOS), including atypical autism, Asperger's Disorder, or a combination thereof.

In one embodiment of the present invention, an oligonucleotide array, e.g., gene chip array, is provided wherein the oligonucleotides are specific for at least one of the genes set out in Table 1, Table 2, or Table 3, or a combination thereof. The oligonucleotides are preferably specific for unmethylated or methylated regions in the gene(s) or for unmethylated or methylated CpG islands of the promoter(s) of the gene(s).

In another aspect of the invention, a method of screening a subject for a neurological disease or disorder comprising the steps of: (a) isolating a sample of nucleic acid, protein or cellular extract from at least one cell from the subject; (b) measuring the level of DNA methylation of at least one gene in Table 1, Table 2, Table 3 or a combination thereof in a sample, wherein the at least one gene has been determined to have differential DNA methylation pattern in subjects with a neurological disease or disorder as compared to a control sample from individuals without the neurological condition, wherein the subject is diagnosed to be at risk for or affected by a neurological disease or disorder if there is a statistically significant difference in the level of DNA methylation of at least one gene listed in Table 1, Table 2, or Table 3 compared to the level of DNA methylation of the same genes from a sample from healthy individuals without the neurological condition.

In one embodiment of the screening method of the present invention, the neurological disease comprises at least one autism spectrum disorder, autistic disorder, pervasive developmental disorder-not otherwise specified (PDD-NOS) including atypical autism, Asperger's Disorder, or a combination thereof.

In another embodiment of the screening method of the present invention, the at least one differentially DNA methylated gene in Table 1, Table 2, Table 3 or a combination thereof comprise genes involved in nervous system development, axon guidance, synaptic transmission or plasticity, myelination, long-term potentiation, neuron toxicity, Purkinje cell differentiation, cerebellar development, embryonic development, regulation of actin networks, digestion, inflammation, oxidative stress, epilepsy, apoptosis, morphogenesis, cell survival, differentiation, the unfolded protein response, Type II diabetes and insulin signaling, digestion, liver toxicity (hepatic stellate cell activation, fibrosis, and cholestasis), endocrine function, circadian rhythm, cholesterol metabolism and the steroidogenesis pathway, or a combination thereof.

In yet another embodiment of the screening method of the present invention, the healthy individual without the neurological condition is a non-phenotypic discordant twin, sibling of the subject, or healthy, unrelated individual.

In yet another embodiment of the screening method of the present invention, the method distinguishes between different variants of autism spectrum disorder comprising a lower severity scores across all ADIR items, an intermediate severity across all ADIR items, a higher severity scores on spoken language items on the ADIR, a higher frequency of savant skills, and a severe language impairment, or a combination thereof.

In yet another embodiment of the screening method of the present invention, the level of DNA methylation is quantified with an assay comprising bisulfite-sequencing, methylation-specific PCR (MSP), large scale CpG island microarray analysis using methylation-enriched and -unenriched samples, promoter analysis using methylation-enriched and -unenriched samples, combined bisulfite and restriction analysis (COBRA), pyrosequncing, Methyl-Light (Premier Biosoft International) or Methyl-Profiler (SABiosciences) methylation analysis, Sequenom mass analyzer, bisulfite treatment of DNA combined with selected amplification of the specific promoter regions of differentially methylated genes with the addition of a T7 promoter tag, followed by T7-mediated transcription and RNase T1 cleavage of the transcript with methylation sites determined by MALDI-- TOF mass spectrometry analysis in the manner of Schatz et al. (2004) Nucleic Acids Res. 32(21): e167, or a combination thereof.

In yet another aspect of the invention, a method is provided for determining a differential DNA methylation profile for at least one autism spectrum disorder, comprising (a) preparing samples of control and experimental DNA, wherein the experimental DNA is generated from a nucleic acid sample isolated from a subject suspected of being afflicted with the at least one autism spectrum disorder and the control DNA is generated from a nucleic acid sample isolated from a healthy individual; (b) preparing one or more microarrays comprising a plurality of different oligonucleotides having specificity for differentially methylated genes associated with the at least one autism spectrum disorder; (c) applying the prepared samples to the one or more microarrays to allow hybridization between the oligonucleotides and the control DNA and the oligonucleotide and the experimental DNA ; (d) identifying the oligonucleotides on the microarray which display differential hybridization to the experimental DNA relative to the control DNA thereby determining a differential DNA methylation profile for the at least one autism spectrum disorder.

In one embodiment of the differential DNA methylation profiling method of the present invention, the plurality of different oligonucleotides is specific for at least one differentially methylated gene in Table 1, Table 2, Table 3 or a combination thereof.

In another embodiment of the differential DNA methylation profiling method of the present invention, the at least one autism spectrum disorder comprises autistic disorder, pervasive developmental disorder-not otherwise specified (PDD-NOS), including atypical autism, Asperger's Disorder, or a combination thereof.

In yet another aspect of the invention, a method is provided for distinguishing between different phenotypes of an autism spectrum disorder comprising severely language impaired (L), mildly affected (M), or "savants" (S) comprising (a) preparing samples of control and experimental DNA , wherein the experimental DNA is generated from a nucleic acid sample isolated from a subject suspected of being afflicted with at least one phenotype comprising the severely language impaired (L), mildly affected (M), or "savants" (S); (b) preparing one or more microarrays comprising a plurality of different oligonucleotides having specificity for differentially methylated genes associated with the at least one phenotype; (c) applying the prepared samples to the one or more microarrays to allow hybridization between the oligonucleotides and the control and experimental DNA s; (d) identifying the oligonucleotides on the microarray which display differential hybridization to the experimental DNA relative to the control DNA thereby determining a DNA methylation profile for distinguishing among the different phenotypes of autism spectrum disorder.

In another embodiment of the phenotype distinguishing method of the present invention, the plurality of different oligonucleotides is specific for at least one differentially methylated gene in Table 1, Table 2, Table 3 or a combination thereof.

In yet another embodiment of the phenotype distinguishing method of the present invention, the at least one autism spectrum disorder comprises autistic disorder, pervasive developmental disorder-not otherwise specified (PDD-NOS), including atypical autism, Asperger's Disorder, or a combination thereof.

In yet another aspect of the invention, a method is provided for predicting efficacy of a test compound for altering a behavioral response in a subject with at least one autism spectrum disorder comprising: (a) preparing a microarray comprising a plurality of different oligonucleotides, wherein the oligonucleotides have specificity for differentially DNA-methylated gene promoter regions or CpG islands associated with at least one autism spectrum disorder; (b) obtaining a differential DNA-methylation profile representative of the differential DNA methylation profile of at least one sample of a selected tissue type from a subject subjected to each of at least one of a plurality of selected behavioral therapies which promote the behavioral response; (c) administering the test compound to the subject; and (d) comparing a differential DNA methylation profile data in at least one sample of the selected tissue type from the subject treated with the test compound to determine a degree of similarity with one or more differential DNA methylation profile associated with an autism spectrum disorder; wherein the predicted efficacy of the test compound for altering the behavioral response is correlated to said degree of similarity.

In another embodiment of the compound efficacy testing method of the present invention, the plurality of oligonucleotides is specific for at least one differentially methylated gene in Table 1, Table 2, Table 3 or a combination thereof. The oligonucleotides may be specific for, e.g., (i) the promoter region of the gene, or CpG island(s) of the gene, as methylated in healthy individuals without a neurological disorder, e.g., an autism spectrum disorder, or (ii) specific for the promoter region of the genes or CpG island(s) as methylated in a subject having a neurological disorder, e.g., an autism spectrum disorder, or a combination of both (i) and (ii).

In yet another embodiment of the compound efficacy testing method of the present invention, the autism spectrum disorder neurological condition comprises autistic disorder, pervasive developmental disorder-not otherwise specified (PDD-NOS), including atypical autism, Asperger's Disorder, or a combination thereof.

In yet another embodiment of the compound efficacy testing method of the present invention, step (a) comprises obtaining a differential DNA methylation profile representative of the differential DNA methylation profile of at least two samples of a selected tissue type.

In yet another embodiment of the compound efficacy testing method of the present invention, the selected tissue type comprises a neuronal tissue type.

In yet another embodiment of the compound efficacy testing method of the present invention, the neuronal tissue type is selected from the group consisting of olfactory bulb cells, cerebrospinal fluid, hypothalamus, amygdala, pituitary, nervous system, brainstem, cerebellum, cortex, frontal cortex, hippocampus, striatum, and thalamus.

In yet another embodiment of the compound efficacy testing method of the present invention, the selected tissue type is selected from the group consisting of lymphocytes, blood, or mucosal epithelial cells, brain, spinal cord, heart, arteries, esophagus, stomach, small intestine, large intestine, liver, pancreas, lungs, kidney, urinary tract, ovaries, breasts, uterus, testis, penis, colon, prostate, bone, muscle, cartilage, thyroid gland, adrenal gland, pituitary, bone marrow, blood, thymus, spleen, lymph nodes, skin, eye, ear, nose, teeth or tongue.

In yet another embodiment of the compound efficacy testing method of the present invention, the test compound is an antibody, a nucleic acid molecule, a small molecule drug, or a nutritional or herbal supplement.

In yet another embodiment of the compound efficacy testing method of the present invention, the behavioral therapy comprises applied behavior analysis (ABA) intervention methods, dietary changes, exercise, massage therapy, group therapy, talk therapy, play therapy, conditioning, or alternative therapies such as sensory integration and auditory integration therapies.

In yet another aspect of the invention a method is provided for assessing the efficacy of a treatment in an individual having at least one autism spectrum disorder comprising (a) determining the level of DNA methylation of at least one differentially DNA-methylated genes in Table 1, Table 2, Table 3 or a combination thereof in the sample, or a combination thereof, in a plurality of patient samples of a selected tissue type; (b) determining a degree of similarity between (a) the level of DNA methylation of at least one differentially DNA-methylated genes in Table 1, Table 2, Table 3 or a combination thereof in the sample profile data in the patient samples; and (b) the level of DNA methylation of at least one differentially DNA-methylated genes in Table 1, Table 2, Table 3 or a combination thereof in the sample profile specific for the genes set out in listed in Table 1, Table 2, Table 3, , or a combination thereof, produced by a therapy which has been shown to be efficacious in treatment of the at least one autism spectrum disorder; wherein a high degree of similarity of the differential methylation profile data is indicative that the treatment is effective.

In yet another embodiment of the invention a method is provided for assessing the efficacy of a treatment in an individual having at least one autism spectrum disorder comprising (a) determining a DNA methylation profile data specific for at least one differentially DNA-methylated genes set out in Table 1, Table 2, Table 3 or a combination thereof, in a plurality of patient samples of a selected tissue type; (b) determining a degree of similarity between (a) the DNA methylation profile data in the patient samples; and (b) a DNA methylation profile specific for the genes set out in listed in Table 1, Table 2, Table 3 or a combination thereof, produced by a therapy which has been shown to be efficacious in treatment of the at least one autism spectrum disorder; wherein a high degree of similarity of the DNA methylation profile data is indicative that the treatment is effective.

In yet another aspect of the invention, a method is provided for determining a DNA methylation profile indicative of administration of a therapeutic treatment to a subject with at least one autism spectrum disorder comprising (a) preparing samples of control and experimental nucleic acid, wherein the experimental nucleic acid is generated from a nucleic acid sample isolated from a subject who has received the therapeutic treatment; (b) preparing one or more microarrays comprising a plurality of different oligonucleotides, wherein the oligonucleotides are specific for genes associated with an autism spectrum disorder; (c) applying the prepared samples to the one or more microarrays to allow hybridization between the oligonucleotides and the control and experimental nucleic acids; (d) identifying the oligonucleotides on the microarray which display differential hybridization to the experimental nucleic acid relative to the control nucleic acid thereby determining a differential DNA methylation profile indicative for the administration of the therapeutic treatment to the subject with at least one autism spectrum disorder.

In one embodiment of the method of the present invention, the plurality of different oligonucleotides is specific for at least one differentially methylated gene in Table 1, Table 2, Table 3, or a combination thereof.

In another embodiment of the method of the present invention, the at least one autism spectrum disorder neurological condition comprises autistic disorder, pervasive developmental disorder-not otherwise specified (PDD-NOS), including atypical autism, Asperger's Disorder, or a combination thereof.

In yet another aspect of the invention, a method is provided for conducting drug discovery comprising (a) generating a database of DNA methylation profile data representative of the genetic expression response of at least one selected neuronal tissue type from a subject that was subjected to at least one of a plurality of behavioral therapies and that has undergone a selected physiological change since commencement of the behavioral therapy; (b) administering small molecule test agents to untreated subjects to obtain DNA methylation profile data associated with administration of the agents and comparing the obtained data with the one or more selected DNA methylation profiles; (c) selecting test agents that induce DNA methylation profiles similar to DNA methylation profiles obtainable by administration of behavioral therapy; (d) conducting therapeutic profiling of the selected test compound(s), or analogs thereof, for efficacy and toxicity in subjects; and (e) identifying a pharmaceutical preparation including one or more agents identified in step (d) as having an acceptable therapeutic and/or toxicity profile.

In another embodiment of the method of the present invention, the behavioral therapy comprises applied behavior analysis (ABA) intervention methods, dietary changes, exercise, massage therapy, group therapy, talk therapy, play therapy, conditioning, or alternative therapies such as sensory integration and auditory integration therapies.

In yet another embodiment of the method of the present invention, the selected physiological change includes one or more improvements in social interaction, language abilities, restricted interests, repetitive behaviors, sleep disorders, seizures, gastrointestinal, hepatic, and mitochondrial function, neural inflammation, or a combination thereof.

In yet another embodiment of the method of the present invention, prior to administration of behavioral therapy, the subject shows at least one symptom of a psychological or physiological abnormality.

In yet another embodiment of the method of the present invention, the neuronal tissue type is selected from the group consisting of olfactory bulb cells, cerebrospinal fluid, hypothalamus, amygdala, pituitary, nervous system, brainstem, cerebellum, cortex, frontal cortex, hippocampus, striatum, and thalamus.

In one embodiment of the compositions of the present invention, the gene chip array having a plurality of different oligonucleotides with specificity for the differentially DNA-methylated gene promoter regions or CpG islands of retinoic acid receptor-related orphan receptor alpha (RORA), B cell CLL/lymphoma 2 gene (BCL-2), or a combination thereof

In one embodiment of the compositions of the present invention, the gene chip array having a plurality of different oligonucleotides with specificity for the differentially DNA-methylated gene promoter regions or CpG islands one or more genes of Table 1, Table 2, Table 3, or a combination thereof, and specifically excludes a plurality of oligonucleotides with specificity the differentially DNA-methylated MeCP₂ gene and the oxytocin receptor gene, or a combination thereof

In one embodiment of each of the methods of the present invention, the differentially methylated gene comprises RORA *or BCL-2,* or a combination thereof.

In one embodiment of each of the methods of the present invention, the differentially methylated gene specifically excludes the differentially DNA-methylated MeCP2 gene and the oxytocin receptor gene, or a combination thereof.

In yet another aspect of the invention, a kit is provided for identifying a compound for treating at least one autism spectrum disorder comprising (a) a database having information stored therein one or more DNA methylation profiles specific for the genes set out in listed in Table 1, Table 2, Table 3 or a combination thereof, of subjects that have been subjected to at least one of a plurality of selected autism spectrum disorder neurological therapies and wherein the subject has undergone a desired physiological change; and (b) a computer program for comparing DNA methylation profile data obtained from assays wherein a test compound is administered to a subject with the database and providing information representative of a measure of similarity between the DNA methylation profile data and one or more stored DNA methylation profiles.

In yet another aspect of the invention, a computer-readable medium on which is encoded programming code for analyzing autism spectrum disorder from a plurality of data points comprising a profile of differentially expressed DNA methylation genes, wherein said differential DNA methylation profile is specific for at least one differentially DNA methylated gene set out in Table 1, Table 2, or a combination thereof.

In yet another embodiment of the invention, in each of the screening methods, DNA methylation profiling methods, phenotype distinguishing methods, drug discovery methods, compound efficacy testing methods, computer program for determining a DNA methylation profile, and kits specifically provided for *supra* (and infra) may also be, without any limitation, made and/or practiced with at least two to at least fifty, or any integer value thereof, different differentially DNA methylated genes set out in Table 1, Table 2, Table 3, or a combination thereof.

### BRIEF DESCRIPTION OF THE DRAWINGS

The foregoing and other aspects and advantages of the invention will be appreciated more fully from the following further description thereof, with reference to the accompanying drawings wherein:

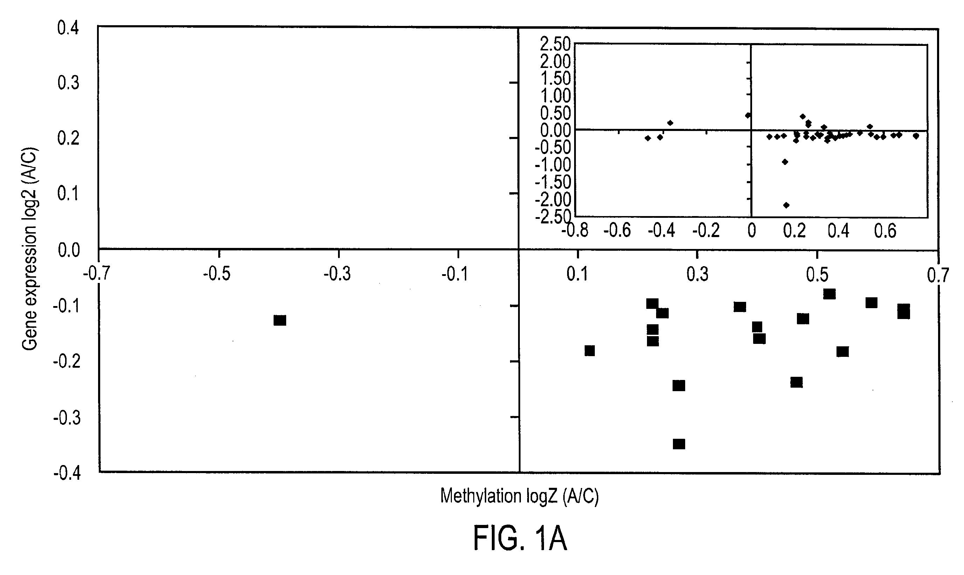

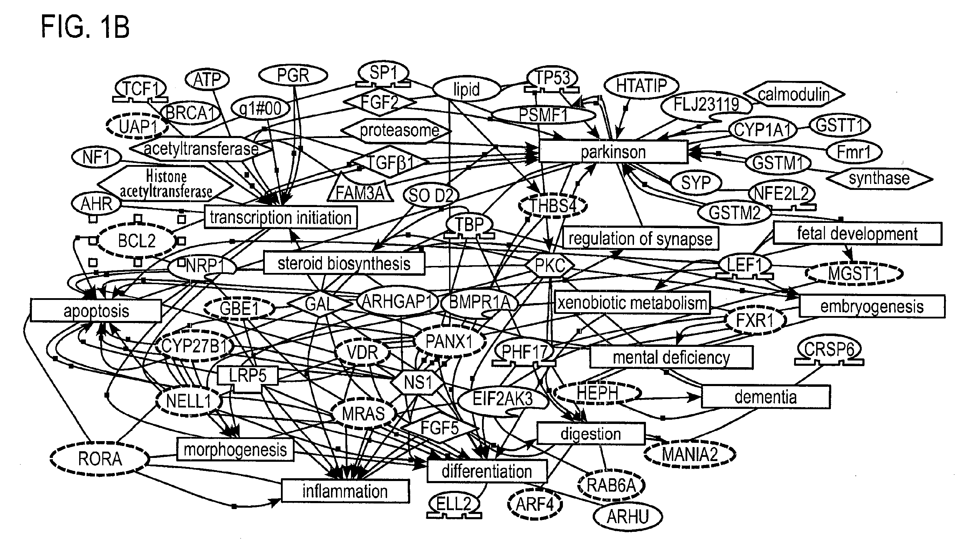

Figure 1 depicts the overlap of dysregulated genes identified from CpG island microarray and global gene expression profiling previously performed using the same samples. A) Scatter plot shows inverse correlation of log2 ratios of gene expression and methylation of genes with differentially methylated CpG islands directly overlapping with its 5' end. The inset graph plots log2 ratios of genes with differentially methylated CpG islands located either upstream or downstream of the CpG island. Interestingly, the majority of genes identified from these analyses were hypermethylated in the twins relative to their respective nonautistic siblings, with a corresponding decrease in gene expression. B) Network analysis of 25 genes (circumscribed in dashed line) that were both differentially methylated and expressed. The network was generated using Pathway Studio 5 network prediction software (Ariadne Genomics, Rockville, MD) and identified common biological themes including apoptosis, cellular differentiation and inflammation. The analysis also revealed neurologically relevant functions and disorders including synaptic regulation, development, and mental deficiency.

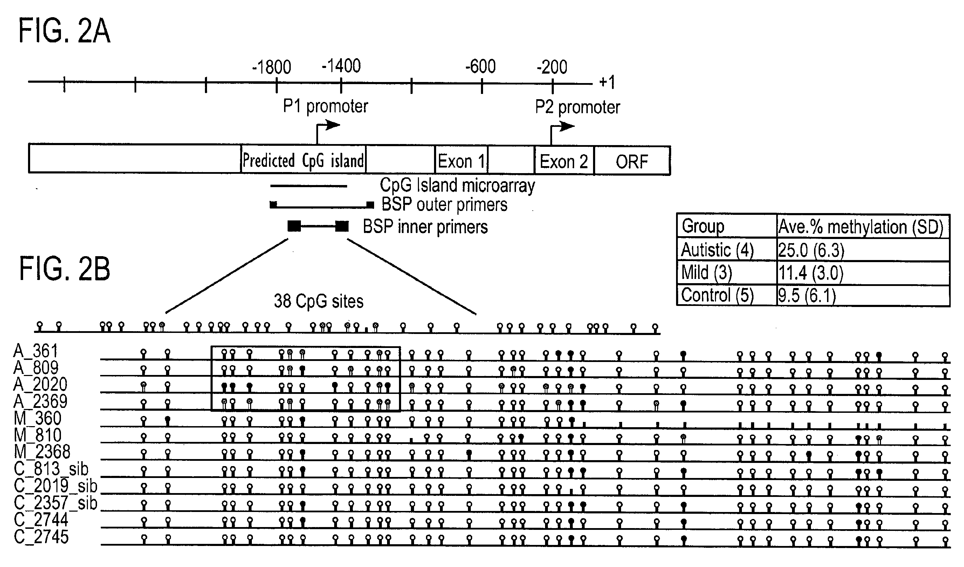

Figure 2 depicts bisulfite sequencing of the BCL-2 PI promoter region (shown in A). Bisulfite sequencing was performed across 38 CpG sites from genomic DNA isolated from LCL of discordant MZ twins (B). The autistic co-twins are designated by "A_" preceding the blood identifier number (e.g., A_809), and the undiagnosed co-twins are designated by "M_" preceding the blood number (e.g., M_810). Control individuals (C) include unaffected siblings (e.g., C_813), one normal monozygotic twin pair (C_2744 and C_2745). A pair of siblings, one autistic (A_2020) and one unaffected (C_2019), was also included in this analysis. Inset table shows average % methylation with standard deviation (SD) across all 38 CpG sites for each group of samples.

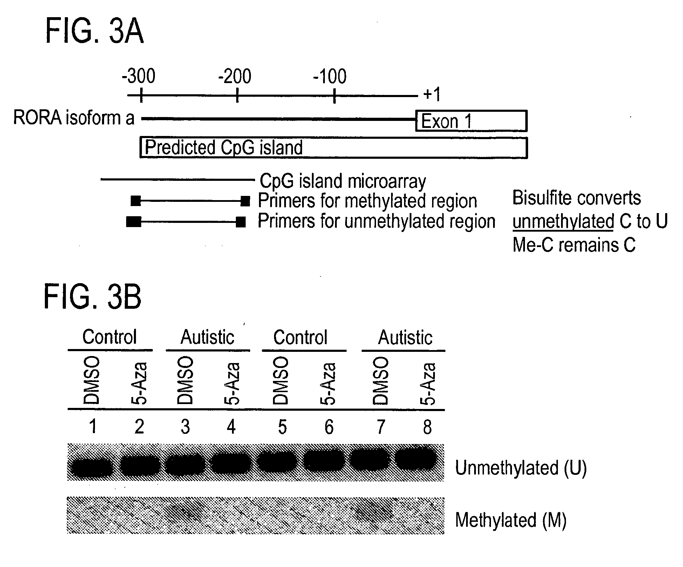

Figure 3 depicts representative image of methylation specific-PCR (MSP) of the upstream CpG island region of RORA (shown in A) in LCL from autistic (A_737 and A_2020) and unaffected control (C_735 and C_2019) siblings. LCLs were treated with DMSO (vehicle control) or 5uM of 5-Aza-2-deoxycytidine for 48 hours. Genomic DNA was isolated and bisulfite modified. B) Modified DNA was used for PCR containing primers specific for unmethylated (U) and methylated (M) CpG sites of RORA.

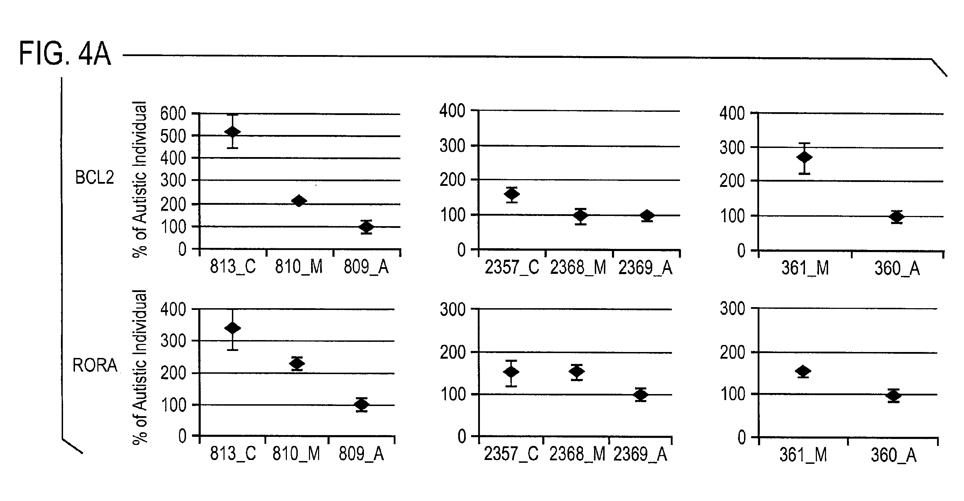

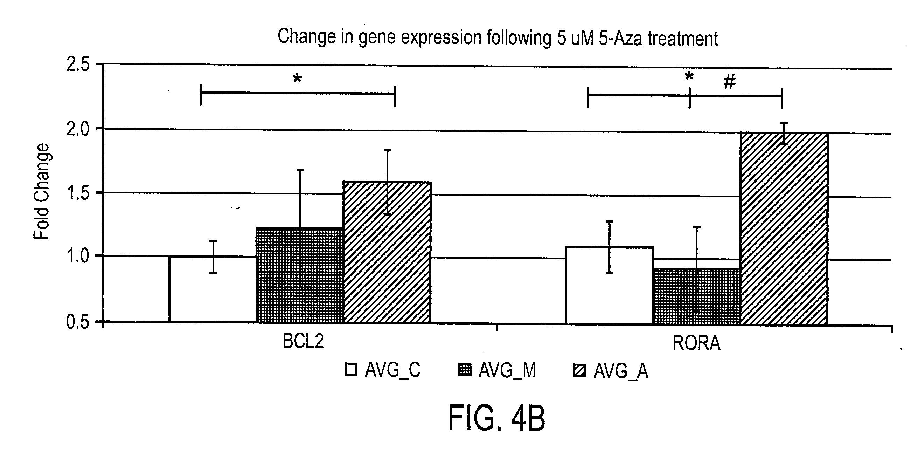

Figure 4 depicts qRT-PCR of BCL-2 and RORA transcripts from LCL of 3 pairs of discordant twins and respective sibling controls for 2 pairs of twins. (A) Results are shown as percent of gene expression of autistic co-twin (#_A). (B) RNA isolated from LCLs following 48hr treatment with 5uM 5-Aza-2-deoxycytidine was also analyzed by qRT-PCR. Results shown are the average fold-change ± SE in expression following treatment.

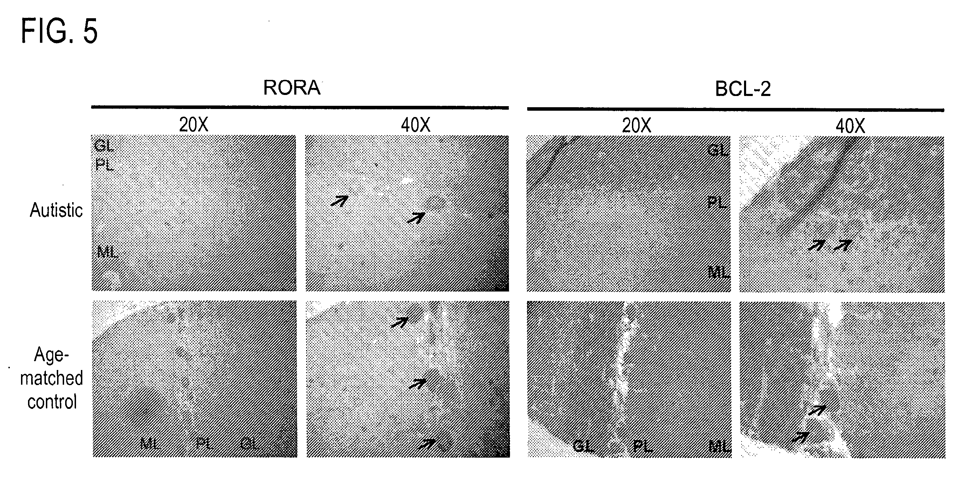

Figure 5 depicts higher magnification (40x) images of immunohistochemical staining for RORA and BCL-2 on cerebellar sections from autistic and age-matched control subjects. ML: molecular layer; PL: Purkinje layer; GL: granule layer. Arrows point to Purkinje cells.

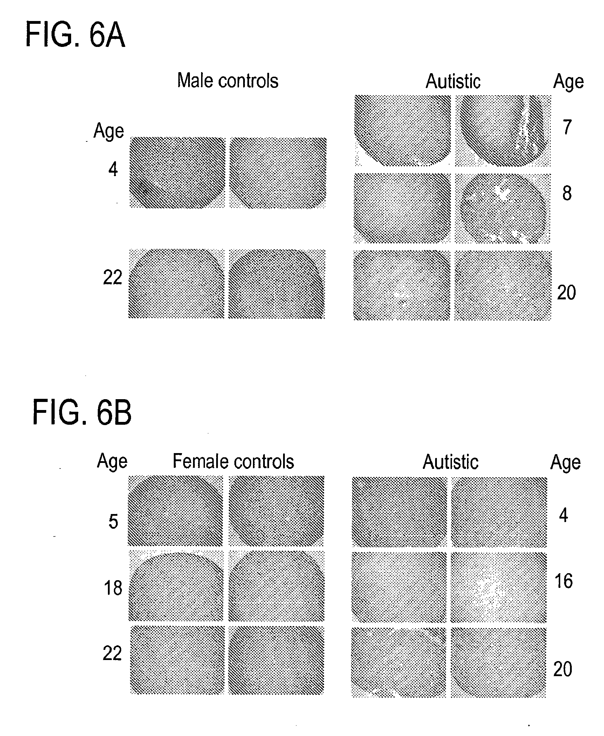

Figure 6 depicts RORA protein also appears to be reduced in the frontal cortex (BA9) in the majority of tissue sections from age- and sex-matched autistic and control subjects. This tissue array, obtained through the Autism Tissue Program, was prepared as described by Nagarajan et al. (2006) (Nagarajan, R. P. et al (2006) Epigenetics 1 172-182) in the laboratory of Dr. Janine LaSalle.

### DETAILED DESCRIPTION OF THE INVENTION

The invention disclosed herein provides methods and compositions for diagnosis and treatment of neurological conditions. In particular, the invention provides microarray technology to diagnose and treat autism spectrum disorders. The invention relates, in part, to sets of genetic markers whose DNA methylation patterns correlate with therapeutic treatments of neurological, and in particular, autism spectrum disorders.

The invention provides not only methods of identifying differentially methylated gene profiles for neurological conditions, but also methods of using such differentially methylated gene profiles in order to select particular therapeutic compounds useful in the prevention and treatment of such neurological conditions. The invention further relates to the application of differentially methylated gene profiles for the identification of therapeutic targets, and related pharmaceutical methods and kits.

The systems and methods described herein include microarray systems including gene chips and arrays of nucleotide sequences for detecting differentially methylated gene profiles of neurological conditions, and in particular, autism spectrum disorder conditions. The systems and methods described herein provide microarrays that have a plurality of oligonucleotide primers immobilized thereon and have specificity for differentially methylated genes associated with neurological conditions, and in particular, autism spectrum disorder conditions.

To provide an overall understanding of the invention, certain illustrative embodiments will now be described. However, it will be understood by one of ordinary skill in the art that the systems and methods described herein can be adapted and modified for other suitable applications and that such other additions and modifications will not depart from the scope hereof.

### Definitions

For convenience, certain terms employed in the specification, examples, and appended claims, are collected here. Unless defined otherwise, all technical and scientific terms used herein have the same meaning as commonly understood by one of ordinary skill in the art to which this invention belongs.

The articles "a" and "an" are used herein to refer to one or to more than one (i.e., to at least one) of the grammatical object of the article. By way of example, "an element" means one element or more than one element.

The term "including" is used herein to mean, and is used interchangeably with, the phrase "including but not limited to".

The term "or" is used herein to mean, and is used interchangeably with, the term "and/or," unless context clearly indicates otherwise.

The term "such as" is used herein to mean, and is used interchangeably, with the phrase "such as but not limited to".

A "patient" or "subject" to be treated by the method of the invention can mean either a human or non-human animal, preferably a mammal.

The term "encoding" comprises an RNA product resulting from transcription of a DNA molecule, a protein resulting from the translation of an RNA molecule, or a protein resulting from the transcription of a DNA molecule and the subsequent translation of the RNA product.

The term "expression" is used herein to mean the process by which a polypeptide is produced from DNA. The process involves the transcription of the gene into mRNA and the translation of this mRNA into a polypeptide. Depending on the context in which used, "expression" may refer to the production of RNA, protein or both.

The term "transcriptional regulator" refers to a biochemical element that acts to prevent or inhibit the transcription of a promoter-driven DNA sequence under certain environmental conditions (e.g., a repressor or nuclear inhibitory protein), or to permit or stimulate the transcription of the promoter-driven DNA sequence under certain environmental conditions (e.g., an inducer or an enhancer).

The terms "array", "microarray", "GeneChip", "genome chip", and "biochip", as used herein refer to an ordered arrangement of hybridizable array elements. The array elements are arranged so that there are preferably at least one or more different array elements on a substrate surface, such as paper, nylon or other type of membrane, filter, chip, glass slide, or any other suitable solid support. The hybridization signal from each of the array elements is individually distinguishable.

The terms "complementary" or "complementarity" as used herein refer to polynucleotides (i.e., a sequence of nucleotides) related by the base-pairing rules. For example, for the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods which depend upon binding between nucleic acids.

As used herein, the term "hybridization" is used in reference to the pairing of complementary nucleic acids. Hybridization and the strength of hybridization (i.e., the strength of the association between the nucleic acids) is impacted by such factors as the degree of complementary between the nucleic acids, stringency of the conditions involved, the Tm of the formed hybrid, and the G:C ratio within the nucleic acids.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (i.e., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxy ribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (i.e., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein, the terms "compound" and "test compound" refer to any chemical entity, pharmaceutical, drug, and the like that can be used to treat or prevent a disease, illness, conditions, or disorder of bodily function. Compounds comprise both known and potential therapeutic compounds. A compound can be determined to be therapeutic by screening using the screening methods of the present invention. A "known therapeutic compound" refers to a therapeutic compound that has been shown (e.g., through animal trials or prior experience with administration to humans) to be effective in such treatment. In other words, a known therapeutic compound is not limited to a compound efficacious in the treatment of cancer. Examples of test compounds include, but are not limited to peptides, polypeptides, synthetic organic molecules, naturally occurring organic molecules, nucleic acid molecules, and combinations thereof.

A "sample" may include a single cell or multiple cells or fragments of cells or an aliquot of body fluid or a tissue, taken from a subject, by means including venipuncture, excretion, ejaculation, massage, biopsy, needle aspirate, lavage sample, scraping, surgical incision or intervention or other means known in the art.

As used herein, the term "subject" refers to an organism, e.g., a human or non-human animal, whether *in vivo, ex vivo* or *in vitro,* under observation.

As used herein, the term "increased expression" refers to the level of a gene expression product that is made higher and/or the activity of the gene expression product that is enhanced. Preferably, the increase is by at least 1.22-fold, 1.5-fold, more preferably the increase is at least 2-fold, 5-fold, or 10-fold, and most preferably, the increase is at least 20-fold, relative to a control.

As used herein, the term "decreased expression" refers to the level of a gene expression product that is made lower and/or the activity of the gene expression product that is lowered. Preferably, the decrease is at least 25%, more preferably, the decrease is at least 50%, 60%, 70%, 80%, or 90% and most preferably, the decrease is at least one-fold, relative to a control.

As used herein, the term "gene profile" or "differentially methylated gene profile" refers to an experimentally verified subset of values associated with the methylation level of a set of genes. A differentially methylated gene profile may provide for identification of a biological condition or a physiological process.

As used herein, the term "differentially methylated gene expression profile," or "gene expression profile" refers to the level or amount of gene expression of particular genes, for example, informative genes, as assessed by methods described herein. The differentially methylated gene expression profile or gene expression profile can comprise data for one or more informative genes and can be measured at a single time point or over a period of time. For example, the differentially methylated gene expression profile or gene expression profile can be determined using a single informative gene, or it can be determined using two or more informative genes, three or more informative genes, five or more informative genes, ten or more informative genes, twenty-five or more informative genes, or fifty or more informative genes. A differentially methylated gene expression profile or gene expression profile may include expression levels of genes that are not informative, as well as informative genes. Phenotype classification (e.g., the presence or absence of a neurological disorder) can be made by comparing the differentially methylated gene expression profile or gene expression profile of the sample with respect to one or more informative genes with one or more differentially methylated gene expression profile or gene expression profiles (e.g., in a database). Using the methods described herein, expression of numerous genes can be measured simultaneously. The assessment of numerous genes provides for a more accurate evaluation of the sample because there are more genes that can assist in classifying the sample. A differentially methylated gene expression profile or gene expression profile may involve only those genes that are increased in expression in a sample, only those genes that are decreased in expression in a sample, or a combination of genes that are increased and decreased in expression in a sample.

The terms "disorders" and "diseases" are used inclusively and refer to any deviation from the normal structure or function of any part, organ or system of the body (or any combination thereof). A specific disease is manifested by characteristic symptoms and signs, including biological, chemical and physical changes, and is often associated with a variety of other factors including, but not limited to, demographic, environmental, employment, genetic and medically historical factors. Certain characteristic signs, symptoms, and related factors can be quantitated through a variety of methods to yield important diagnostic information.

The term "neurological condition" or "neurological disorder" is used herein to mean mental, emotional, or behavioral abnormalities. These include but are not limited to autism spectrum disorder conditions including autism, Asperger's Disorder, bipolar disorder I or II, schizophrenia, schizoaffective disorder, psychosis, depression, stimulant abuse, alcoholism, panic disorder, generalized anxiety disorder, attention deficit disorder, post-traumatic stress disorder, Parkinson's disease, or a combination thereof.

### Gene Chips

One aspect of the invention provides gene chips. Gene chips, also called "biochips" or "arrays" or "microarrays" are miniaturized devices typically with dimensions in the micrometer to millimeter range for performing chemical and biochemical reactions and are particularly suited for embodiments of the invention. Arrays may be constructed via microelectronic and/or microfabrication using essentially any and all techniques known and available in the semiconductor industry and/or in the biochemistry industry, provided that such techniques are amenable to and compatible with the deposition and screening of polynucleotide sequences. Microarrays are particularly desirable for their virtues of high sample throughput and low cost for generating profiles and other data.

One specific aspect of the invention provides a gene chip having a plurality of different oligonucleotides having specificity for DNA-methylated genes or fragments thereof associated with neurological conditions, and in particular, autism spectrum disorder conditions including pervasive developmental disorder-not otherwise specified (PDD-NOS), including atypical autism, Asperger's Disorder, or a combination thereof. In a related embodiment, the invention provides a gene chip having a plurality of different oligonucleotides having specificity for genes whose DNA methylation level changes in a subject who is afflicted with neurological conditions, and in particular, autism spectrum disorder conditions including pervasive developmental disorder-not otherwise specified (PDD-NOS), including atypical autism, Asperger's Disorder, or a combination thereof when the subject responds favorably to a therapeutic treatment that is intended to treat the neurological condition.

In one embodiment of the gene chips provided herein, the oligonucleotides on the gene chip comprise a plurality oligonucleotides that are specific for the differentially DNA-methylated genes, or promoter regions or CpG islands thereof, set out in Tables 1, 2, 3, or combinations thereof. In another embodiment, the gene chip has oligonucleotides specific for the genes associated with autism spectrum disorder conditions including pervasive developmental disorder-not otherwise specified (PDD-NOS), including atypical autism, Asperger's Disorder, or a combination thereof.

In another specific embodiment, the gene chip has at least one oligonucleotide specific for genes associated with the cellular response to androgens.

In another specific embodiment, the gene chip has at least one oligonucleotide specific for genes associated with circadian rhythm. In another specific embodiment, the gene chip has at least one oligonucleotide specific for the circadian rhythm associated genes, or any of the genes set out in Table 1, Table 2, or Table 3, or any combination thereof.

In another specific embodiment, the gene chip has at least one oligonucleotide specific for target genes associated with nervous system development, WNT signaling, axon guidance, regulation of the cytoskeleton, synaptic transmission or plasticity, myelination, long-term potentiation, neuron toxicity, Purkinje cell differentiation, cerebellar development, embryonic development, regulation of actin networks, digestion, inflammation, oxidative stress, epilepsy, apoptosis, morphogenesis, cell survival, differentiation, the unfolded protein response, Type II diabetes and insulin signaling, digestion, liver toxicity (hepatic stellate cell activation, fibrosis, and cholestasis), epilepsy, apoptosis, cell survival, differentiation, the unfolded protein response, endocrine function, circadian rhythm, cholesterol metabolism and the steroidogenesis pathway, or a combination thereof.

In another embodiment, the gene chip comprises oligonucleotide probes specific for genes associated with apoptosis and inflammation, as well as many neurological and metabolic processes commonly associated with ASD, such as myelination, neuron plasticity, synaptic transmission, and hypercholesterolemia. In one embodiment, the gene chip comprises oligonucleotides specific for BCL-2, RORA, or a combination thereof.

In another specific embodiment of the gene chips provided herein, the gene chip comprises at least 3, 5, 10, 15, 20 or 25 of the probes are derived from oligonucleotides that are specific for the differentially DNA-methylated genes set out in any one of Tables 1, 2, 3, or a combination thereof. In a related embodiment, at least 50% of the probes on the gene chip are derived from oligonucleotides that are specific for the differentially DNA-methylated genes present in any one of Tables 1, 2, 3, or a combination thereof. In a related embodiment, at least 70%, 80%, 90%, 95% or 98% of the probes on the gene chip are derived from oligonucleotides that are specific for the differentially DNA-methylated genes present in any one of Tables 1, 2, 3, or combinations thereof.

The invention further provides a gene chip for distinguishing cell samples from individuals having a positive prognosis and cell samples from individuals having a negative prognosis, wherein prognosis refers to the progression of disease or prognosis for successful treatment by a given treatment regimen or agent, comprising a positionally-addressable array of polynucleotide probes bound to a support, said polynucleotide probes comprising a plurality of polynucleotide probes of different nucleotide sequences, each of said different nucleotide sequences comprising a sequence complementary and specifically hybridizable to at least one of a plurality of differentially DNA-methylated genes, said plurality consisting of at least one of the differentially DNA-methylated genes listed in Tables 1, 2, 3, or a combination thereof.

In some embodiments of the gene chips, processes, methods and kits provided by the invention, the neurological condition is selected from the group consisting of autism spectrum disorders, autism, atypical autism, pervasive developmental disorder-not otherwise specified (PDD-NOS), Asperger's Disorder, Rett's syndrome, allodynia, catalepsy, hypernocieption, Parkinson's disease, parkinsonism, cognitive impairments, age-associated memory impairments, cognitive impairments, dementia associated with neurologic and/or neurological conditions, allodynia, catalepsy, hypernocieption, and epilepsy, brain tumors, brain lesions, multiple sclerosis, Down's syndrome, progressive supranuclear palsy, frontal lobe syndrome, schizophrenia, delirium, Tourette's syndrome, myasthenia gravis, attention deficit hyperactivity disorder, dyslexia, mania, depression, apathy, myopathy, Alzheimer's disease, Huntington's Disease, dementia, encephalopathy, schizophrenia, severe clinical depression, brain injury, Attention Deficit Disorder (ADD), Attention Deficit Hyperactivity Disorder (ADHD), hyperactivity disorder, Asperger's Disorder, bipolar manic-depressive disorder, ischemia, alcohol addiction, drug addiction, obsessive compulsive disorders, Pick's disease and Binswanger's disease.

DNA microarray and methods of analyzing data from microarrays are well-described in the art, including in DNA Microarrays: A Molecular Cloning Manual, Ed by Bowtell and Sambrook (Cold Spring Harbor Laboratory Press, 2002); Microarrays for an Integrative Genomics by Kohana (MIT Press, 2002); A Biologist's Guide to Analysis of DNA Microarray Data, by Knudsen (Wiley, John & Sons, Incorporated, 2002); and DNA Microarrays: A Practical Approach, Vol. 205 by Schema (Oxford University Press, 1999); and Methods of Microarray Data Analysis II, ed. by Lin et al. (Kluwer Academic Publishers, 2002), hereby incorporated by reference in their entirety.

Microarrays may be prepared by selecting probes which comprise a polynucleotide sequence, and then immobilizing such probes to a solid support or surface. For example, the probes may comprise DNA sequences, RNA sequences, or copolymer sequences of DNA and RNA. The polynucleotide sequences of the probes may also comprise DNA and/or RNA analogues, or combinations thereof. For example, the polynucleotide sequences of the probes may be full or partial fragments of genomic DNA. The polynucleotide sequences of the probes may also be synthesized nucleotide sequences, such as synthetic oligonucleotide sequences. The probe sequences can be synthesized either enzymatically *in vivo,* enzymatically *in vitro* (e.g., by PCR), or non-enzymatically *in vitro.*

The probe or probes used in the methods and gene chips of the invention may be immobilized to a solid support which may be either porous or non-porous. For example, the probes of the invention may be polynucleotide sequences which are attached to a nitrocellulose or nylon membrane or filter covalently at either the 3' or the 5' end of the polynucleotide. Such hybridization probes are well known in the art (see, e.g., Sambrook et al., MOLECULAR CLONING--A LABORATORY MANUAL (2ND ED.), Vols. 1-3, Cold Spring Harbor Laboratory, Cold Spring Harbor, N.Y. (1989). Alternatively, the solid support or surface may be a glass or plastic surface. In one embodiment, hybridization levels are measured to microarrays of probes consisting of a solid phase on the surface of which are immobilized a population of polynucleotides, such as a population of DNA or DNA mimics, or, alternatively, a population of RNA or RNA mimics. The solid phase may be a nonporous or, optionally, a porous material such as a gel.

In one embodiment, a microarray comprises a support or surface with an ordered array of binding (e.g., hybridization) sites or "probes" each representing one of the markers described herein. Preferably the microarrays are addressable arrays, and more preferably positionally addressable arrays. More specifically, each probe of the array is preferably located at a known, predetermined position on the solid support such that the identity (i.e., the sequence) of each probe can be determined from its position in the array (i.e., on the support or surface). In preferred embodiments, each probe is covalently attached to the solid support at a single site.

Microarrays can be made in a number of ways, of which several are described below. However produced, microarrays share certain characteristics. The arrays are reproducible, allowing multiple copies of a given array to be produced and easily compared with each other. Preferably, microarrays are made from materials that are stable under binding (e.g., nucleic acid hybridization) conditions. The microarrays are preferably small, e.g., between 1 cm² and 25 cm², between 12 cm² and 13 cm², or about 3 cm². However, larger arrays are also contemplated and may be preferable, e.g., for use in screening arrays. Preferably, a given binding site or unique set of binding sites in the microarray will specifically bind (e.g., hybridize) to the product of a single gene in a cell (e.g., to a specific mRNA). However, in general, other related or similar sequences will cross hybridize to a given binding site.

The microarrays of the present invention include one or more test probes, each of which has a polynucleotide sequence that is complementary to a subsequence of RNA or DNA to be detected. Preferably, the position of each probe on the solid surface is known. Indeed, the microarrays are preferably positionally addressable arrays. Specifically, each probe of the array is preferably located at a known, predetermined position on the solid support such that the identity (i.e., the sequence) of each probe can be determined from its position on the array (i.e., on the support or surface).

According to one aspect of the invention, the microarray is an array (i.e., a matrix) in which each position represents one of the markers or gene biomarkers as described herein. For example, each position can contain a DNA or DNA analogue based on genomic DNA to which a particular RNA transcribed from that genetic marker or biomarker can specifically hybridize. The DNA or DNA analogue can be, for example, a synthetic oligomer or a gene fragment. In one embodiment, probes representing each of the differentially DNA-methylated genes or biomarkers in Tables 1, 2, 3, or a combination thereof are present on the array.

As noted above, the "probe" to which a particular polynucleotide molecule specifically hybridizes according to the invention contains a complementary polynucleotide sequence. In one embodiment, the probes of the differentially DNA-methylated gene array consist of nucleotide sequences of 10 to 1,000 nucleotides. In a preferred embodiment, the nucleotide sequences of the probes are in the range of 10-200 nucleotides in length and are genomic sequences of a species of organism, such that a plurality of different probes is present, with sequences complementary and thus capable of hybridizing to the genome of such a species of organism, sequentially tiled across all or a portion of such genome. In other specific embodiments, the probes are in the range of 10-30 nucleotides in length, in the range of 10-40 nucleotides in length, in the range of 20-50 nucleotides in length, in the range of 40-80 nucleotides in length, in the range of 50-150 nucleotides in length, in the range of 80-120 nucleotides in length, and most preferably are 60 nucleotides in length.

The probes may comprise DNA or DNA "mimics" (e.g., derivatives and analogues) corresponding to a portion of an organism's genome. In another embodiment, the probes of the microarray are complementary RNA or RNA mimics. DNA mimics are polymers composed of subunits capable of specific, Watson-Crick-like hybridization with DNA, or of specific hybridization with RNA. The nucleic acids can be modified at the base moiety, at the sugar moiety, or at the phosphate backbone. Exemplary DNA mimics include, e.g., phosphorothioates.

DNA can be obtained, e.g., by polymerase chain reaction (PCR) amplification of genomic DNA or cloned sequences. PCR primers are preferably chosen based on a known sequence of the genome that will result in amplification of specific fragments of genomic DNA. Computer programs that are well known in the art are useful in the design of primers with the required specificity and optimal amplification properties, such as OLIGO version 5.0 (National Biosciences, Inc. Plymouth, MN). Typically each probe on the microarray will be between 10 bases and 50,000 bases, usually between 300 bases and 1,000 bases in length. PCR methods are well known in the art, and are described, for example, in Innis et al., eds., PCR: Protocols: A Guide to Methods and Applications, Academic Press Inc., San Diego, Calif. (1990). It will be apparent to one skilled in the art that controlled robotic systems are useful for isolating and amplifying nucleic acids.

An alternative, means for generating the polynucleotide probes of the microarray is by synthesis of synthetic polynucleotides or oligonucleotides, e.g., using N-phosphonate or phosphoramidite chemistries (Froehler et al., Nucleic Acid Res. 14:5399- 5407 (1986); McBride et al., Tetrahedron Lett. 24:246-248 (1983)). Synthetic sequences are typically between about 10 and about 500 bases in length, more typically between about 20 and about 100 bases, and most preferably between about 40 and about 70 bases in length. In some embodiments, synthetic nucleic acids include non-natural bases, such as, but by no means limited to, inosine. As noted above, nucleic acid analogues may be used as binding sites for hybridization. An example of a suitable nucleic acid analogue is peptide nucleic acid and methods for the preparation of peptide nucleic acids are known in the art (see, e.g., Egholm et al., Nature 363:566-568 (1993); U.S. Pat. No. 5,539,083). Probes are preferably selected using an algorithm that takes into account binding energies, base composition, sequence complexity, cross-hybridization binding energies, and secondary structure (see Friend et al.., International Patent Publication WO 01/05935, published Jan. 25, 2001; Hughes et al., Nat. Biotech. 19:342-7 (2001)).

A skilled artisan will also appreciate that positive control probes, e.g., probes known to be complementary and hybridizable to sequences in the DNA molecules, and negative control probes, e.g., probes known to not be complementary and hybridizable to sequences in the DNA molecules, should be included on the array. In one embodiment, positive controls are synthesized along the perimeter of the array. In another embodiment, positive controls are synthesized in diagonal stripes across the array. In still another embodiment, the reverse complement for each probe is synthesized next to the position of the probe to serve as a negative control. In yet another embodiment, sequences from other species of organism are used as negative controls or as "spike-in" controls.

The probes may be attached to a solid support or surface, which may be made, e.g., from glass, plastic (e.g., polypropylene, nylon), polyacrylamide, nitrocellulose, gel, or other porous or nonporous material. A preferred method for attaching the nucleic acids to a surface is by printing on glass plates, as is described generally by Schena et al., Science 270:467-470 (1995). This method is especially useful for preparing microarrays of cDNA (See also, DeRisi et al., Nature Genetics 14:457-460 (1996); Shalon et al., Genome Res. 6:639-645 (1996); and Schena et al., Proc. Natl. Acad. Sci. U.S.A. 93:10539-11286 (1995)).

A second preferred method for making microarrays is by making high-density oligonucleotide arrays. Techniques are known for producing arrays containing thousands of oligonucleotides complementary to defined sequences, at defined locations on a surface using photolithographic techniques for synthesis in situ (see, Fodoret al., 1991, Science 251:767-773; Pease et al., 1994, Proc. Natl. Acad. Sci. U.S.A. 91:5022-5026; Lockhart et al., 1996, Nature Biotechnology 14:1675; U.S. Pat. Nos. 5,578,832; 5,556,752; and 5,510,270) or other methods for rapid synthesis and deposition of defined oligonucleotides (Blanchard et al., Biosensors & Bioelectronics 11:687-690). When these methods are used, oligonucleotides (e.g., 60-mers) of known sequence are synthesized directly on a surface such as a derivatized glass slide. Usually, the array produced is redundant, with several oligonucleotide molecules per RNA.

Other methods for making microarrays, e.g., by masking (Maskos and Southern, 1992, Nuc. Acids. Res. 20:1679-1684), may also be used. In principle, and as noted supra, any type of array, for example, dot blots on a nylon hybridization membrane (see Sambrook et al., MOLECULAR CLONING--A LABORATORY MANUAL (2ND ED.), Vols. 1-3, Cold Spring Harbor Laboratory, Cold Spring Harbor, N.Y. (1989)) could be used. However, as will be recognized by those skilled in the art, very small arrays will frequently be preferred because hybridization volumes will be smaller. In one embodiment, the arrays of the present invention are prepared by synthesizing polynucleotide probes on a support. In such an embodiment, polynucleotide probes are attached to the support covalently at either the 3' or the 5' end of the polynucleotide.

In a one embodiment, microarrays of the invention are manufactured by means of an ink jet printing device for oligonucleotide synthesis, e.g., using the methods and systems described by Blanchard in U.S. Pat. No. 6,028,189; Blanchard et al., 1996, Biosensors and Bioelectronics 11:687-690; Blanchard, 1998, in SYNTHETIC DNA ARRAYS IN GENETIC ENGINEERING, Vol. 20, J. K. Setlow, Ed., Plenum Press, New York at pages 111-123. Specifically, the oligonucleotide probes in such microarrays are preferably synthesized in arrays, e.g., on a glass slide, by serially depositing individual nucleotide bases in "microdroplets" of a high surface tension solvent such as propylene carbonate. The microdroplets have small volumes (e.g., 100 pL or less, more preferably 50 pL or less) and are separated from each other on the microarray (e.g., by hydrophobic domains) to form circular surface tension wells which define the locations of the array elements (i.e., the different probes). Microarrays manufactured by this ink-jet method are typically of high density, preferably having a density of at least about 2,500 different probes per 1 cm2. The polynucleotide probes are attached to the support covalently at either the 3' or the 5' end of the polynucleotide.

Also an embodiment of this invention are methods of screening a subject for a neurological disease or disorder, and assessing a subject's risk for having or developing a neurological disease or disorder, e.g., and autism spectrum disorder The methods comprise isolating nucleic acid, e.g., a DNA sample, from a sample from a test subject, and assaying the differential DNA methylation of at least one gene in Table 1, Table 2, Table 3 or a combination thereof in the isolated DNA of a subject and then comparing the level of methylation of the genes in the test sample to the methylation of those same genes in a control sample. Preferably the gene(s) comprise RORA or RORA and BCL-2. Where the methylation profile from the test subject is compared to a negative control, a statistically significant difference in the differential methylation of at least one gene listed in Table 1, Table 2, Table 3 between the test sample and the negative control aides in assessing the test subject's likelihood of having or developing the neurological disease or disorder. For example, a difference of log2 ratio greater than +/- 0.3 between the level of methylation of the gene(s) of the test sample and the same genes in the control sample indicates an increased risk for having or developing a neurological disorder or disease, e.g. an autism spectrum disorder The a negative control may be, e.g., a nucleic acid, DNA, sample from a plurality of subjects who do not have a neurological disease or disorder, particularly the neurological disorder for which the subject is being tested, e.g., an autism spectrum disorder. The control may be, e.g., non-phenotypic discordant twin, siblings of the subject, or unrelated subjects. The autism spectrum disorder may comprise, or be selected from the group consisting of, pervasive developmental disorder-not otherwise specified (PDD-NOS), including atypical autism, and Asperger's Disorder. The autism spectrum disorder may also be characterized by a lower severity score across all ADIR items, or an intermediate severity score across all ADIR items, or a higher severity scores on spoken language items on the ADIR, or a higher frequency of savant skills, or a severe language impairment, or a combination thereof.

The nucleic acid may be obtained from a neurological sample as described herein. The nucleic acid may be obtained from a peripheral tissue sample, e.g., epithelial cells from a cheek swab, or a lymphocyte containing sample, e.g., blood. The nucleic acid may also be obtained from a lymphoblastoid cell line (LCL) derived from the test or control subject.

The level of DNA methylation in the nucleic acid samples may be quantified by methods known in the art some of which are disclosed herein, e.g., bisulfite-sequencing, methylation-specific PCR (MSP), large scale CpG island microarray analysis using methylation-enriched and -unenriched samples, promoter analysis using methylation-enriched and -unenriched samples, combined bisulfite and restriction analysis (COBRA), pyrosequncing, Methyl-Light (Premier Biosoft International, Palo Alto CA) or Methyl-Profiler (SABiosciences, Frederick, MD) methylation analysis, Sequenom mass analyzer, bisulfite treatment of DNA combined with selected amplification of the specific promoter regions of differentially methylated genes with the addition of a T7 promoter tag, followed by T7-mediated transcription and RNase T1 cleavage of the transcript with methylation sites determined by MALDI-TOF mass spectrometry analysis in the manner of Schatz et al. (2004), or a combination thereof.

### Methods of Determining Differentially DNA-Methylated Gene Profiles

One aspect of the invention provides methods for determining a differentially DNA-methylated gene profile for a specific neurological disorder or neurological condition, such as autism spectrum disorder conditions including autistic disorder, pervasive developmental disorder-not otherwise specified (PDD-NOS), including atypical autism, Asperger's Disorder. Furthermore, the systems and methods described herein may be employed to generate differentially DNA-methylated gene profiles for diseases or disorders of interest. This expression data may be analyzed independently to determine a differentially DNA-methylated gene profile of interest, or combined with the existing biological data stored in a plurality of different types of databases. Statistical analyses may be applied as well as machine learning techniques that are used to discover trends and patterns in the underlying data. These techniques include clustering methods, which can be used for example to organize microarray expression data.

One specific aspect of the invention provides a method for determining a differentially DNA-methylated gene profile for a neurological condition, comprising (i) preparing samples of control and experimental DNA , wherein the experimental DNA is generated from a nucleic acid sample isolated from a subject suspected of being afflicted with the neurological condition; (ii) preparing one or more microarrays comprising a plurality of different oligonucleotides having specificity for differentially DNA-methylated genes associated with the neurological condition; (iii) applying the prepared samples to the one or more microarrays to allow hybridization between the oligonucleotides and the control and experimental DNA s; (v) identifying the oligonucleotides on the microarray which display differential hybridization to the experimental DNA relative to the control DNA ; and (vi) identifying a set of genes from the oligonucleotides identified in step (v) thereby determining a differentially DNA-methylated gene profile for the neurological condition.

In a preferred embodiment, the neurological condition is an autism spectrum disorder condition including autistic disorder, pervasive developmental disorder-not otherwise specified (PDD-NOS), including atypical autism, Asperger's Disorder, or a combination thereof. In another embodiment, the neurological condition is selected from the group consisting of autism spectrum disorder conditions including autistic disorder, pervasive developmental disorder-not otherwise specified (PDD-NOS), including atypical autism, Asperger's Disorder, Rett's syndrome, Parkinson's disease, parkinsonism, cognitive impairments, age-associated memory impairments, cognitive impairments, dementia associated with neurologic and/or neurological conditions, allodynia, catalepsy, hypernocieption, and epilepsy, brain tumors, brain lesions, multiple sclerosis, Down's syndrome, progressive supranuclear palsy, frontal lobe syndrome, schizophrenia, delirium, Tourette's syndrome, myasthenia gravis, attention deficit hyperactivity disorder, dyslexia, mania, depression, apathy, myopathy, Alzheimer's disease, Huntington's Disease, dementia, encephalopathy, schizophrenia, severe clinical depression, brain injury, Attention Deficit Disorder (ADD), Attention Deficit Hyperactivity Disorder (ADHD), hyperactivity disorder, bipolar manic-depressive disorder, ischemia, alcohol addiction, drug addiction, obsessive compulsive disorders, Pick's disease and Binswanger's disease.

In another embodiment, the samples of experimental DNA may be isolated from a subject or group of subjects suspected of being afflicted or afflicted with one or more neurological conditions, such as autism spectrum disorder condition including autistic disorder, pervasive developmental disorder-not otherwise specified (PDD-NOS), including atypical autism, Asperger's Disorder, or a combination thereof. Control DNA may be derived from a nucleic acid sample of a subject or group of subjects which are not afflicted with the neurological conditions that the subjects from which the experimental DNA was derived. In another embodiment, the subjects from which the experimental and control samples are derived may both be suspected of being afflicted or afflicted with the condition, but the severity of the condition or a treatment plan in the two subject groups may differ.

A related aspect of the invention provides a method of determining a differentially DNA-methylated gene profile for the administration of a therapeutic treatment to a subject. Such methods are useful to detect the gene expression changes that accompany the underlying therapeutic treatments. A differentially DNA-methylated gene profile for such genetic changes may be used to determine if a second therapeutic treatment is expected to have the same effect, by comparing the differentially DNA-methylated gene profile of the second treatment to the differentially DNA-methylated gene profile of the first.

Accordingly, one specific aspect of the invention provides a method of determining a differentially DNA-methylated gene profile indicative for the administration of a therapeutic treatment to a subject, the method comprising (i) preparing samples of control and experimental DNA , wherein the experimental DNA is generated from a nucleic acid sample isolated from a subject who has received or is receiving the therapeutic treatment; (ii) preparing one or more microarrays comprising a plurality of different oligonucleotides wherein the oligonucleotides are specific to differentially DNA-methylated genes associated with an autism spectrum disorder; (iii) applying the prepared samples to the one or more microarrays to allow hybridization between the oligonucleotides and the control and experimental DNAs; (v) identifying the oligonucleotides on the microarray which display differential hybridization to the experimental DNA relative to the control DNA; (vi) identifying a set of genes or differentially DNA-methylated genes associated with an autism spectrum disorder from the oligonucleotides identified in step (v) thereby determining a differentially DNA-methylated gene profile for the administration of the therapeutic treatment to the subject.

In yet another aspect of the invention, a method is provided for determining a differentially DNA-methylated gene profile for at least one autism spectrum disorder, comprising (a) preparing samples of control and experimental DNA , wherein the experimental DNA is generated from a nucleic acid sample isolated from a subject suspected of being afflicted with the at least one autism spectrum disorder and the control DNA is generated from a nucleic acid sample isolated from a healthy individual; (b) preparing one or more microarrays comprising a plurality of different oligonucleotides having specificity for differentially DNA-methylated genes associated with the at least one autism spectrum disorder; (c) applying the prepared samples to the one or more microarrays to allow hybridization between the oligonucleotides and the control DNA s and the oligonucleotide and the experimental DNA s; (d) identifying the oligonucleotides on the microarray which display differential hybridization to the experimental DNA relative to the control DNA thereby determining a differentially DNA-methylated gene profile for the at least one autism spectrum disorder.

In yet another aspect of the invention, a method is provided for distinguishing between different phenotypes of an autism spectrum disorder comprising severely language impaired (L), mildly affected (M), or "savants" (S) comprising (a) preparing samples of control and experimental DNA, wherein the experimental DNA is generated from a nucleic acid sample isolated from a subject suspected of being afflicted with at least one phenotype comprising the severely language impaired (L), mildly affected (M), or "savants" (S); (b) preparing one or more microarrays comprising a plurality of different oligonucleotides having specificity for differentially DNA-methylated genes associated with the at least one phenotype; (c) applying the prepared samples to the one or more microarrays to allow hybridization between the oligonucleotides and the control and experimental DNA s; (d) identifying the oligonucleotides on the microarray which display differential hybridization to the experimental DNA relative to the control DNA thereby determining a differentially DNA-methylated gene profile for distinguishing among the different phenotypes of autism spectrum disorder. The control may be e.g., a sample from a subject who is known to have an autism spectrum disorder, e.g., severely language impaired (L), mildly affected (M), or "savants" (S), wherein a greater similarity of the experimental profile to one of the ASD profiles more than to the others distinguishes the phenotype of the experimental profile.

### Methods for categorizing subjects as severely language impaired (L), mildly affected (M), or "savants" (S)

In yet another embodiment of the screening method of the present invention, the method distinguishes between different variants of autism spectrum disorder comprising a lower severity scores across all ADIR items, an intermediate severity across all ADIR items, a higher severity scores on spoken language items on the ADIR, a higher frequency of savant skills, and a severe language impairment, or a combination thereof. Methods for determining severity scores across ADIR items, savant skills and language impairment are known. See, e.g., Hu and Steinberg (2009) Autism Research 2:67-77, e.g., Tables 1 and Supplementary Table 1 therein, wherein ADIR scores of about 2000 autistic individuals were used to identify phenotypic subgroups with idiopathic ASD who were characterized by combined symptoms across multiple domains.

In one embodiment of the methods for determining a differentially DNA-methylated gene profile for the administration of a therapeutic treatment, administration of therapeutic treatment results in a physiological change in the subject, such as a beneficial change. In a specific embodiment, the physiological change comprises one or more improvements in social interaction, language abilities, restricted interests, repetitive behaviors, sleep disorders, seizures, gastrointestinal, hepatic, and mitochondrial function, neural inflammation, or a combination thereof. In another embodiment, the control DNA may be derived from the subject(s) prior to administration of the therapeutic treatment, or from a subject or group of subjects who do not receive the therapeutic treatment.

In another embodiment of the methods for determining a differentially DNA-methylated gene profile for the administration of a therapeutic treatment to a subject suspected of being afflicted with or afflicted with autism spectrum disorder conditions including autistic disorder, pervasive developmental disorder-not otherwise specified (PDD-NOS), including atypical autism, Asperger's Disorder, the therapeutic treatment may comprise a single procedure or it may comprise an aggregate of treatment procedures. In one embodiment, therapeutic treatment comprises a behavioral therapy, such as applied behavior analysis (ABA) intervention methods, dietary changes, exercise, massage therapy, group therapy, talk therapy, play therapy, conditioning, or alternative therapies such as sensory integration and auditory integration therapies. In another embodiment, the therapeutic treatment comprises administering to the subject a drug, such as an antidepressant or antipsychotic drug. In another embodiment, the subject is afflicted with a neurological condition other than autism spectrum disorder conditions including autistic disorder, pervasive developmental disorder-not otherwise specified (PDD-NOS), including atypical autism, Asperger's Disorder. Such condition may be one which the therapeutic treatment is intended to treat. In another embodiment, the subject is a healthy subject who is not afflicted with a neurological condition. In another embodiment, the therapeutic treatment is a treatment for the autism spectrum disorder neurological conditions including autistic disorder, pervasive developmental disorder-not otherwise specified (PDD-NOS), including atypical autism, Asperger's Disorder.

In another embodiment, the drug being administered in the single procedure or the aggregate of treatment procedures is a serotonergic antidepressant medication, such as one selected from the group consisting of citalopram, flubxetine, fluvoxamine, paroxetine, or sertraline, or the drug is a catecholaminergic antidepressant medication, such as bupropion.

In another preferred embodiment of the methods described herein, both the control DNA and the experimental DNA are derived from a nucleic acid sample isolated from the subject. Samples may be isolated from a mammal, such as a human. In a specific embodiment, the sample is isolated post-mortem from a human. Nucleic acid samples may be isolated from any tissue or bodily fluid, including blood, saliva, tears, cerebrospinal fluid, pericardial fluid, synovial fluid, aminiotic fluid, semen, bile, ear wax, gastric acid, sweat, urine, or fluid drained from an edema. In a further specific embodiment, the nucleic acid sample is isolated from lymphoblastoid cells or lyphoblastoid cell lines (LCL) derived from blood cells of subjects. In some embodiments of the methods described herein, the sample is isolated from a neuronal tissue or a combination of tissue types, such as olfactory bulb cells, cerebrospinal fluid, hypothalamus, amygdala, pituitary, spinal cord, brainstem, cerebellum, cortex, frontal cortex, hippocampus, choroid plexus, striatum, and thalamus.

In one embodiment of the methods described herein, the microarray is any one of the microarrays, or differentially DNA-methylated gene chips described herein. In a preferred embodiment, the oligonucleotides on the microarray comprise those specific to differentially DNA-methylated genes selected from Table 1, Table 2, Table 3, or a combination thereof. In a specific embodiment, the oligonucleotides of the microarray are specific to differentially DNA-methylated genes associated with nervous system development, axon guidance, synaptic transmission or plasticity, myelination, long-term potentiation, neuron toxicity, Purkinje cell differentiation, cerebellar development, embryonic development, regulation of actin networks, digestion, inflammation, oxidative stress, epilepsy, apoptosis, morphogenesis, cell survival, differentiation, the unfolded protein response, Type II diabetes and insulin signaling, digestion, liver toxicity (hepatic stellate cell activation, fibrosis, and cholestasis), endocrine function, circadian rhythm, cholesterol metabolism and the steroidogenesis pathway, or a combination thereof as described supra. In a preferred embodiment, at least 10%, 20%, 30%, 40%, 50%, 60%, 70%, 80%, 90%, 95%, or 99% of the genes on the microarray are specific to differentially DNA-methylated genes selected from Table 1, Table 2, Table 3, or a combination thereof.

In another embodiment of the methods described herein, the control DNA and the experimental DNA are hybridized to the same microarray, while in another embodiment they are hybridized to separate but substantially identical microarrays. If the same microarray is used, the DNA samples may be labeled using fluorescent compounds having different emission wavelengths such that the signals generated by each DNA type may be distinguished from a single microarray.

In yet another embodiment of the methods described herein, the control and experimental DNA is isolated from one or more subjects. In one embodiment, the control DNA and experimental DNA are isolated each from at least 3, 5, 10, 15 or 20 subjects. The DNAs from each subject may be hybridized to the microarrays separately, or the control DNAs, or the experimental DNAs, may be pooled together, such that, for example, an experimental DNA sample is derived from multiple subjects. In preferred embodiments, the subjects are mammals, such as rodents, primates or humans.

In one embodiment of the methods described herein, the set of differentially DNA-methylated genes in the gene profile comprise differentially DNA-methylated genes which have a differential expression in the experimental DNA relative to the control DNA. Differential expression may refer to a lower expression level or to a higher expression. In preferred embodiments, the difference in expression level is statistically significant for each differentially DNA-methylated gene, or marker, on the set. In preferred embodiments, the difference in expression is at least 10%, 20%, 30%, 40%, 50%, 60%, 70%, 80%, 90%, 100%, 150%, 200%, 300%, 400%, or 500% greater in the experimental DNA than in the control DNA , or vice versa. In another preferred embodiment, the difference in expression is at least about 1.22-fold, 1.5-fold, 2-fold, 2.5- fold, 3-fold, 3.5-fold, 4-fold, 4.5-fold, 5-fold, 6-fold, 7-fold, 8-fold, 9-fold, 10-fold, 12- fold, 14-fold, 16-fold, 18-fold, 20-fold, 25-fold, 30-fold, 35-fold, 40-fold, 45-fold, 50- fold, 55-fold, 60-fold, 65-fold, 70-fold, 75-fold, 80-fold, 85-fold, 90-fold, 95-fold, 100- fold greater (or intermediate ranges thereof as another example) in the experimental DNA than in the control DNA, or vice versa A gene profile may comprise all the differentially DNA-methylated genes which are differentially expressed between the control and experimental DNAs or it may comprise a subset of those genes. In some embodiments, the gene profile comprises at least 1%, 2%, 3%, 4%, 5%, 10%, 20%, 30%, 40%, 50%, 60%, 70%, 80%, 90%, 95%, 98%, 99% or 100% (or intermediate ranges thereof as another example) of the differentially DNA-methylated genes having differential expression. Differentially DNA-methylated genes showing large, reproducible changes in expression between the two samples are preferred in some embodiments. In preferred embodiments, the differentially DNA-methylated gene profile further comprises a subset of values associated with the expression level of each of the differentially DNA-methylated gene in the profile, such that differentially DNA-methylated gene profile allows the identification of a biological and/or pathological condition, an agent and/or its biological mechanism of action, or a physiological process.

The preparation of samples of control and experimental DNA may be carried out using techniques known in the art. The DNA molecules analyzed by the present invention may be from any clinically relevant source. In one embodiment, the DNA is derived from RNA, including, but by no means limited to, total cellular RNA, poly(A)⁺ messenger RNA (mRNA) or fraction thereof, cytoplasmic mRNA, or RNA transcribed from cDNA (i.e., cRNA; see, e.g., U.S. Pat. Nos. 5,545,522, 5,891,636, or 5,716,785). Methods for preparing total and poly(A)⁺ RNA are well known in the art, and are described generally, e.g., in Sambrook et al., MOLECULAR CLONING--A LABORATORY MANUAL (2ND ED.), Vols. 1-3, Cold Spring Harbor Laboratory, Cold Spring Harbor, N.Y. (1989). In one embodiment, RNA is extracted from a sample of cells of the various tissue types of interest, such as the lymphoblastoid cell or lymphoblastoid cell line derived therefrom or from the aforementioned neuronal tissue types, using guanidinium thiocyanate lysis followed by CsCl centrifugation (Chirgwin et al., 1979, Biochemistry 18:5294-5299). In another embodiment, total RNA is extracted using a silica gel-based column, commercially available examples of which include RNeasy^{™} (Qiagen, Valencia, Calif.) and StrataPrep (Stratagene, La Jolla, Calif.). Poly(A)⁺ RNA can be selected, e.g., by selection with oligo-dT cellulose or, alternatively, by oligo-dT primed reverse transcription of total cellular RNA. In one embodiment, RNA can be fragmented by methods known in the art, e.g., by incubation with ZnCl₂, to generate fragments of RNA. In another embodiment, the polynucleotide molecules analyzed by the invention comprise PCR products of amplified polynucleotides (e.g., RNA or cDNA, among others). DNA molecules that are poorly expressed in particular cells may be enriched using normalization techniques (Bonaldo et al., 1996, Genome Res. 6:791-806).

The DNAs may be detectably labeled at one or more nucleotides. Any method known in the art may be used to detectably label the DNAs. Preferably, this labeling incorporates the label uniformly along the length of the RNA, and more preferably, the labeling is carried out at a high degree of efficiency. One embodiment for this labeling uses oligo-dT primed reverse transcription to incorporate the label; however, conventional methods of this method are biased toward generating 3' end fragments. Thus, in one embodiment, random primers (e.g., 9-mers) are used in reverse transcription to uniformly incorporate labeled nucleotides over the full length of the DNAs. Alternatively, random primers may be used in conjunction with PCR methods or T7 promoter-based in vitro transcription methods in order to amplify the cDNAs.

In one embodiment, the detectable label is a luminescent label. For example, fluorescent labels, bioluminescent labels, chemiluminescent labels, and colorimetric labels may be used in the present invention. In one preferred embodiment, the label is a fluorescent label, such as a fluorescein, a phosphor, a rhodamine, or a polymethine dye derivative. Examples of commercially available fluorescent labels include, for example, fluorescent phosphoramidites such as FLUOREPRIME^{™} (Amersham Pharmacia, Piscataway, N.J.), Fluoredite (Millipore, Bedford, Mass.), FAM (ABI, Foster City, Calif.), and Cy3 or Cy5 (Amersham Pharmacia, Piscataway, N.J.). In another embodiment, the detectable label is a radiolabeled nucleotide.

In a further preferred embodiment, the experimental DNAs are labeled differentially from the control DNA, especially if both the DNA types are hybridized to the same microarray. The control DNA can comprise target polynucleotide molecules from normal individuals (i.e., those not afflicted with the neurological disorder or subjects who have not undergone to therapeutic treatment). In one preferred embodiment, the control DNA comprises target polynucleotide molecules pooled from samples from normal individuals. In one embodiment of the methods for generating a gene profile or DNA methylation profile of a therapeutic treatment, the control DNA is derived from the same subject, but taken at a different time point, such as before, during or after the therapeutic treatment.

Nucleic acid hybridization and wash conditions are chosen so that the DNA molecules specifically bind or specifically hybridize to the complementary polynucleotide sequences of the array, preferably to a specific array site, wherein its complementary DNA is located. Arrays containing double-stranded probe DNA situated thereon are preferably subjected to denaturing conditions to render the DNA single-stranded prior to contacting with the DNA molecules. Arrays containing single-stranded probe DNA (e.g., synthetic oligodeoxyribonucleic acids) may need to be denatured prior to contacting with the DNA molecules. Optimal hybridization conditions will depend on the length (e.g., oligomer versus polynucleotide greater than 200 bases) and type (e.g., RNA, or DNA) of probe and target nucleic acids. One of skill in the art will appreciate that as the oligonucleotides become shorter, it may become necessary to adjust their length to achieve a relatively uniform melting temperature for satisfactory hybridization results. General parameters for specific (i.e., stringent) hybridization conditions for nucleic acids are described in Sambrook et al., MOLECULAR CLONING--A LABORATORY MANUAL (2ND ED.), Vols. 1-3, Cold Spring Harbor Laboratory, Cold Spring Harbor, N.Y. (1989), and in Ausubel et al., CURRENT PROTOCOLS IN MOLECULAR BIOLOGY, vol. 2, Current Protocols Publishing, New York (1994). Typical hybridization conditions for the cDNA microarrays of Schena et al. are hybridization in 5X SSC plus 0.2% SDS at 65° C for four hours, followed by washes at 25° C in low stringency wash buffer (IX SSC plus 0.2% SDS), followed by 10 minutes at 25° C in higher stringency wash buffer (0.1X SSC plus 0.2% SDS) (Schena et al., Proc. Natl. Acad. Sci. U.S.A. 93:10614 (1993)). Useful hybridization conditions are also provided in, e.g., Tijessen, 1993, HYBRIDIZATION WITH NUCLEIC ACID PROBES, Elsevier Science Publishers B. V.; and Kricka, 1992, NONISOTOPIC DNA PROBE TECHNIQUES, Academic Press, San Diego, Calif. Hybridization conditions may include hybridization at a temperature at or near the mean melting temperature of the probes (e.g., within 5° C., more preferably within 2° C) in 1 M NaCl, 50 mM MES buffer (pH 6.5), 0.5% sodium sarcosine and 30% formamide.

When fluorescently labeled DNAs are used in the aforementioned methods, the fluorescence emissions at each site of a microarray may be, preferably, detected by scanning confocal laser microscopy. In one embodiment, a separate scan, using the appropriate excitation line, is carried out for each of the two fluorophores used. Alternatively, a laser may be used that allows simultaneous specimen illumination at wavelengths specific to the two fluorophores and emissions from the two fluorophores can be analyzed simultaneously (see Shalon et al., 1996, "A DNA microarray system for analyzing complex DNA samples using two-color fluorescent probe hybridization," Genome Research 6:639-645, which is incorporated by reference in its entirety for all purposes). In one preferred embodiment, the arrays are scanned with a laser fluorescent scanner with a computer controlled X-Y stage and a microscope objective. Sequential excitation of the two fluorophores is achieved with a multi-line, mixed gas laser and the emitted light is split by wavelength and detected with two photomultiplier tubes. Fluorescence laser scanning devices are described in Schena et al., Genome Res. 6:639- 645 (1996), and in other references cited herein. Alternatively, the fiber-optic bundle described by Ferguson et al., Nature Biotech. 14:1681-1684 (1996), may be used to monitor differentially DNA-methylated gene or DNA abundance levels at a large number of sites simultaneously.

Signals may be recorded and, in a preferred embodiment, analyzed by computer, e.g., using a 12 or 16 bit analog to digital board. In one embodiment the scanned image is despeckled using a graphics program (e.g., Hijaak Graphics Suite) and then analyzed using an image gridding program that creates a spreadsheet of the average hybridization at each wavelength at each site. If necessary, an experimentally determined correction for "cross talk" (or overlap) between the channels for the two fluors may be made. For any particular hybridization site on the transcript array, a ratio of the emission of the two fluorophores can be calculated. The ratio is independent of the absolute expression level of the differentially DNA-methylated gene, but is useful for differentially DNA-methylated genes whose expression is significantly modulated in association with the different neurological conditions.

In another embodiment of the present invention, changes in differentially DNA-methylated gene expression may be assayed in at least one cell of a subject by measuring transcriptional initiation, transcript stability, translation of transcript into protein product, protein stability, or a combination thereof. The gene, gene products, transcript, or polypeptide can be assayed by techniques such as in vitro transcription, quantitative nuclease protection assay (qNPA) analysis, focused gene chip analysis, Northern hybridization, nucleic acid hybridization, reverse transcription-polymerase chain reaction (RT-PCR), run-on transcription, Southern hybridization, electrophoretic mobility shift assay (EMSA), fluorescent or histochemical staining, microscopy and digital image analysis, and fluorescence activated cell analysis or sorting (FACS).

A reporter or selectable marker gene whose protein product is easily assayed may be used for convenient detection. Reporter genes include, for example, alkaline phosphatase, β-galactosidase (LacZ), chloramphenicol acetyltransferase (CAT), β-glucoronidase (GUS), bacterial/insect/marine invertebrate luciferases (LUC), green and red fluorescent proteins (GFP and RFP, respectively), horseradish peroxidase (HRP), β-lactamase, and derivatives thereof (e.g., blue EBFP, cyan ECFP, yellow-green EYFP, destabilized GFP variants, stabilized GFP variants, or fusion variants sold as LIVING COLORS^{™} fluorescent proteins by Clontech, Mountain View, CA). Reporter genes would use cognate substrates that are preferably assayed by a chromogen, fluorescent, or luminescent signal. Alternatively, assay product may be tagged with a heterologous epitope (e.g., FLAG, MYC, SV40 T antigen, glutathione transferase, hexahistidine, maltose binding protein) for which cognate antibodies or affinity resins are available.

In another embodiment, the gene or transcript can be assayed by using systems that employ expression vectors. An expression vector is a recombinant polynucleotide that is in chemical form either a deoxyribonucleic acid (DNA) and/or a ribonucleic acid (RNA). The physical form of the expression vector may also vary in strandedness (e.g., single-stranded or double-stranded) and topology (e.g., linear or circular). The expression vector is preferably a double-stranded deoxyribonucleic acid (dsDNA) or is converted into a dsDNA after introduction into a cell (e.g., insertion of a retrovirus into a host genome as a provirus). The expression vector may include one or more regions from a mammalian gene expressed in the microvasculature, especially endothelial cells (e.g., ICAM-2, tie), or a virus (e.g., adenovirus, adeno-associated virus, cytomegalovirus, fowlpox virus, herpes simplex virus, lentivirus, Moloney leukemia virus, mouse mammary tumor virus, Rous sarcoma virus, SV40 virus, vaccinia virus), as well as regions suitable for genetic manipulation (e.g., selectable marker, linker with multiple recognition sites for restriction endonucleases, promoter for in vitro transcription, primer annealing sites for in vitro replication). The expression vector may be associated with proteins and other nucleic acids in a carrier (e.g., packaged in a viral particle) or condensed with chemicals (e.g., cationic polymers) to target entry into a cell or tissue.

The expression vector further comprises a regulatory region for gene expression (e.g., promoter, enhancer, silencer, splice donor and acceptor sites, polyadenylation signal, cellular localization sequence). Transcription can be regulated by tetracycline or dimerized macrolides. The expression vector may be further comprised of one or more splice donor and acceptor sites within an expressed region; Kozak consensus sequence upstream of an expressed region for initiation of translation; and downstream of an expressed region, multiple stop codons in the three forward reading frames to ensure termination of translation, one or more mRNA degradation signals, a termination of transcription signal, a polyadenylation signal, and a 3' cleavage signal. For expressed regions that do not contain an intron (e.g., a coding region from a cDNA), a pair of splice donor and acceptor sites may or may not be preferred. It would be useful, however, to include mRNA degradation signal(s) if it is desired to express one or more of the downstream regions only under the inducing condition. An origin of replication may also be included that allows replication of the expression vector integrated in the host genome or as an autonomously replicating episome. Centromere and telomere sequences can also be included for the purposes of chromosomal segregation and protecting chromosomal ends from shortening, respectively. Random or targeted integration into the host genome is more likely to ensure maintenance of the expression vector but episomes could be maintained by selective pressure or, alternatively, may be preferred for those applications in which the expression vector is present only transiently.

An expressed region may be derived from any gene of interest, and be provided in either orientation with respect to the promoter; the expressed region in the antisense orientation will be useful for making cRNA and antisense polynucleotide. The gene may be derived from the host cell or organism, from the same species thereof, or designed de novo; but it is preferably of archael, bacterial, fungal, plant, or animal origin. The gene may have a physiological function of one or more nonexclusive classes: axon guidance, synaptic transmission or plasticity, myelination, long-term potentiation, neuron toxicity, embryonic development, regulation of actin networks, KEGG pathway, digestion, liver toxicity (hepatic stellate cell activation, fibrosis, and cholestasis), inflammation, oxidative stress, epilepsy, apoptosis, cell survival, differentiation, the unfolded protein response, Type II diabetes and insulin signaling, endocrine function, circadian rhythm, cholesterol metabolism and the steroidogenesis pathway, adhesion proteins; steroids, cytokines, hormones, and other regulators of cell growth, mitosis, meiosis, apoptosis, differentiation, circadian rhythm, or development; soluble or membrane receptors for such factors; adhesion molecules; cell-surface receptors and ligands thereof; cytoskeletal and extracellular matrix proteins; cluster differentiation (CD) antigens, antibody and T-cell antigen receptor chains, histocompatibility antigens, and other factors mediating specific recognition in immunity; chemokines, receptors thereof, and other factors involved in inflammation; enzymes producing lipid mediators of inflammation and regulators thereof; clotting and complement factors; ion channels and pumps; transporters and binding proteins; neurotransmitters, neurotrophic factors, and receptors thereof; cell cycle regulators, oncogenes, and tumor suppressors; other transducers or components of signaling pathways; proteases and inhibitors thereof; catabolic or metabolic enzymes, and regulators thereof. Some genes produce alternative transcripts, encode subunits that are assembled as homopolymers or heteropolymers, or produce propeptides that are activated by protease cleavage. The expressed region may encode a translational fusion; open reading frames of the regions encoding a polypeptide and at least one heterologous domain may be ligated in register. If a reporter or selectable marker is used as the heterologous domain, then expression of the fusion protein may be readily assayed or localized. The heterologous domain may be an affinity or epitope tag.

### Methods of Identifying or Characterizing Therapeutic Compounds

Another aspect of the invention is identification or screening of chemical or genetic compounds, derivatives thereof, and compositions including same that are effective in treatment of neurological diseases or disorders and individuals at risk thereof. The amount that is administered to an individual in need of therapy or prophylaxis, its formulation, and the timing and route of delivery is effective to reduce the number or severity of symptoms, to slow or limit progression of symptoms, to inhibit expression of one or more of the aforementioned differentially DNA-methylated genes that are transcribed at a higher level in neurological disease, to activate expression of one or more of the aforementioned differentially DNA-methylated genes that are transcribed at a lower level in neurological disease, or any combination thereof. Determination of such amounts, formulations, and timing and route of drug delivery is within the skill of persons conducting in vitro assays, in vivo studies of animal models, and human clinical trials.

A screening method may comprise administering a candidate compound to an organism or incubating a candidate compound with a cell, and then determining whether or not gene expression is modulated. Such modulation may be an increase or decrease in activity that partially or fully compensates for a change that is associated with or may cause neurological disease. Differentially DNA-methylated gene expression may be increased at the level of rate of transcriptional initiation, rate of transcriptional elongation, stability of transcript, translation of transcript, rate of translational initiation, rate of translational elongation, stability of protein, rate of protein folding, proportion of protein in active conformation, functional efficiency of protein (e.g., activation or repression of transcription), or combinations thereof. See, for example, U.S. Patent Numbers 5,071,773 and 5,262,300. High-throughput screening assays are possible (e.g., by using parallel processing and/or robotics).

The screening method may comprise incubating a candidate compound with a cell containing a reporter construct, the reporter construct comprising transcription regulatory region covalently linked in a cis configuration to a downstream gene encoding an assayable product; and measuring production of the assayable product. A candidate compound which increases production of the assayable product would be identified as an agent which activates gene or cDNA expression while a candidate compound which decreases production of the assayable product would be identified as an agent which inhibits gene or cDNA expression. See, for example, U.S. Patent Numbers 5,849,493 and 5,863,733.

The screening method may comprise measuring in vitro transcription from a reporter construct in the presence or absence of a candidate compound (the reporter construct comprising a transcription regulatory region) and then determining whether transcription is altered by the presence of the candidate compound. In vitro transcription may be assayed using a cell-free extract, partially purified fractions of the cell, purified transcription factors or RNA polymerase, or combinations thereof. See, for example, U.S. Patent Numbers 5,453,362, 5,534,410, 5,563,036, 5,637,686, 5,708,158 and 5,710,025.

Techniques for measuring transcriptional or translational activity in vivo are known in the art. For example, a nuclear run-on assay may be employed to measure transcription of a reporter gene. Translation of the reporter gene may be measured by determining the activity of the translation product. The activity of a reporter gene can be measured by determining one or more of transcription of polynucleotide product (e.g., RT-PCR of GFP transcripts), translation of polypeptide product (e.g., immunoassay of GFP protein), and enzymatic activity of the reporter protein per se (e.g., fluorescence of GFP or energy transfer thereof).

Another aspect of the invention provides methods of identifying, or predicting the efficacy of, test compounds. In particular, the invention provides methods of identifying compounds which mimic the effects of behavioral therapies. In still another aspect, the systems and methods described herein provide a method for predicting efficacy of a test compound for altering a behavioral response, by obtaining a database, e.g., as described in greater detail above, treating a test animal or human (e.g., a control animal or human that has not undergone other therapies, such as behavioral therapy) with the test compound, and comparing genomic or cDNA expression data of tissue samples from the animal or human treated with the test compound to measure a degree of similarity with one or more differentially DNA-methylated gene profiles in said database. In certain embodiments, the untreated animal or human exhibits a psychological and/or behavioral abnormality possessed by the animals or humans used to generate the database prior to administration of the behavioral therapy.

In another aspect of the invention, a method is provided for predicting efficacy of a test compound for altering a behavioral response in a subject with at least one autism spectrum disorder comprising: (a) preparing a microarray comprising a plurality of different oligonucleotides, wherein the oligonucleotides are specific to differentially DNA-methylated genes associated with an autism spectrum disorder; (b) obtaining a differentially DNA-methylated gene profile representative of the differentially DNA-methylated gene profile of at least one sample of a selected tissue type from a subject subjected to each of at least one of a plurality of selected behavioral therapies which promote the behavioral response; (c) administering the test compound to the subject; and (d) comparing the differentially DNA-methylated gene profile data in at least one sample of the selected tissue type from the subject treated with the test compound to determine a degree of similarity with one or more differentially DNA-methylated gene profiles associated with an autism spectrum disorder; wherein the predicted efficacy of the test compound for altering the behavioral response is correlated to said degree of similarity.

In another aspect, the systems and methods described herein relate to methods of identifying small molecules useful for treating neurological conditions.

For example, in another embodiment a database of differentially DNA-methylated gene profile data representative of the genetic expression response of a selected neuronal tissue type from an animal that was subjected to at least one of a plurality of behavioral therapies and that has undergone a selected physiological change since commencement of the behavioral therapy may be obtained. In an exemplary embodiment, subjects (e.g., subjects that display a preselected behavioral abnormality, such as an autism spectrum disorder neurological condition (including for example autistic disorder, pervasive developmental disorder-not otherwise specified (PDD-NOS), including atypical autism, Asperger's Disorder, Rett's syndrome), Parkinson's disease, parkinsonism, cognitive impairments, age-associated memory impairments, cognitive impairments, dementia associated with neurologic and/or neurological conditions, allodynia, catalepsy, hypernocieption, and epilepsy, brain tumors, brain lesions, multiple sclerosis, Down's syndrome, progressive supranuclear palsy, frontal lobe syndrome, schizophrenia, delirium, Tourette's syndrome, myasthenia gravis, attention deficit hyperactivity disorder, dyslexia, mania, depression, apathy, myopathy, Alzheimer's disease, Huntington's Disease, dementia, encephalopathy, schizophrenia, severe clinical depression, brain injury, Attention Deficit Disorder (ADD), Attention Deficit Hyperactivity Disorder (ADHD), hyperactivity disorder, bipolar manic-depressive disorder, ischemia, alcohol addiction, drug addiction, obsessive compulsive disorders, Pick's disease and Binswanger's disease or a combination thereof), are subjected to behavioral therapy (including, for example, applied behavior analysis (ABA) intervention methods, dietary changes, exercise, massage therapy, group therapy, talk therapy, play therapy, conditioning, or alternative therapies such as sensory integration and auditory integration therapies), and their tissues (including, for example, and not by way of limitation, lymphocytes, blood, or mucosal epithelial cells, brain, spinal cord, heart, arteries, esophagus, stomach, small intestine, large intestine, liver, pancreas, lungs, kidney, urinary tract, ovaries, breasts, uterus, testis, penis, colon, prostate, bone, muscle, cartilage, thyroid gland, adrenal gland, pituitary, bone marrow, blood, thymus, spleen, lymph nodes, skin, eye, ear, nose, teeth or tongue, and/or neurological tissues (including, for example, and not by way of limitation, olfactory bulb cells, cerebrospinal fluid, hypothalamus, amygdala, pituitary, nervous system, brainstem, cerebellum, cortex, frontal cortex, hippocampus, striatum, and thalamus) or a combination thereof are examined for physiological changes (one or more improvements in social interaction, language abilities, restricted interests, repetitive behaviors, sleep disorders, seizures, gastrointestinal, hepatic, and mitochondrial function, neural inflammation, or a combination thereof), and differentially DNA methylation status and/or genetic expression responses are obtained for tissues that have undergone a desired change. In certain embodiments, the subjects are further selected for having undergone a desired change in behavior as well.

From such a database, biological targets for intervention can be identified, such as potential therapeutics (e.g., genes or cDNAs that are upregulated and thus may exert a beneficial effect on the physiology and/or behavior of the subject), potential receptor targets (e.g., receptors associated with upregulated proteins, the activation of which receptors may exert a beneficial effect on the physiology and/or behavior of the subject; or receptors associated with downregulated proteins, the inhibition of which may exert a beneficial effect on the physiology and/or behavior of the subject). In certain embodiments, one or more genes or one or more cDNAs, the expression of which differs by a statistically significant amount in a treated subject as compared to an untreated control, may be selected as targets for intervention.

Small molecule test agents may then be screened in any of a number of assays to identify those with potential therapeutic applications. The term "small molecule" refers to a compound having a molecular weight less than about 2500 amu, preferably less than about 2000 amu, even more preferably less than about 1500 amu, still more preferably less than about 1000 amu, or most preferably less than about 750 amu. For example, subjects or tissue samples may be treated with such test agents to identify those that produce similar changes in expression of the targets, or produce similar gene profiles or DNA methylation profiles, as can be obtained by administration of behavioral therapy. Alternatively or additionally, such test agents may be screened against one or more target receptors to identify compounds that agonize or antagonize these receptors, singly or in combination, e.g., so as to reproduce or mimic the effect of behavioral therapy.

Compounds that induce a desired effect on targets, tissue, or subjects may then be selected for clinical development, and may be subjected to further testing, e.g., therapeutic profiling, such as testing for efficacy and toxicity in subjects. Analogs of selected compounds, e.g., compounds having similar cores but varying substituents and stereochemistry, may similarly be developed and tested. Agents that have acceptable characteristics for therapeutic use in humans or animals may be prepared as pharmaceutical preparations, e.g., with a pharmaceutically acceptable excipient (such as a non-pyrogenic or sterile excipient). Such agents may also be licensed to a manufacturer for development and/or commercialization, e.g., for manufacture and sale of a pharmaceutical preparation comprising said selected agent.

Accordingly, one aspect of the invention provides a method for predicting efficacy of a test compound for altering a behavioral response in a subject with at least one autism spectrum disorder comprising: (a) preparing a microarray comprising a plurality of different oligonucleotides, wherein the oligonucleotides are specific to differentially DNA-methylated genes associated with an autism spectrum disorder; (b) obtaining a differentially DNA-methylated gene profile representative of the differentially DNA-methylated gene profile of at least one sample of a selected tissue type from a subject subjected to each of at least one of a plurality of selected behavioral therapies which promote the behavioral response; (c) administering the test compound to the subject; and (d) comparing differentially DNA-methylated gene profile data in at least one sample of the selected tissue type from the subject treated with the test compound to determine a degree of similarity with one or more differentially DNA-methylated gene profiles associated with an autism spectrum disorder; wherein the predicted efficacy of the test compound for altering the behavioral response is correlated to said degree of similarity.

In one embodiment of the foregoing methods, step (a) comprises obtaining a differentially DNA-methylated gene profile representative of the differentially DNA-methylated gene profile of at least two samples of a selected tissue type referred to supra. In a related embodiment, step (a) comprises obtaining a differentially DNA-methylated gene profile data representative of the differentially DNA-methylated gene profile of at least three samples of a selected tissue referred to supra. In one embodiment in which the more than one sample of a selected tissue type referred to supra is used to determine a differentially DNA-methylated gene profile, the selected tissue types are different tissue types, whereas in other embodiments the tissue types are the same. For example, in an exemplary embodiment, a tissue type may be lymphoblastoid cells and a second tissue type olfactory bulb cells, such that the differentially DNA-methylated gene profile generated from these two tissue samples in the treated subject may be compared to the differentially DNA-methylated gene profiles derived from the subjects subjected to the behavioral therapy. In other embodiments, differentially DNA-methylated gene profiles may be generated from multiple samples of the same tissue type from the same animal, such as blood samples taken at different intervals during the behavioral therapy.

In another embodiment of the foregoing methods, the differentially DNA-methylated gene profile is that shown in Table 1, Table 2, Table 3, or a combination thereof. In another embodiment, the differentially DNA-methylated gene comprises at least 5%, 10%, 20%, 30%, 40%, 50%, 60%, 70%, 80%, 90%, 95% or 98% of the differentially DNA-methylated genes shown in Table 1, Table 2, Table 3, or a combination thereof. In another embodiment, the differentially DNA-methylated gene profile comprises at least 5, 10, 15, 20, 25 or 30 of differentially DNA-methylated genes listed in Table 1, Table 2, Table 3, or a combination thereof. In yet another embodiment of the foregoing methods, the differentially DNA-methylated gene profile comprises an increase or a decrease in expression of at least one to 73 or any integer value thereof, at least one to 201 or any integer value thereof, or at least one to 50 or any integer value thereof, of any of the genes listed in Table 1, Table 2, or Table 3, respectively, or a combination thereof.

In one embodiment of the foregoing methods, the selected tissue type comprises a neuronal tissue type, such as a neuronal tissue type selected from the group consisting of olfactory bulb cells, cerebrospinal fluid, hypothalamus, amygdala, pituitary, nervous system, brainstem, cerebellum, cortex, frontal cortex, hippocampus, striatum, and thalamus. In another embodiment, the selected tissue type is selected from the group consisting of brain, spinal cord, heart, arteries, esophagus, stomach, small intestine, large intestine, liver, pancreas, lungs, kidney, urinary tract, ovaries, breasts, uterus, testis, penis, colon, prostate, bone, muscle, cartilage, thyroid gland, adrenal gland, pituitary, bone marrow, blood, thymus, spleen, lymph nodes, skin, eye, ear, nose, teeth and tongue.

In one embodiment, the behavioral therapy comprises applied behavior analysis (ABA) intervention methods, dietary changes, exercise, massage therapy, group therapy, talk therapy, play therapy, conditioning, or alternative therapies such as sensory integration and auditory integration therapies.

In one embodiment of the foregoing methods, the test subject or animal is a human. In another embodiment, the animal is a non-human animal. Such non-human animals include vertebrates such as rodents, non-human primates, ovines, bovines, ruminants, lagomorphs, porcines, caprines, equines, canines, felines, aves, etc. Preferred non-human animals are selected from the order Rodentia, most preferably mice. The term "order Rodentia" refers to rodents (i.e., placental mammals (Class Euthria) which include the family Muridae (rats and mice). In a specific embodiment, the test animal is a mammal, a primate, a rodent, a mouse, a rat, a guinea pig, a rabbit or a human.

The test compound may be administered to the subject or animal using any mode of administration, including, intravenous, subcutaneous, intramuscular, intrastemal, topical, liposome-mediate, rectal, intravaginal, opthalmic, intracranial, intraspinal or intraorbital. The test compound may be administered once or more than once as part of a treatment regimen. In some embodiments, additional test compounds or agents may be administered to the subject animal to ascertain the efficacy of the test compound or the combination of test compounds or agents. In some embodiments, a differentially DNA-methylated gene profile may also be obtained from the subject or animal prior to treatment with the test agent. In such embodiments, the efficacy of the test agent may be determined by comparing the differentially DNA-methylated gene profile of the subject or animal after treatment with the compound with (a) the differentially DNA-methylated gene profile prior to treatment with the compound and (b) to the differentially DNA-methylated gene profile for the behavioral therapy. For example, if the test compound causes the differentially DNA-methylated gene profile to approach that of said differentially DNA-methylated gene profile, the test compound may be predicted to be efficacious.

It is understood by one skilled in the art that the order of steps (a) and (b) in the foregoing methods may be interchanged i.e. the subject or animal may be treated with the compound prior to obtaining the differentially DNA-methylated gene profile for the behavior therapy. Accordingly, the invention also provides a method wherein step (b) is performed prior to step (a).

When comparing the gene differentially DNA-methylated gene profile data in at least one sample of the selected tissue type from the subject or animal treated with the test compound to determine a degree of similarity with one or more differentially DNA-methylated gene profiles, any number of statistical methods known to one skilled in the art may be used. In some embodiments, a differentially DNA-methylated gene profile may be obtained from samples of a test subject or animal prior to the administration of the test compound or from a control subject or animal to generate a control differentially DNA-methylated gene profile for each of the tissue types of interest. In such embodiments, the differentially DNA-methylated gene profile from the tissue types of the test subjects or animal(s) may be compared to both the control differentially DNA-methylated gene profiles and the differentially DNA-methylated gene profiles resulting from the behavioral therapy to determine to which of these differentially DNA-methylated gene profiles the differentially DNA-methylated gene profile is most similar. If they are more similar to the control differentially DNA-methylated gene profile, the test compound may be considered less efficacious, whereas if it is more similar to the differentially DNA-methylated gene profile of the behavioral therapy then the compound is considered more efficacious.

In one variation of the methods described herein, more than one test compound may be administered to the test subject or animal, such that the efficacy of a combination of test compounds is tested. In another variation, rather than using, or in addition to using, a test compound, a nonchemical test agent is also applied to the subject or animal, such as for example, and not by way of limitation, temperature, humidity, sunlight exposure or any other environmental factor. In yet another environment, the subject or animal is subjected to an invasive or noninvasive surgical procedure, in lieu or in addition to the test compound. In such embodiments, the efficacy of the surgical procedure may be ascertained.

In still yet another aspect, the systems and methods described herein relate to a kit for identifying a compound for treating a behavioral disorder, comprising a database, e.g., as described in greater detail above, and a computer program for comparing differentially DNA-methylated gene profile data obtained from assays wherein a test compound is administered to an untreated subject or animal with differentially DNA-methylated gene profile data in the database and identifying similarity between the differentially DNA-methylated gene profile data from the assays and one or more stored differentially DNA-methylated gene profiles.

In yet another aspect of the invention, the systems and methods described herein relate a kit is provided for identifying a compound for treating at least one autism spectrum disorder comprising (a) a database having information stored therein one or more differentially DNA-methylated gene profiles specific for the differentially DNA-methylated gene listed in Table 1, Table 2, Table 3, or a combination thereof, of subjects that have been subjected to at least one of a plurality of selected autism spectrum disorder neurological therapies and wherein the subject has undergone a desired physiological change; and (b) a computer program for comparing differentially DNA-methylated gene profile data obtained from assays wherein a test compound is administered to a subject with the database and providing information representative of a measure of similarity between the differentially DNA-methylated gene profile data and one or more stored differentially DNA-methylated gene profiles.

Another aspect of the invention provides a method of assessing treatment efficacy in an individual having a neurological disorder comprising determining the expression level of one or more of the aforementioned informative differentially DNA-methylated genes in Table 1, Table 2, Table 3, or a combination thereof at multiple time points during treatment, wherein (i) a decrease in expression of the one or more informative differentially DNA-methylated genes shown to be expressed at increased levels in individuals having a neurological disorder or at risk for developing a neurological disorder as compared with a control, or (ii) an increase in expression of the one or more informative differentially DNA-methylated genes shown to be expressed at decreased levels in individuals having a neurological disorder or at risk for developing a neurological disorder as compared with a control, or both (i) and (ii), is indicative that treatment is effective.

The invention also provides a method of assessing the efficacy of a treatment in an individual having a neurological disorder, comprising (i) determining differentially DNA-methylated gene profile data in a plurality of patient samples, obtained at multiple time points during treatment of the patient, of a selected tissue type; (ii) determining a degree of similarity between (a) the differentially DNA-methylated gene profile data in the patient samples; and (b) a differentially DNA-methylated gene profile produced by a therapy which has been shown to be efficacious in treatment of the neurological disorder; wherein a high degree of similarity is indicative that the treatment is effective. Preferably, the high degree of similarity is a log2 ratio of less than +/- 0.3.

In one embodiment, the invention also provides a method for assessing the efficacy of a treatment in an individual having at least one autism spectrum disorder comprising (a) determining differentially DNA-methylated gene profile data specific for at least one of the differentially DNA-methylated genes set out in Table 1 or Table 2 or a combination thereof, in a plurality of patient samples of a selected tissue type from a patient receiving the treatment; (b) determining a degree of similarity between (i) the differentially DNA-methylated gene profile data in the patient samples; and (ii) a differentially DNA-methylated gene specific for the differentially DNA-methylated genes set out in listed in Table 1, Table 2, Table 3, or a combination thereof, produced by a therapy which has been shown to be efficacious in treatment of the at least one autism spectrum disorder; wherein a high degree of similarity between the differentially DNA-methylated gene profile data of (ii) with the differentially DNA-methylated gene profile data of (a) during or after treatment is indicative that the treatment is effective. Preferably the high degree of similarity is a log 2 ratio of less than +/- 0.3.

Another aspect of the invention provides kits. One aspect provides a kit for identifying a compound for treating a behavioral or neurological disorder, comprising (i) a database having information stored therein differentially DNA-methylated gene profile data representative of the differentially DNA-methylated gene expression response of selected tissue type samples from subjects or animals that have been subjected to at least one of a plurality of selected behavioral therapies and wherein the tissue has undergone a desired physiological change; and (ii) a computer program for (a) comparing differentially DNA-methylated gene profile data obtained from assays, where a test compound is administered to a subject or an animal, with the database; and (b) providing information representative of a measure of similarity between the differentially DNA-methylated gene profile data and one or more stored profiles.

In yet another aspect of the invention, a kit is provided for identifying a compound for treating at least one autism spectrum disorder comprising (a) a database having information stored therein one or more differentially DNA-methylated gene profiles specific for the differentially DNA-methylated genes listed in Table 1, Table 2, Table 3, or a combination thereof, of subjects that have been subjected to at least one of a plurality of selected autism spectrum disorder neurological therapies and wherein the subject has undergone a desired physiological change; and (b) a computer program for comparing differentially DNA-methylated gene profile data obtained from assays wherein a test compound is administered to a subject with the database and providing information representative of a measure of similarity between the differentially DNA-methylated gene profile data and one or more stored gene profiles or DNA methylation profiles.

In some embodiments of the methods described herein, the test compound comprises an antibody or fragment thereof, a nucleic acid molecule, antisense reagent, a small molecule drug, or a nutritional or herbal supplement. Test compounds can be screened individually, in combination with one or more other compounds, or as a library of compounds. In one embodiment, test compounds include nucleic acids, peptides, polypeptides, peptidomimetics, RNAi constructs, antisense oligonucleotides, ribozymes, antibodies, small molecules, and nutritional or herbal supplements or a combination thereof.

In general, test compounds for modulation of neurological disorders, including those autistic spectrum disorders such as autistic disorder, pervasive developmental disorder-not otherwise specified (PDD-NOS), including atypical autism, Asperger's Disorder, or a combination thereof, can be identified from large libraries of natural products or synthetic (or semi-synthetic) extracts or chemical libraries according to methods known in the art. Those skilled in the field of drug discovery and development will understand that the precise source of test extracts or compounds is not critical to the screening procedure(s) of the invention. Accordingly, virtually any number of chemical extracts or compounds can be screened using the exemplary methods described herein. Examples of such extracts or compounds include, but are not limited to, plant-, fungal-, prokaryotic- or animal-based extracts, fermentation broths, and synthetic compounds, as well as modification of existing compounds. Numerous methods are also available for generating random or directed synthesis (e.g., semi-synthesis or total synthesis) of any number of chemical compounds, including, but not limited to, saccharide-, lipid-, peptide-, and nucleic acid-based compounds. Synthetic compound libraries are commercially available, e.g., Chembridge (ChemBridge Corporation, San Diego, Calif.). Alternatively, libraries of natural compounds in the form of bacterial, fungal, plant, and animal extracts are commercially available from a number of sources, including Biotics (Sussex, UK), Xenova (Slough, UK), Harbor Branch Oceanographics Institute (Ft. Pierce, Fla.), and PharmaMar, U.S.A. (Cambridge, Mass.). In addition, natural and synthetically produced libraries are generated, if desired, according to methods known in the art, e.g., by standard extraction and fractionation methods. Furthermore, if desired, any library or compound is readily modified using standard chemical, physical, or biochemical methods.

### Methods of Conducting Drug Discovery

Another aspect of the invention provides methods for conducting drug discovery related to the methods and differentially DNA-methylated gene chips provided herein.

One aspect of the invention provides a method for conducting drug discovery comprising: (a) generating a database of differentially DNA-methylated gene profile data representative of the genetic expression response of at least one selected tissue type (for example, one of the aforementioned neuronal tissue types) from a subject or an animal that was subjected to at least one of a plurality of behavioral therapies and that has undergone a selected physiological change since commencement of the behavioral therapy; (b) selecting at least one differentially DNA-methylated gene profile from Table 1, Table 2, Table 3, or a combination thereof and selecting at least one target as a function of the selected differentially DNA-methylated gene profiles; (c) screening a plurality of small molecule test agents in assays to obtain differentially DNA-methylated gene profile data associated with administration of the agents and comparing the obtained data with the one or more selected differentially DNA-methylated gene profiles; (d) selecting for clinical development test agents that exhibit a desired effect on the target as evidenced by the differentially DNA-methylated gene profiles data; (e) for test agents selected for clinical development, conducting therapeutic profiling of the test compound, or analogs thereof, for efficacy and toxicity in subjects or animals; and (f) selecting at least one test agent that has an acceptable therapeutic and/or toxicity profile.

Another aspect of the invention provides a method for conducting drug discovery comprising: (a) generating a database of differentially DNA-methylated gene profile data representative of the genetic expression response of at least one selected neuronal tissue type from a subject or an animal that was subjected to at least one of a plurality of behavioral therapies and that has undergone a selected physiological change since commencement of the behavioral therapy; (b) administering small molecule test agents to test subjects or animals to obtain differentially DNA-methylated gene profile data associated with administration of the agents and comparing the obtained data with the one or more selected differentially DNA-methylated gene profiles; (c) selecting test agents that induce profiles similar to profiles obtainable by administration of behavioral therapy; (d) conducting therapeutic profiling of the selected test compound(s), or analogs thereof, for efficacy and toxicity in subjects or animals; and (e) identifying a pharmaceutical preparation including one or more agents identified in step (e) as having an acceptable therapeutic and/or toxicity profile.

In one embodiment, the database of differentially DNA-methylated gene profile data representative of the genetic expression response of at least one selected neuronal tissue type from a subject or an animal that was subjected to at least one of a plurality of behavioral therapies and that has undergone a selected physiological change since commencement of the behavioral therapy comprises at least one differentially DNA-methylated gene profile from Table 1, Table 2, Table 3, or a combination thereof

### EXAMPLES

The invention now being generally described, it will be more readily understood by reference to the following examples, which are included merely for purposes of illustration of certain aspects and embodiments of the present invention, and are not intended to limit the invention, as one skilled in the art would recognize from the teachings hereinabove and the following examples, that other DNA microarrays, neurological conditions, cognitive therapies or data analysis methods, all without limitation, can be employed, without departing from the scope of the invention as claimed. The contents of any patents, patent applications, patent publications, or scientific articles referenced anywhere in this application are herein incorporated in their entirety.

### EXAMPLE 1

Global methylation profiling of lymphoblastoid cell lines reveals epigenetic contributions to autism spectrum disorders and a novel autism candidate gene, RORA, whose protein product is reduced in autistic brain

In this example global methylation profiling of discordantly diagnosed monozygotic twins and their nonautistic siblings on CpG island arrays is used to test the hypothesis that differential gene expression in idiopathic autism is, at least in part, the result of aberrant methylation. The study reveals distinct methylation differences in multiple genes between the discordant MZ twins as well as common epigenetic differences distinguishing the twins (the undiagnosed twin exhibiting milder autistic traits that are below the threshold for diagnosis) from nonautistic sibling controls. Overlaying methylation profiles with gene expression profiles previously obtained for these individuals (18) revealed specific differentially expressed ASD candidate genes which are potential targets of aberrant methylation. Immunohistochemical staining of the protein products of two of the candidate genes (Bcl-2 and RORA) which showed increased methylation in LCL revealed protein reduction in autistic cerebellum and frontal cortex as postulated, demonstrating the usefulness of LCL as a surrogate model with which to explore mechanisms of dysregulation of gene expression in ASD.

### Materials and Methods

### Cell culture and 5-Aza-2-deoxycytidine treatment

LCL, which were obtained from the Autism Genetic Research Exchange (AGRE; Los Angeles, CA) were derived from lymphocytes of autistic and normal siblings and cultured as previously described (Hu et al. (2006) BMC Genomics 7, 118). Demethylating treatments were carried out by incubating cells in culture with 5µM 5-Aza-2-deoxycytidine (Fluka, St. Louis, MO) or vehicle control, DMSO (FisherBioTech, Pittsburgh, PA) for 48 hours before harvesting cells.

### DNA and RNA isolation procedures

Genomic DNA was isolated using the Qiagen DNeasy^{™} Blood and Tissue Kit (Valencia, CA) and used for bisulfite modification for methylation analyses. Total RNA was isolated using TRIzol reagent (Invitrogen, Carlsbad, CA) according to the manufacturer's protocol and used for gene expression analyses by quantitative RT-PCR. The RNA samples were further purified using the RNeasy Mini kit (Qiagen, Valencia, CA) and tested for integrity on RNA 6000 NanoChips using an Agilent 2100 Bioanalyzer.

### Selection of samples

Three pairs of male monozygotic twins discordant for diagnosis of autism as determined by the Autism Diagnostic Interview-Revised (ADI-R) assessment instrument were selected for this study. The undiagnosed co-twin from each set, though not clinically autistic, exhibited some autistic traits and were described as either "Broad spectrum" or "Not quite autistic (NQA)" by the AGRE repository based on their stated criteria (www.agre.org). Cell lines derived from respective non-autistic siblings of two pairs of twins were also included in the analyses, in addition to cell lines derived from a set of monozygotic twins unaffected by autism. For confirmation experiments (qRT-PCR, bisulfite sequencing, and methylation-specific PCR), two additional case-control pairs of siblings (affected autistic individual and respective unaffected male siblings) were analyzed. LCL from individuals with specific genetic and chromosomal abnormalities, diagnosed co-morbid disorders, or those born prematurely (<35 weeks of gestation) were excluded from this study.

### Global methylation profiling using CpG island microarrays

Global methylation profiling on CpG island arrays was performed as previously described by Rauch et al. (Rauch et al. (2006) Cancer Res 66, 7939-47) to screen for differentially methylated autism candidate genes. Briefly, 20µg of genomic DNA was digested with the frequent cutter, MseI (5'- TTAA). Two unidirectional linkers: oligo-Long (5'-GCGGTGACCCGGGAGATCTGAATTC-3')(SEQ ID NO: 1) and oligo-MseI (5'- TAGAATTCAGATCTCCCG-3') (SEQ ID NO:2) were annealed and ligated to the MseI digested genomic DNA by incubation at 16°C overnight. Enrichment of the methylated fraction was accomplished by methyl-CpG-island recovery assay (MIRA). Briefly, 1µg of purified GST-tagged MBD2b protein and 1 µg of purified His-tagged MBD3L1 protein were pre-incubated with glutathione beads in the presence of SC110 genomic DNA at 4°C on a rocking platform. The beads were then incubated with 1 µg of the digested and linker-ligated genomic DNA in a binding reaction mixture [10mmol/L Tris-HCl (pH 7.5), 50 mmol/L NaCl, 1 mmol/L EDTA, 1 mmol/L DTT, 3 mmol/L MgCl₂, 0.1% Triton-X100, 5% glycerol, 25 µg/mL bovine serum albumin, and 1.25 µg/mL (dcm minus) SC110 bacterial DNA]. The methylation-enriched MIRA fraction was washed three times with wash buffer [10mM Tris-HCl (pH 7.5), 700mM NaCl, 1mM EDTA, 3mM MgCl₂, 0.1% Triton-X-100], eluted from the glutathione beads using the Qiagen QIAquick^{™} PCR purification kit (Valencia, CA) and PCR-amplified. Enriched and unenriched fractions derived from each individual were then indirectly labeled with aminoallyl-dUTP, conjugated with either Alexa 647 or Alexa 555 (Invitrogen, Carlsbad, CA) and hybridized onto 8.1K CpG island microarrays (UHN Microarray Centre, University of Toronto, Canada). For all paired analyses, a direct comparison was performed in which the methylation-enriched fractions from two individuals were pooled and hybridized onto the same microarray. In addition, indirect comparisons were performed by co-hybridizing the methylation-enriched (MIRA) fraction with the respective unenriched DNA fraction obtained from the same individual. For each paired analysis (between autistic MZ twins and/or between autistic co-twin and unaffected sibling), a total number of 4 replicates were performed, including direct and indirect comparisons.

### Microarray data analysis

Following hybridization and washing according to established protocols, the CpG island microarray slides were scanned using an Axon GenePix^{™} 4000B laser scanner to elicit dye intensities for each element on the array. Analysis of raw intensity data was performed using the TIGR TM4 Software Suite (Quackenbush, J. (2002) Microarray data normalization and transformation. Nature Genetics 32, 496-501). Raw intensity data were normalized and filtered using LOWESS normalization and standard deviation (variance) regularization programs contained within the MIDAS module of TM4. The normalized intensities were then uploaded into the MeV module and a 70% data filter was applied to remove CpG island regions for which log2 intensity values are missing in >30% of the samples before statistical analyses of the data. Differentially methylated CpG islands were identified using the Significance Analysis of Microarrays (SAM) (Tusher et al. (2001) Proc Natl Acad Sci USA 98, 5116-21) module within MeV, which also provided false discovery rates (FDR) for each analysis. A one-class SAM analysis identified differential methylation between discordant monozygotic twins as well as across both twins in comparison to unaffected control siblings.

To identify genes that were differentially expressed in these samples, gene expression data from our earlier expression profiling of LCL from these subjects (Hu et al. (2006) BMC Genomics 7, 118) were downloaded from the Gene Expression Omnibus database (GEO Accession # GSE4187) and analyzed using a 70% data filter and a one-class SAM across all sib pairs. This analysis resulted in the identification of a total of 3043 differentially expressed genes with a FDR of 10%. Microsoft Access software was used to identify the overlap between differentially methylated and differentially expressed genes between the twins and normal siblings, using Gene Symbol as the common identifier between datasets. The log2 values indicating relative expression or methylation for the twins vs. the normal siblings were obtained by averaging the values across all replicates.

### Quantitative PCR analyses (qRT-PCR)

Two candidate genes, BCL-2 and RORA, were chosen for quantitative real-time (RT)-PCR analysis to examine the correlation between expression of mRNA transcript and methylation status as predicted by microarray analysis, as well as to examine the effect of 5-Aza-2-deoxycytidine treatment on transcript levels. Total RNA was reverse transcribed into cDNA using the iScript cDNA Synthesis Kit (Bio-Rad, Hercules, CA) following the manufacturer's protocol, and qPCR was accomplished using Invitrogen's Platinum SYBR Green qPCR SuperMix-UDG with ROX. The qPCR reactions (in triplicate) were analyzed on an ABI Prism 7300 Sequence Detection System and transcript levels of BCL-2 and RORA were quantified and normalized to standard curves obtained using universal 18S rRNA primers. Sequences of primers used for qRT-PCR are given in Table 4.

### Bisulfite modification, bisulfite sequencing and methylation-specific PCR (MSP)

Two ug of genomic DNA isolated from LCL were treated with sodium bisulfite using the Active Motif MethylDetector kit (Carlsbad, CA) according to the manufacturer's specifications. Successful bisulfite treatment and conversion was verified using positive control PCR primers for the p16 (CDKN2A) locus provided in the MethylDetector kit.

For direct bisulfite sequencing of the BCL-2 promoter region, bisulfite-modified DNA was used for a nested PCR amplifying the PI promoter region of BCL-2, which overlapped with the CpG island region (UHNhscpg0002696; chr18:59137439-59137855) contained on the 8.1K CpG island microarray. Successful PCR amplification was verified by gel electrophoresis. PCR products were extracted from the gel and purified using the Qiagen Qiaquick Gel Extraction kit (Valencia, CA) according to the manufacturer's specifications. Purified PCR products were directly sequenced using the Applied Biosystems Automated 3730x1 DNA Analyzer (Cornell DNA Sequencing Facility, Ithaca, NY) with the reverse primer to obtain methylation status of CpG dinucleotides.

For methylation-specific PCR (MSP), 2 sets of primers spanning a region approximately 300 bp upstream of the first exon of RORA (UHNhscpg0014103; chr15:59309160-59309428) were designed according to assumed differences in methylation status (i.e., fully methylated or fully unmethylated). These primers were previously successful in identifying methylation-specific silencing of RORA in several gastric cancer cell lines (Yamashita et al. (2006) Cancer Science 97, 64-71). Bisulfite-modified genomic DNA was used as a template for PCR and PCR products were resolved by agarose gel electrophoresis. Sequences of primers used for bisulfite sequencing analysis of BCL-2 and MSP of RORA are given in Table 4.

### Immunohistochemistry

Two different sets of tissue arrays were obtained through the Autism Tissue Program (San Diego, CA). One set of arrays was prepared by Dr. Charles Eberhart in the Johns Hopkins Pathology Department Tissue Microarray Core Facility, as described previously (Eberhart et al. (2006) Journal of Autism and Developmental Disorders 36, 1131-1135). The samples included thin sections from the cerebellar cortex of 5 autistic, 1 Rett individual and 5 age-matched control individuals. Additional clinical data on each case has been previously reported by Eberhart et al. (Eberhart et al. (2006)). The second set of postmortem tissue arrays are from Dr. Janine LaSalle's laboratory and contained slices from the BA9 region of the frontal cortex from control and age- and sex-matched autistic individuals as well as individuals with a variety of developmental disorders as described by Nagarajan et al. (Nagarajan et al. (2006) Epigenetics 1, 172-182).

Immunohistochemistry for RORA and BCL-2 protein was performed using a standard avidin-biotin complex method of detection described by Eberhart et al. (Eberhart et al. (2006)).. Briefly, tissue array slides were deparaffinized in xylene and rehydrated using a dilution series of 100%, 95% and 75% ethanol. Slides were subjected to heat-induced antigen retrieval by incubation in Tris-EDTA buffer (10nM Tris Base, 1mM EDTA, 0.05% Tween 20, pH 9.0) at 95°C for 25 minutes. Slides were then incubated overnight at 4°C with a rabbit polyclonal primary antibody (1:50 dilution) against RORA (4ug/mL, Santa Cruz Biotechnology: sc-28612) or against BCL-2 (4ug/mL, Santa Cruz Biotechnology: sc-492). Biotinylated secondary goat anti-rabbit antibody (1:200 dilution) was added to slides for 30 minutes at room temperature. All incubations were conducted in a humidified chamber. Antibodies were detected using the Santa Cruz rabbit ABC Staining System using an avidin-biotin complex method and diaminobenzadine (DAB) as a chromagen. Fixed slides were imaged using an Olympus BX-60 upright light microscope.

### Pathway and functional analyses of differentially methylated/expressed genes

Differentially methylated (or expressed) genes were uploaded into either Ingenuity Pathway Analysis (IPA) (Ingenuity Systems, Inc. Redwood City, CA) or Pathway Studio 5 (PS5)(Ariadne Genomics Inc., Rockville MD) to discover relationships between the genes as well as their association with various processes and disorders. The Fisher Exact Test was used to identify significant functions and pathways represented within the respective datasets, with the p value indicating the probability that the stated function or process is not associated with the genes in the dataset.

### Results

Global methylation profiling of LCL from discordant monozygotic twins and unaffected siblings reveals differential methylation of genes relevant to neurological functions and disorders.

CpG island arrays were used to identify differentially methylated genes from LCL of monozygotic twins discordant in diagnosis of autism. Methylation-enriched DNA was isolated from genomic DNA using a pull-down method called methylated CpG island recovery assay (MIRA), previously established by Rauch et al. (Rauch et al. (2006) Cancer Res 66, 7939-47). Methylation-enriched and unenriched DNA fractions were then hybridized onto CpG island microarrays to identify CpG islands with differential methylation patterns between individuals. The CpG island microarray analysis identified 73 CpG islands as differentially methylated between discordant monozygotic twins [Table 1]. Network analyses of the differentially methylated genes immediately at the 3'-end, or overlapping, the CpG islands revealed enrichment of high level functions biologically relevant to the autism phenotype, and included numerous genes involved in neurological disorders as well as nervous system development and function (Table 5). In fact, although only 1 twin of each pair is diagnosed autistic, his co-twin still has autistic traits (which we call "mild"), but falls below the criteria for being diagnosed autistic. Thus, if the combined CpG island data from both co-twins are compared against their respective normal sibs, there are a larger number (201) of differentially methylated CpG islands because the phenotypes involved in the comparisons are distinctly different, even though the genotypes are similar (but not identical) since the twins and their respective nonautistic sibling are genetically related [Table 2]. Network analysis of this set of differentially methylated genes revealed enrichment for high level functions shown in Table 6. Interestingly, the function with the highest statistical significance (p = 9.02 x10⁻⁸ by the Fisher Exact test) includes genes involved in transcriptional control of gene expression, many of which are required for proper brain development. Other significant high level functions represented within the gene dataset are nervous system development and function, neurological disease, and cell death.

### Overlap of differentially methylated genes with differentially expressed genes

To prioritize ASD candidate genes for further analysis, differentially methylated genes from the analysis of twins vs. normal siblings were compared to differentially expressed genes derived from prior gene expression profiling of these same samples on DNA microarrays (Hu et al. 2006). Table 3 lists the genes that are both differentially expressed and methylated, and Figure 1A displays the inverse relationship between expression level and methylation level for all but one of these genes. Network analysis was then performed to examine the relationship between this set of genes and biological processes. As shown in Figure 1B, many of the associated processes within the network, including synaptic regulation, fetal development, morphogenesis, apoptosis, inflammation, digestion, steroid biosynthesis, and mental deficiency, have been associated with autism. Two genes from this network, BCL-2 and RORA, were selected for further study because of their respective roles in apoptosis and morphogenesis/inflammation. Interestingly, BCL-2 protein has been demonstrated to be reduced in the cerebellum and frontal cortex of autistic subjects relative to control subjects, but RORA, a nuclear steroid hormone receptor and transcriptional activator that is involved in Purkinje cell differentiation (Boukhtouche et al. (2006) Cerebellum 5, 97-104) and cerebellar development (Gold et al. (2007) Brain Research 1140, 19-25), has never before been implicated in autism. In addition, RORA, a regulator of circadian rhythm, is also neuroprotective against inflammation and oxidative stress.

### Confirmation of differential methylation of specific CpG sites in upstream promoter regions of BCL-2 and RORA.

The CpG islands immediately upstream of BCL-2 and RORA were chosen for further confirmation of methylation status in LCL of autistic and control individuals. Specifically, a 417bp CpG island overlapping the PI promoter region of the BCL-2 gene (chr18:59137439-59137855) was identified as hypermethylated in all co-twins exhibiting autistic traits compared to unaffected siblings in the CpG island microarray, and a 269bp CpG island associated with the 5' end of the RORA1 isoform (chr15:59309160- 59309428) was identified as hypermethylated in the same analysis. To obtain high-resolution information regarding which specific CpG sites may be hyper-methylated in autistic individuals, 2 separate experimental approaches taking advantage of bisulfite modification of genomic DNA were employed: bisulfite sequencing and methylation-specific PCR. Bisulfite modification of genomic DNA results in deamination of all unmethylated cytosine residues to uracils, while methylated cytosine residues are protected against conversion.

Figure 2 displays the results from bisulfite sequencing of the BCL-2 promoter region after bisulfite treatment of genomic DNA isolated from LCL derived from the monozygotic twins, their unaffected siblings, an additional autistic/unaffected sib pair (A_2020/C_2019), and a pair of unaffected monozygotic twins (C_2744/C_2745). Following bisulfite conversion, nested primers were designed to amplify across the PI promoter region of BCL-2 (Fig. 2A). This is the same region interrogated by the CpG island microarrays and spans 38 total CpG sites. As shown in Figure 2B, an overall increase in % methylation across the 38 CpG sites (~25% on average) was observed in the diagnosed autistic samples in comparison to an average of 11.4% for the undiagnosed co-twins and 9.5% for the unaffected siblings. Increased methylation was especially noted on CpG sites #3-13 for autistic individuals only. Also, there appears to be a decrease in methylation at site 34 in the autistic samples that is found methylated in 4 out of 5 control individuals. Interestingly, the same methylated CpG sites are not observed across all autistic subjects (with the exception of CpG site #12), suggesting that regulation is not dependent upon specific sites, but instead may involve the entire region encompassed by CpG sites #3-13.

Methylation-specific PCR (MSP) was utilized to analyze methylation in a region upstream of RORA because of the presence of homopolymer sequences between the CpG sites of interest which would complicate sequencing analyses. This region overlaps with the region interrogated by the CpG island microarray, and is located approximately 300 bp before the first exon of the RORct1 isoform (Fig. 3A). Two sets of primers encompassing several CpG sites were designed according to assumed differences in methylation status to amplify the same region upstream of RORA. These particular primers were previously successful in identifying epigenetic silencing of RORA in several gastric cancer cell lines (Yamashita et al. (2006) Cancer Science 97, 64-71). The presence of a PCR product corresponding to methylated DNA only in the vehicle-treated samples of autistic individuals in contrast to those of the unaffected controls (Fig. 3B) confirms methylation of the RORA promoter region only in the autistic individuals. The absence of methylated product in the autistic samples following treatment with 5µM 5-Aza-2-deoxycytidine further confirms methylation in the untreated autistic samples and specificity of the methylated PCR primers.

### Confirmation of methylation-dependent silencing of candidate genes identified by CpG island arrays

Quantitative RT-PCR was used to confirm decreased expression of BCL-2 and RORA in autistic samples and to evaluate the effect of a global methylation inhibitor, 5- Aza-2-deoxycytidine, on gene expression. For both BCL-2 and the RORA, gene expression was significantly higher (p< 0.05) in the unaffected control than autistic co-twins (Fig 4A). Generally, the diagnosed autistic co-twin (_A) had the lowest level of expression of BCL-2 and RORA, while the milder undiagnosed co-twin (_M) exhibited transcript levels between that observed for unaffected sibling controls and autistic co-twins. This suggests a quantitative relationship between phenotype and gene expression of these two genes, although additional studies are required to confirm this observation. BCL-2 and RORA gene expression was found to be methylation-dependent as both transcripts were up-regulated by 1.5- and 1.8-fold, respectively, following global inhibition of methylation with 5-Aza-2-deoxycytidine (Fig. 4B). By contrast, there were no statistically significant increases in the expression levels of BCL-2 and RORA in the LCL from the "mild" co-twins with 5-Aza-2-deoxycytidine treatment.

### RORA and BCL-2 proteins are also reduced in post-mortem brain tissues of autistic subjects

To explore the relevance of our findings of enhanced methylation and reduced expression of RORA and BCL-2 in LCL to the expression of these proteins in the brain, the levels of RORA and BCL-2 protein were investigated in post-mortem tissues from autistic subjects and age- and sex-matched controls, using tissue arrays containing sections from both the cerebellum and frontal cortex (Yamashita et al. (2006) Cancer Science 97, 64-71; Fatemi et al. (2001) J Autism Dev Disord 31, 529-35). Immunohistochemical staining of the tissue arrays confirmed reduced levels of RORA protein in the cerebellum of autistic individuals, including the molecular cell, granule cell and Purkinje cell layers, where expression of RORA has been previously demonstrated (Gold et al. (2007) Brain Research 1140, 19-25; Hadj-Sahraoui et al. (2001) Developmental Brain Research 126, 201-209; Doulazmi et al. (1999) Journal of Comparative Neurology 411, 267-273). Representative immunohistochemical staining results were obtained for RORA protein in cerebellum of autistic subjects in comparison to that of respective age- and sex-matched controls (data not shown). Immunoreactivity was decreased in most autistic samples (male: A, C, I and female: E, G) as indicated by the decreased extent of RORA staining. As reduced expression of RORA (as well as other regulators of the circadian rhythm) was observed only in the subtype of ASD exhibiting severe language impairment (Hu et al. (2009) Autism Research 2, 78-97), reduced RORA protein in all autistic brain samples are not expected.

Immunohistochemistry of post-mortem brain tissue also revealed decreased BCL-2 protein in the granule cell and Purkinje cell layers of the cerebellum, with 3 out of 5 autistic samples showing reduced immunoreactivity for BCL-2 protein (data not shown). Higher magnification representative images of the cerebellar sections show that both RORA and BCL-2 protein are specifically reduced in Purkinje cells (Fig. 5). RORA protein was observed in both the cytoplasm and nucleus, as reported previously for different isoforms of RORA (Giguere et al. (1994) Genes and Development 8, 538-553), while counterstaining with hematoxylin revealed that BCL-2 is confined to the cytoplasm. Figure 6 shows that RORA is also reduced in the majority autistic tissue sections vs. age- and sex-matched control sections from the frontal cortex (BA9 region), suggesting that RORA deficiency is not limited to the cerebellum.

In this study, we used microarray-based epigenomic profiling of LCL from discordant monozygotic autistic twins and unaffected siblings to test the hypothesis that DNA methylation is involved in global dysregulation of gene expression in ASD and to screen for candidate genes whose expression is potentially linked to aberrant DNA methylation. We observed DNA-methylation differences in numerous loci containing genes important to the pathobiology of autism. In particular, functional and pathway analyses of the differentially methylated/expressed genes showed enrichment of genes involved in inflammation and apoptosis, cellular differentiation, brain morphogenesis, growth rate, cytokine production, myelination, synaptic regulation, learning, and steroid biosynthesis, all of which were altered in ASD. The candidate genes were prioritized for further analyses by identifying the overlap between the differentially methylated genes and those that had been shown to be differentially expressed in the same set of samples in previous gene expression analyses. Pathway analyses of this filtered set of genes thus focused our attention on 2 genes, BCL-2 and RORA, as candidate genes for ASD whose expression were dysregulated by aberrant methylation.

In this study, global methylation profiling of LCL from phenotypically discordant monozygotic twins and nonautistic siblings highlighted the role of epigenetic regulation in idiopathic autism and reveals DNA methylation as a mechanism through which this regulation may occur. Two candidate genes, BCL-2 and RORA, which were identified from our coupling of methylation with gene expression data, exhibited increased methylation of specific CpG sites in respective upstream regulatory CpG islands that coincided with methylation-specific gene silencing. Translation of these findings from LCL to the detection of decreased BCL-2 and RORA protein in post-mortem brain tissues of autistic individuals further confirms the feasibility of using LCL as a surrogate model for autism, particularly when investigating dysregulated genes with systemic functions, such as apoptosis and circadian rhythm. In addition to identifying key autism candidate genes, these studies also yield further insight into the pathobiology of this complex disorder by elucidating global epigenomic modifications relevant to the autistic phenotype. Aside from shedding light on higher order regulation of gene expression, another compelling reason to investigate epigenetic mechanisms in idiopathic autism is that such modifications can be influenced by exposure to biological modulators and environmental factors. Epigenetics may thus mediate the interaction between genotype and intrinsic (biologic) and extrinsic (environmental) factors contributing to ASD.

### BCL-2 as a candidate gene for autism

BCL-2 is an anti-apoptotic protein located in the outer mitochondrial membrane that is important for cell survival under a variety of stressful conditions. Our data provides evidence of the involvement of reduced BCL-2 in ASD. Moreover, our study provides new evidence that increased DNA methylation of the PI promoter region of BCL-2 is a possible mechanism for increased gene silencing and decreased protein abundance of BCL-2 in the autistic brain. These studies also localized the changes in BCL-2 to Purkinje cells in the cerebellum.

### RORA is a novel candidate gene for autism

RORA is a member of the NR1 subfamily of nuclear hormone receptors and is involved in transcriptional regulation of many genes. Among its many relevant functions are regulation of the circadian clock by activation of BMAL1, neuroprotection in the face of oxidative stress and inflammation, survival and differentiation of Purkinje cells, and cerebellar development. Multiple regulators of circadian rhythm, including RORA, were aberrantly expressed in LCL from autistic individuals with severe language impairment. We have confirmed the methylation and expression differences of RORA in LCL, and the involvement of RORA in autistic brain pathology.

As shown in Figs. 3 and 4 respectively, RORA was confirmed to be inversely differentially methylated and expressed in LCL from autistic vs. nonautistic siblings, with expression dependent on methylation, as demonstrated by the absence of methylation in the presence of 5-Aza-2-deoxycytidine. We have also shown by immunohistochemical staining of cerebellar and frontal cortex regions of autistic vs. normal brain (Fig. 6), that RORA protein is noticeably reduced in the majority of the autistic samples relative to age- and sex-matched controls. This reduction was also specifically demonstrated in Purkinje cells which are dependent on RORA for both survival and differentiation (Fig. 5). These findings thus linked molecular changes identified in a peripheral cell model of ASD to actual pathological changes in the autistic brain, suggesting that LCL was an appropriate surrogate for studies on autism.

### Role of DNA methylation as an epigenetic mechanism for dysregulation of gene expression in autism

The broad functions of the methylation-regulated differentially expressed genes identified in this study provided support for our findings that the pathological symptoms of autism were in part the result of systemic dysregulation of gene expression. Bioinformatic analysis of the differentially methylated genes from the CpG island assays (Tables 5 and 6) revealed enrichment for many high level functions implicated in autism including aberrant response to inflammation, decreased cell survival, and abnormal cellular differentiation leading to abnormal brain development. In addition, these processes reiterated common biological themes in autism that our laboratory has identified from global gene expression profiling of LCL in discordant monozygotic twins (Hu et al. 2006), sibling pairs (Hu et al. (2009) PLoS ONE4, e5775), and large scale case-control studies of unrelated subjects (Hu et al. (2009) Autism Research 2, 78-97).

Coupling methylation profiles with global gene expression profiles of the same autistic and control individuals provided a means of identifying high-priority candidate genes for autism. Confirmation of the postulated decrease in protein products of two of the candidate genes in post-mortem brain tissue of autistic individuals highlights the relevance of our findings in LCL to the autistic brain. This ability to translate findings from peripheral tissues to the brain is consistent with reports of individuals with neuropsychiatric disorders having detectable epigenetic programming disturbances, both in the brain and in secondary tissues. LCL and primary blood lymphocytes have been demonstrated to be reasonable surrogate experimental models of autism to identify both expressed and epigenetic alterations that may also have diagnostic potential as biomarkers for autism (see, e.g., Baron et al. (2006) J Autism Dev Disord 36, 973-82; Nishimura et al. (2007) Hum Mol Genet 16, 1682-98; Gregg et al. (2008) Genomics 91, 22-29, and; Enstrom et al. (2009) Brain, Behavior, and Immunity 23, 124-133).

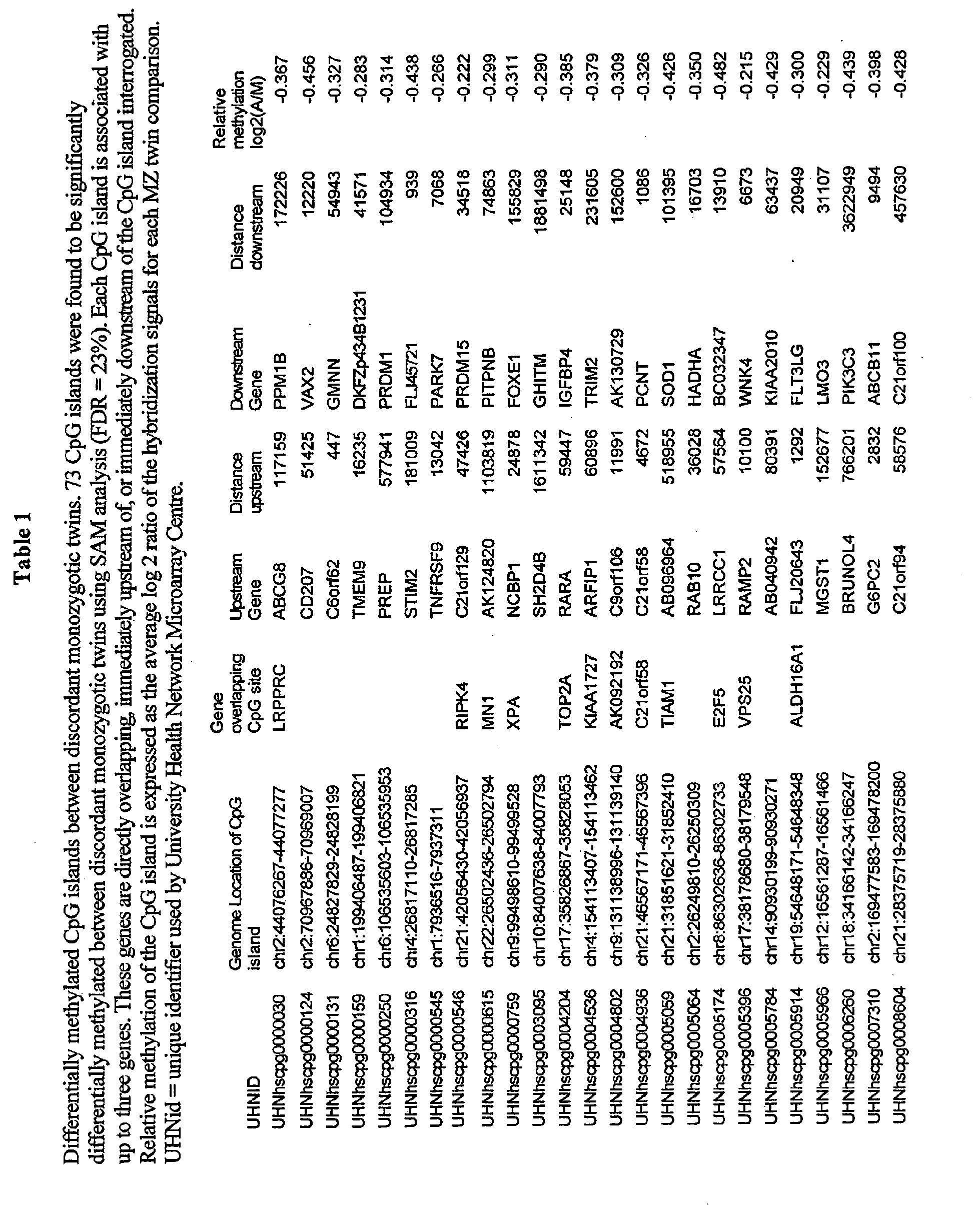

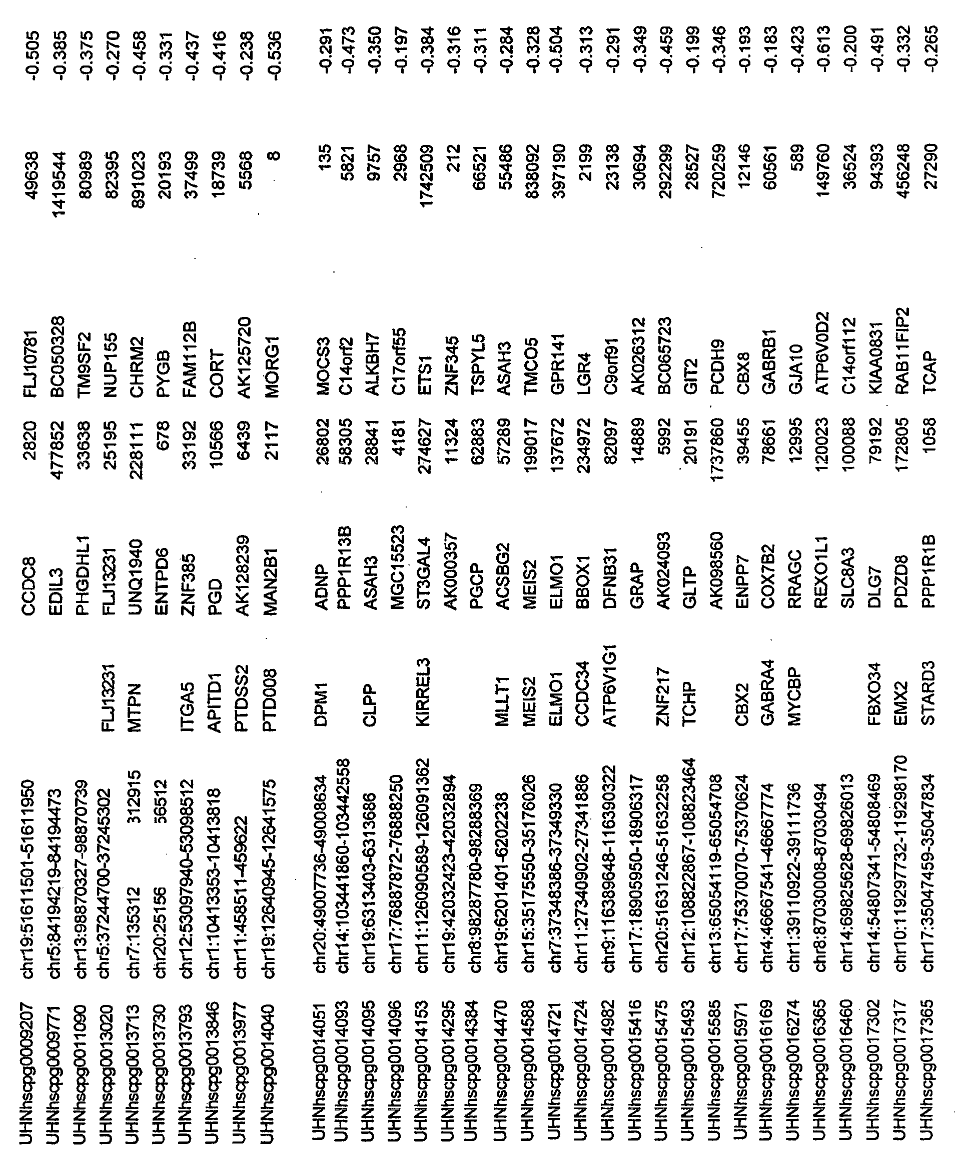

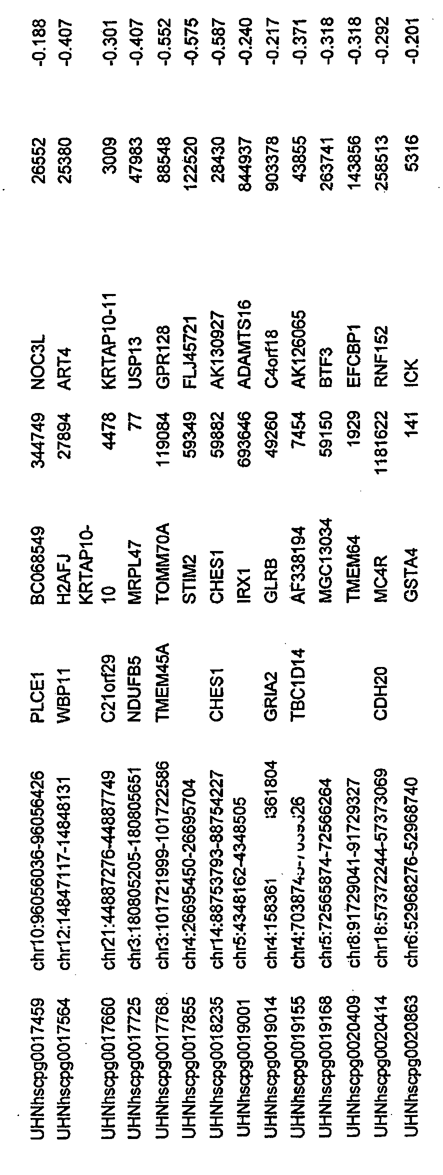

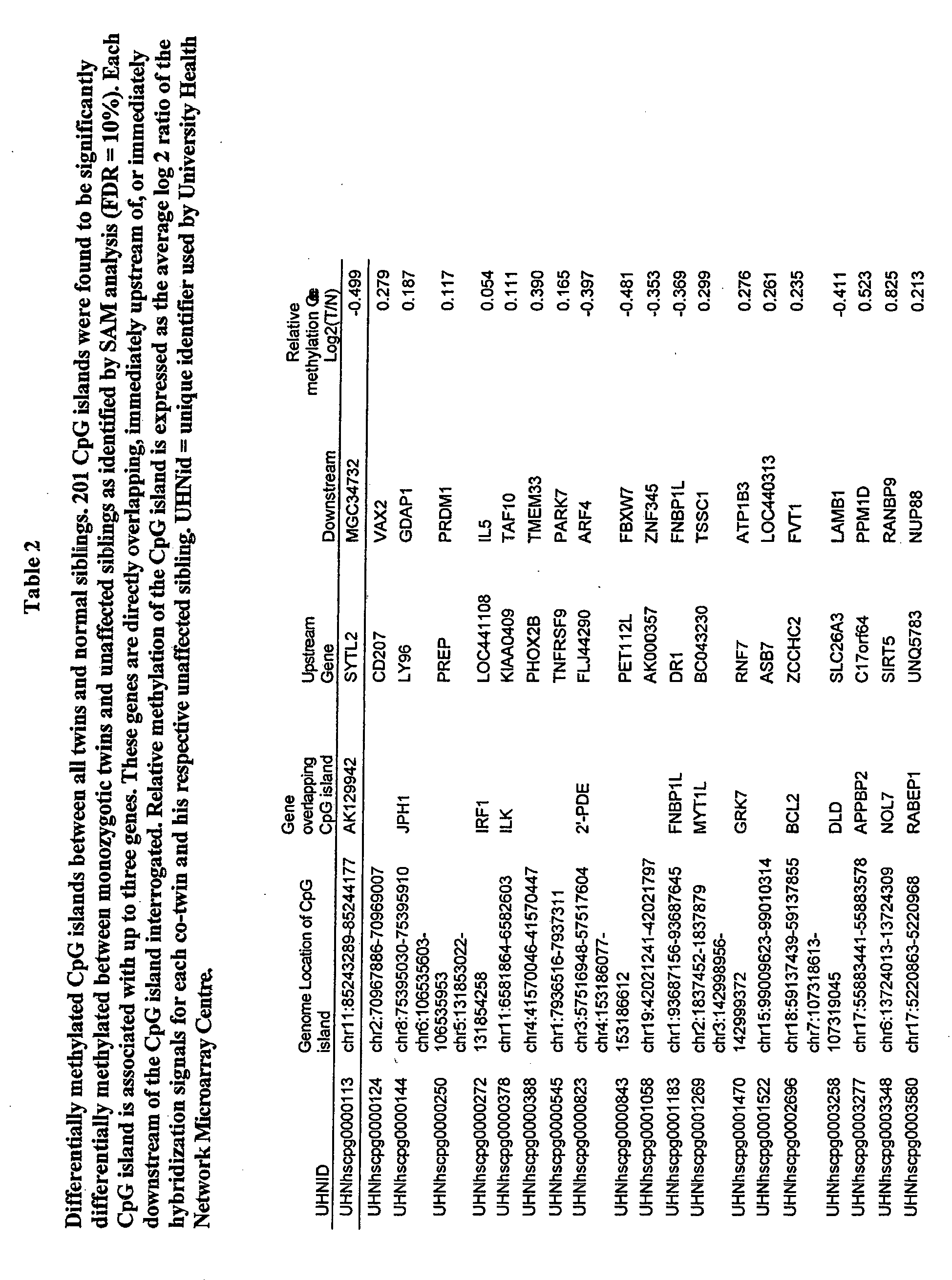

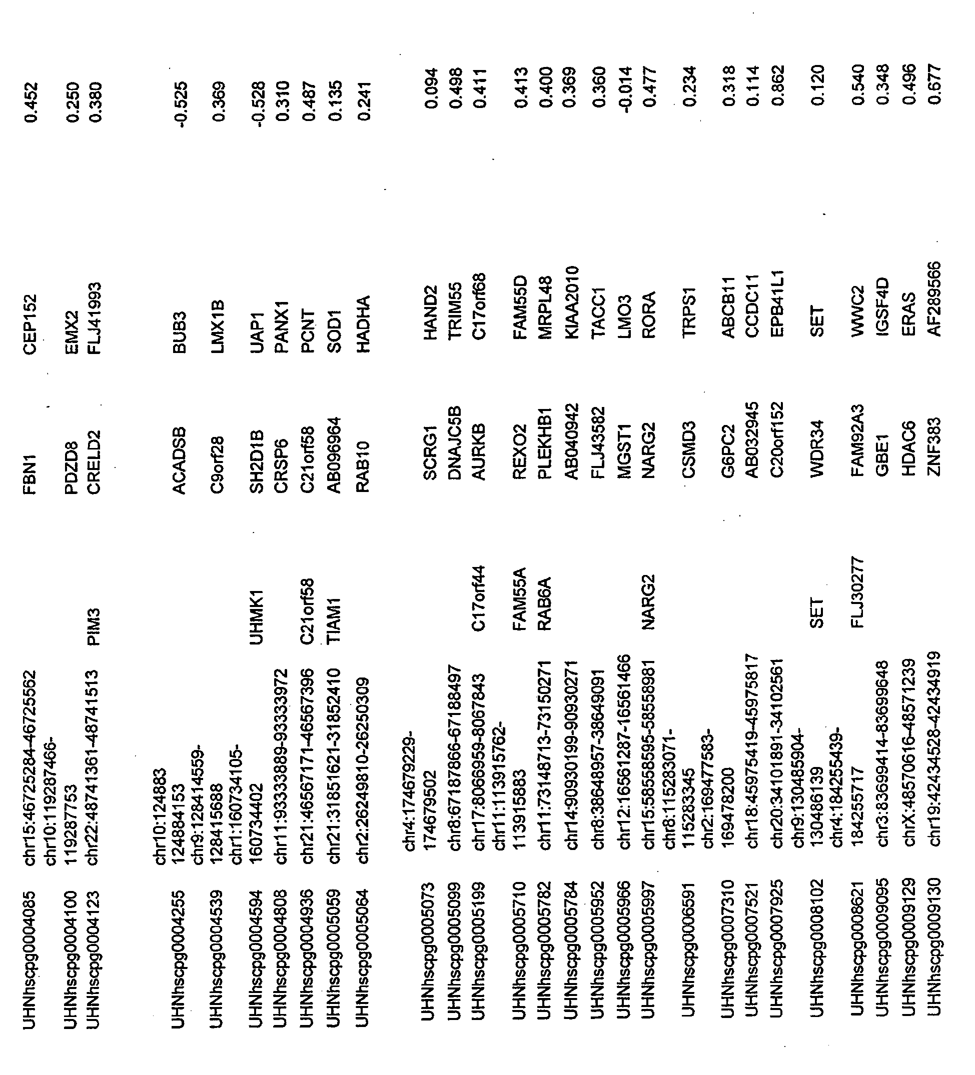

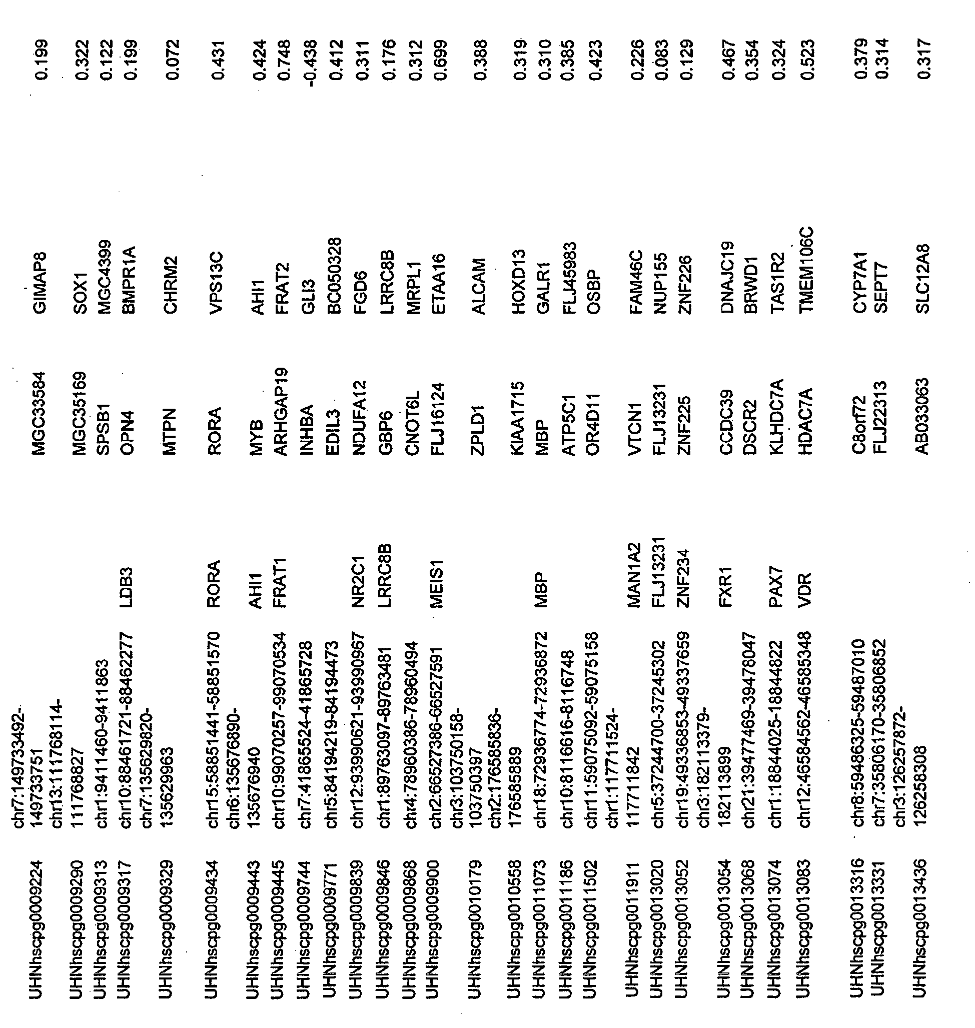

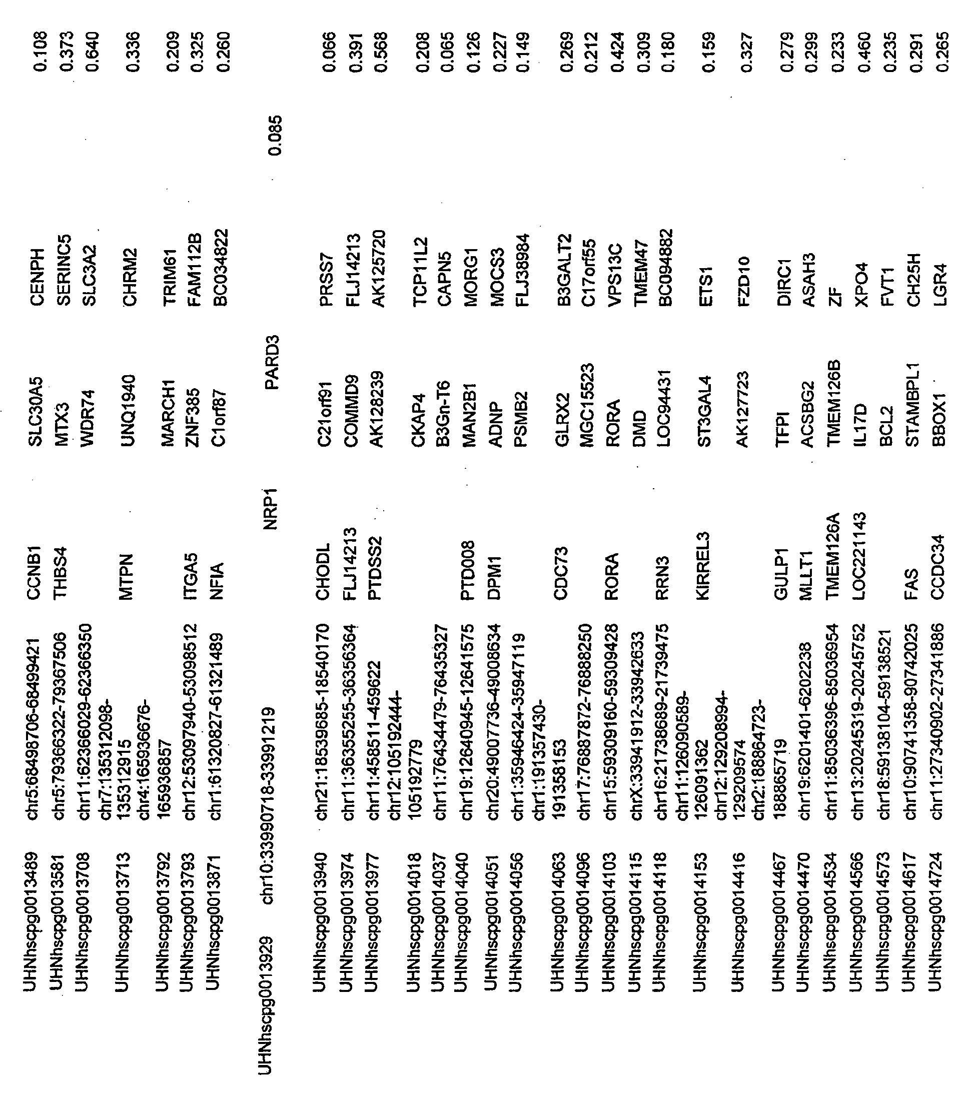

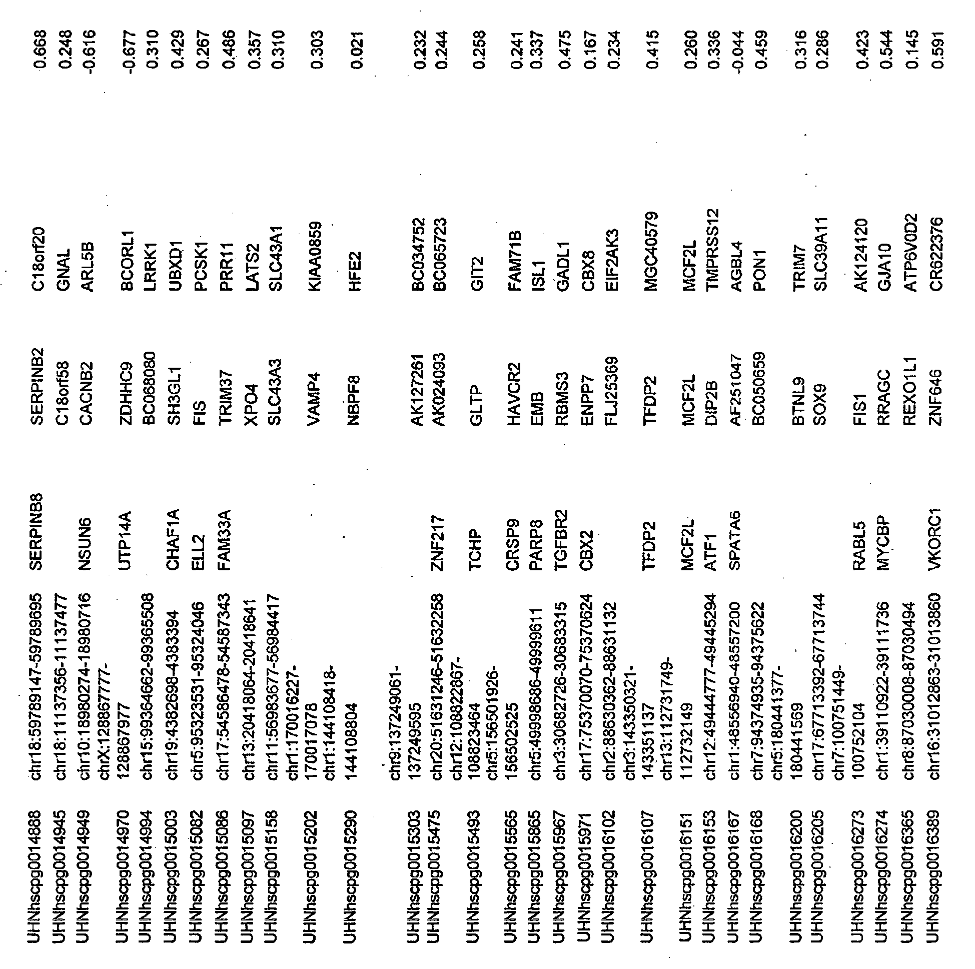

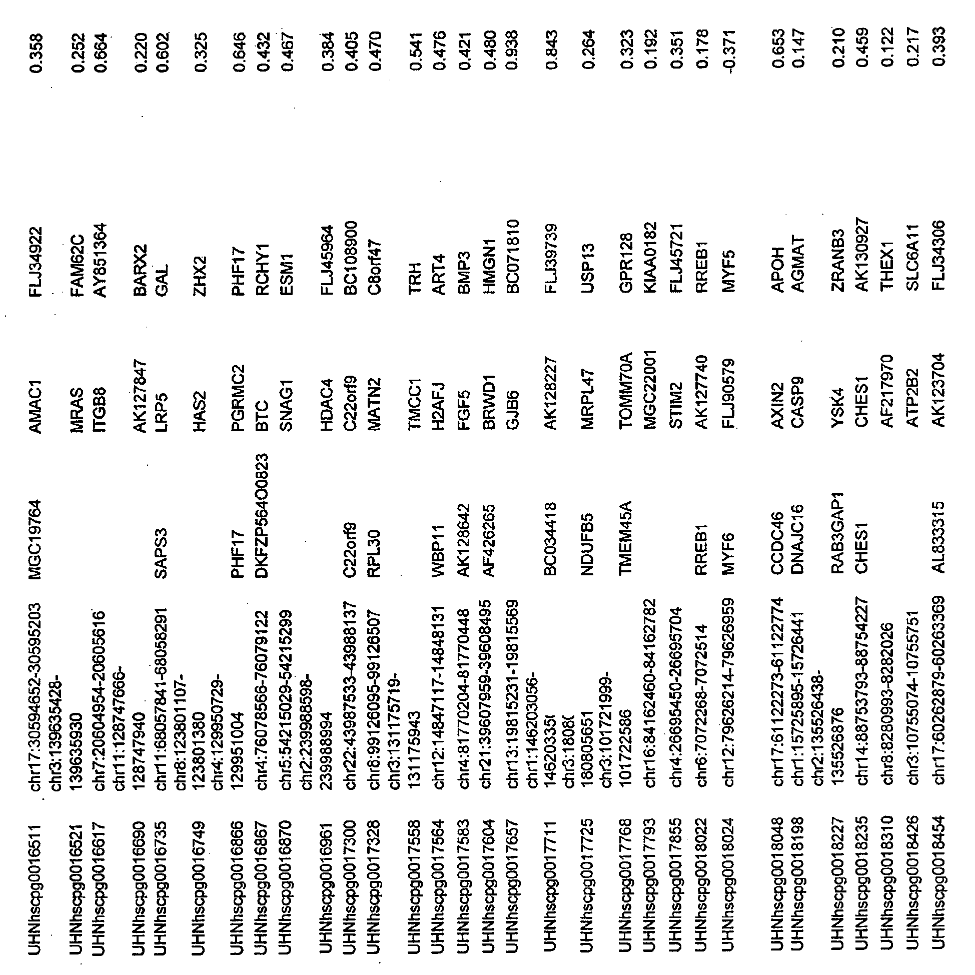

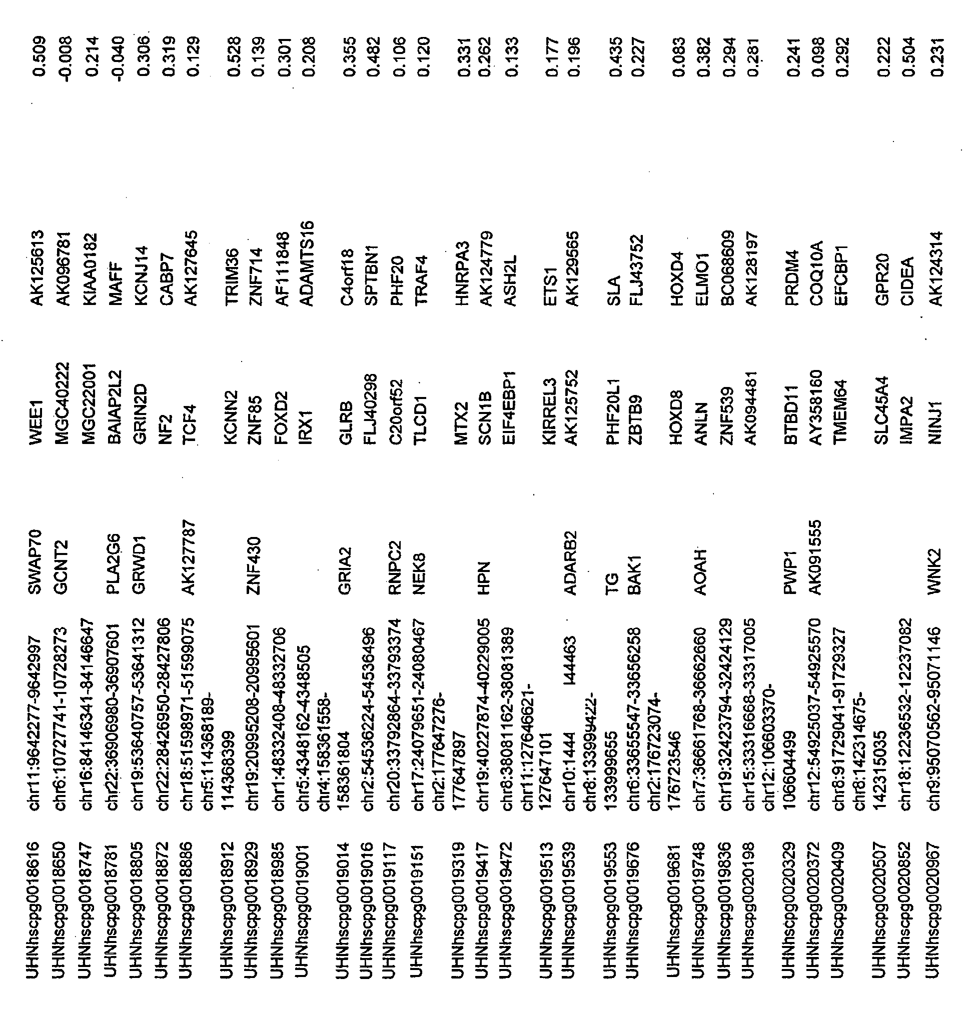

**Table 3**

| | | | | | | |
|---|---|---|---|---|---|---|
| | Overlap of 50 dysregulated genes identified from the CpG island microarray analysis and global gene expression profiling previously performed using the same samples. Log2 ratios of expression values were obtained from gene expression data (GEO accession # GSE4187) and averaged across all replicates corresponding to the same samples used for CpG island microarray analysis. CpG methylation log2 ratios were calculated as described in Methods. Genes both upstream and downstream of the CpG island were included in the overlap analysis, in addition to genes that had direct overlap with the differentially methylated CpG island. | | | | | |
| | | | | | | |
| R96310 | 2'-PDE | 2'-phosphodiesberase | + | 0 | -0.397 | -0.131 |
| T71316 | ARF4 | ADP-ribosylation factor 4 | - | 14525 | -0.416 | -0.207 |
| AA425722 | ARHGAP19 | Rho GTPase activating protein 19 | - | 27854 | 0.748 | -0.130 |
| H43438 | ARHGAP19 RHO GTPASE ACTIVATING PROTEIN 19 | | - | 27854 | 0.748 | -0.153 |
| W63749 | BCL2 | B-cell CLL/lymphoma 2 | - | 511 | 0.235 | -0.286 |
| N27448 | BMPR1A | BONE MORPHOGENETIC PROTEIN RECEPTOR, TYPE IA | + | 44098 | 0.234 | 0.405 |
| AA670419 | C22orf9 | chromosome 22 open reading frame 9 | - | 0 | 0.405 | -0.160 |
| AA670419 | C22orf9 | chromosome 22 open reading frame 9 | - | 522 | 0.405 | -0.160 |
| W20354 | CRSP6 | cofactor required for Sp1 transcriptional activation, subunit 6,77kDa | + | 147787 | 0.310 | -0.154 |
| AA918102 | DPM1 | dolichyl-phosphate mannosyltransferase polypeptide 1, catalytic subunit | - | 0 | 0.227 | -0.144 |
| AA252470 | DR1 | down-regulator of transcription 1, TBP-binding (negative cofactor 2) | + | 86422 | -0.369 | 0.201 |
| AA436178 | EIF2AK3 | eukaryotic translation initiation factor 2-alpha kinase 3 | - | 6243 | 0.249 | -0.062 |
| AA418818 | ELL2 | elongation factor, RNA polymerase II, 2 | - | 0 | 0.267 | -0.245 |
| T87150 | ELI2 | ELONGATION FACTOR, RNA POLYMERASE II, 2 | - | 0 | 0.267 | -0.348 |
| AA058597 | FAM46C | family with sequence similarity 46, member C | + | 238284 | 0.157 | -0.924 |
| W46575 | FGF5 | FIBROBLAST GROWTH FACTOR 5 | + | 339010 | 0.421 | -0.157 |
| H80525 | FLJ34922 | hypothetical protein FLJ34922 | - | 106241 | 0.281 | -0.210 |
| AA430662 | FLJ38984 | hypothetical protein FLJ38984 | - | 4944 | 0.207 | -0.284 |
| AA927951 | FXR1 | fragille X mental retardation, autosomal homolog 1 | + | 0 | 0.467 | -0.238 |
| AI623173 | GAL | GALANIN | + | 150319 | 0.546 | -0.092 |
| | | | | | | |
| | | glucan (1,4-alpha-), branching enzyme 1 (glycogen branching enzyme, Andersen R09069 GBE1 disease, glycogen storage disease type IV) | | | | |
| | | | - | 1805979 | 0.348 | -0.309 |
| | | | + | 137848 | 0.346 | -0.210 |
| AA496097 | HNRPA3 | heterogeneous nuclear ribonucleoprotein A3 | + | 1391174 | 0.383 | -0.199 |
| N62301 | IGSF4D | immunoglobulin superfamily, member 4D | - | 10572 | 0.319 | -0.122 |
| AA101146 | KIAA1715 | KLkA1715 | | | | |
| | | LATS, large tumor suppressor, homolog2 | - | 27937 | 0.334 | 0.105 |
| AA431967 | LATS2 | (Drosophila) low density lipoprotein receptor-related R83038 | + | 84524 | 0.602 | -0.173 |
| | LRP5 | protein 5 | + | 14686 | 0.262 | 0.247 |
| AI240161 | LRRK1 | leucine-rich repeat kinase 1 | + | 14686 | 0.262 | 0.149 |
| R51605 | LRRK1 | leucine-rich repeat kinase 1 | + | 0 | 0.226 | -0.098 |
| AA455062 | MAN1A2 | mannosidase, alpha, class 1A, member 2 | + | 0 | 0.226 | -0.165 |
| R22905 | MAN1A2 | mannosidase, alpha, class 1A, member 2 | - | 412024 | 0.209 | -0.089 |
| AI077321 | MARCH1 | membrane-associated ring finger (C3HC4) 1 | - | 4181 | 0.212 | -0.149 |
| R46332 | MGC15523 | HYPOTHETICAL PROTEIN MGC15523 | | | | |
| AA495936 | MGST1 | microsomal glutithione S-transferase 1 | + | 152677 | -0.014 | 0.426 |
| AA626777 | MRAS | muscle RAS oncogene homolog | + | 28363 | 0.252 | -0.188 |
| AA099873 | MYCBP | c-myc binding protein | - | 0 | 0.544 | -0.183 |
| AA098867 | NRP1 | neuropilin 1 | - | 326879 | 0.085 | -0.185 |
| AA464606 | PANX1 | pannexin 1 progesterone receptor membrane component | + | 167769 | 0.365 | -0.151 |
| AA047567 | PGRMC2 | | - | 522022 | 0.646 | -0.133 |
| AA136664 | PHF17 | PHD finger protein 17 | + | 926 | 0.671 | -0.105 |
| AA505136 | PHF17 | PHD finger protein 17 | + | 926 | 0.671 | -0.116 |
| AA136664 | PHF17 | PHD finger protein 17 | + | 0 | 0.646 | -0.105 |
| AA505136 | PHF17 | PHD finger protein 17 | + | 0 | 0.646 | -0.116 |
| A1015442 | PHF20L1 | PHD finger protein 20-like 1 | + | 70138 | 0.435 | -0.131 |
| N54157 | PSMB2 | PROTEASOME (PROSOME, MACROPAIN) SUBUNIT, BETA TYPE, 2 | - | 66694 | 0.149 | -0.155 |
| AA485992 | PWP1 | nuclear phosphoprotein similar to S. cerevisiae PWP1 | + | 0 | 0.241 | -0.116 |
| H95393 | RAB6A | RAB6A, member RAS oncogene family | - | 0 | 0.400 | -0.138 |
| N71050 | RORA | RAR-RELATED ORPHAN RECEPTOR A | - | 17774 | 0.572 | -0.181 |
| N71050 | RORA | RAR-RELATED ORPHAN RECEPTOR A | - | 0 | 0.120 | -0.181 |
| N71050 | RORA | RAR-RELATED ORPHAN RECEPTOR A | - | 144417 | 0.120 | -0.181 |
| AA485141 | SLA | Src-like-adaptor | - | 118500 | 0.451 | -0.094 |
| AA404709 | SLC43A3 | solute carrier family 43, member 3 | - | 32578 | 0.310 | -0.131 |
| AA453898 | ST3GAL4 | ST3 beta-galactoside alpha-2,3-sialyltransferase 4 | + | 274627 | 0.159 | -2.157 |
| AA432278 | THBS4 | thrombospondin 4 | + | 0 | 0.373 | -0.103 |
| AA676378 | TMCC1 | transmembrane and coiled-coil domains 1 | - | 93720 | 0.541 | 0.120 |
| N68465 | UAP1 | UDP-N-acteylglucosamine pyrophosphorylase 1 | + | 63544 | -0.471 | -0.234 |
| N51629 | VAMP4 | vesicle-associated membrane protein 4 | - | 38390 | 0.303 | -0.090 |
| AA485226 | VDR | vitamin D (1,25-dihydroxyvitamin D3) receptor | - | 0 | 0.523 | -0.081 |
| AA625788 | VKORC1 | vitamin K epoxide reductase complex, subunit 1 | - | 0 | 0.591 | -0.094 |
| AA130669 | WBP11 | WW domain binding protein 11 | - | 0 | 0.476 | -0.122 |
| AA678088 | XPO4 | exportin 4 | - | 5262 | 0.495 | -0.077 |
| AA678088 | XPO4 | exportin 4 | - | 43180 | 0.357 | -0.077 |

**Table 4 Primer sequences**

| Gene Symbol | Application | Forward Primer (5' --> 3') | Reverse Primer (5' --> 3') |
|---|---|---|---|
| BCL2 | qPCR | TATTGTGGCTGCACTTGCTC | TGTTGCCCAACTGCAAAATA |
| RORA | qPCR | ACACCAGCATCAGGCTTCTT | GGTCTGCCACGTTATCTGCT |
| BCL2 | Bisulfite sequencing Nested PCR- Outer | GGTGGTTTAGAGGAGGGTTTTT | ACAAAAATCCTCTTCTAATTAAACTC |
| BCL2 | Bisulfite sequencing Nested PCR- Inner | GAGAATGAAGTAAGAGGATAGGT | CACCCTTTCTCCTCCTCCTAATC |
| RORA | MSP: Methylated | GGTTGGAGAAGTTTTCGTTAGC | GACGAACGAACAAACAAAAACG |
| | MSP: Unmethylated | TTGGTTGGAGAAGTTTTTGTTAGT | CAAACAAAAACACAAAAAAACACA |

| | | | |
|---|---|---|---|
| Forward Primers SEQ ID NOS: from top to bottom: 3, 5, 7, 9, 11, 13 Reverse Primers SEQ ID NOS: from top to bottom: 4, 6, 8, 10, 12, 14 | | | |

**Table 5: Four high level functions associated with genes differentially methylated between discordant monozygotic twins and identified by CpG island microarray. P values represent significance by Fisher Exact Test and indicates the probability that the indicated function is not associated with the dataset of differentially methylated genes (Ingenuity Pathway Analysis software).**

| **Category (# genes)** | **Function Annotation** | **Molecules** | **p-value** |
|---|---|---|---|
| **Neurological Disease** | Huntington's disease | ELMO1, EMX2, FOXN3, GABRA4, MEIS2, NDUFB5, XPA | 2.62E-04 |
| (Lopez Rangel and Lewis,. (2006) Clin Genet 69, 21-2) | neurodegeneration of CA3 neurons | GRIA2 | 2.23E-03 |
| | congenital hydrocephalus of mice | E2F5 | 4.46E-03 |
| | neurological disorder | E2F5, ELMO1, EMX2, FOXN3, GABRA4, GRIA2, MEIS2, NDUFB5, TIAM1, TOP2A, XPA | 6.24E-03 |
| | hypoplasia of cerebellum | XPA | 8.91E-03 |
| | Encephalopathy | E2F5, EMX2 | 1.61E-02 |
| | major depression | GABRA4, GRIA2 | 2.01E-02 |
| | epilepsy, seizures | GABRA4, GRIA2 | 2.09E-02 |
| | cell death of pyramidal neurons | GRIA2 | 2.87E-02 |
| | Dyskinesia | GRIA2 | 2.87E-02 |
| | progressive motor neuropathy | GABRA4, GRIA2, TOP2A | 2.95E-02 |
| **Nervous System Development and Function** | | | |
| (Beaudet, A. L. (2007). Nature Medicine 13, 534-536) | calcium permeability of neurons, innervation of Purkinje cells | GRIA2 | 2.23E-03 |
| | secretion of cerebrospinal fluid | E2F5 | 2.23E-03 |
| | dedepression and depotentiation of CA1 neuron | GRIA2 | 4.46E-03 |
| | formation of cerebellar folia, Size of dendrites, neurogenesis | XPA | 4.46E-03 |
| | size of hippocampus | EMX2 | 6.69E-03 |
| | formation of neurite branches | TIAM1 | 8.91E-03 |
| | size of cerebral cortex | EMX2 | 8.91E-03 |
| | activation of synapse, LTD of CA1 neurons, Purkinje cells, and mossy fibers | GRIA2 | 1.33E-02 |
| | development of dentate gyrus | EMX2 | 11.77E-02 |
| | synaptic transmission of pyramidal neurons | GRIA2 | 1.77E-02 |
| | Memory | GRIA2, ITGA5 | 2.62E-02 |
| | migration of cortical neurons and Schwann cells | TIAM1 | 3.52E-02 |
| | morphology of neurons | TIAM1 | 4.38E-02 |
| **Cellular Assembly and Organization** | retraction of glial cell projections | GRIA2 | 2.23E-03 |
| (Schanen, (2006) Hum Mol Genet 15 Spec No 2, | structural integrity of nucleosomes | TOP2A | 4.46E-03 |
| | retraction of plasma membrane projections | GRIA2, TIAM1 | 8.93E-03 |
| | size of growth cone | TIAM1 | 1.99E-02 |
| | formation of actin stress fibers | ITGA5, TIAM1 | 4.83E-02 |
| **Embryonic Development** (Folstein and Rutter, (1977) J Child Psychol Psychiatry 18, 297-321) | degeneration of notochord | ITGA5 | 2.23E-03 |
| | formation of gonadal ridge neurogenesis of embryonic | CBX2 | 2.23E-03 |
| | tissue cell death of neural crest | XPA | 6.69E-03 |
| | cells | ITGA5 | 1.77E-02 |
| | cleavage of embryo | TOP2A | 1.77E-02 |

**Table 6: Four high level functions associated with genes differentially methylated between monozygotic twins and unaffected siblings and identified by Cpg island microarray. P values represent significance by Fisher Exact Test and indicates the probability that the indicated function is not associated with the dataset of differentially methylated genes (Ingenuity Pathway Analysis software).**

| **Category (# genes)** | **Function Annotation** | **Molecules** | **p-value** |
|---|---|---|---|
| **Gene Expression** | Transcription | ATF1, CBX2, CCNB1, CHAF1A, FOXN3, FRAT1, ILK, IRF1, ITGA5, MED7 (includes EG:9443), MEIS1, MLLT1, MTPN, MYF6, NFIA, NR2C1, PAX7, PWP1, RBM39, RORA, RREB1, RRN3, SET, TFDP2, TG, TGFBR2, VDR, ZNF234 | 9.02E-08 |
| (Nagarajan et al. (2006) Epigenetics 1,172-182) | Transactivation | ATF1, FRAT1, IRF1, NFIA, PAX7, RBM39, RORA, TFDP2, TGFBR2, VDR | 1.92E-03 |
| | activation of TGF beta response element | ITGA5, TGFBR2 | 4.93E-03 |
| | transactivation of response element | VDR | 5.29E-03 |
| | transactivation of RORE binding site | RORA | 5.29E-03 |
| | expression of DNA endogenous promoter | TG, VDR | 7.06E-03 |
| | activation of Atf-1 binding site | ATF1 | 1.05E-02 |
| | activation of NFI binding site | NFIA | 1.05E-02 |
| | binding of TREpal motif | VDR | 1.05E-02 |
| | transcription of beta catenin response element | FRAT1 | 1.05E-02 |
| | activation of HAF1/HAF1a binding site | IRF1 | 1.58E-02 |
| | activation of IRF1/IRF2 binding site | IRF1 | 1.58E-02 |
| | binding ofNFAT response element | VDR | 1.58E-02 |
| Cell Death (Klose and Bird (2006) Trends in Biochemical Sciences 31, 89-97) | | ATF1, BAK1, CCNB1, CDC73, FAS, FRAT1, FXR1, GRIA2, GULP1, HPN, ILK, IRF1, ITGA5, MCF2L, MED7, MTPN, PAX7, PLA2G6, RRN3, | |
| | Apoptosis | TGFBR2, TIAM1, VDR | 4.30E-03 |
| Cell Death | delay in cell death of cortical neurons | BAK1 | 5.29E-03 |
| Cell Death | killing of oligodendrocytes | MBP | 5.29E-03 |
| Cell Death | cell death of microglia | FAS, IRF1 | 5.43E-03 |
| | | ATF1, FAS, ITGA5 ATF1, BAK1, CCNB1, CDC73, FAS, FRAT1, FXR1, GRIA2, GULP1, HPN, ILK, IRF1, ITGA5, MBP, MCF2L, MED7, MEIS1, MTPN, PAX7, PLA2G6, RRN3 | 6.02E-03 |
| Cell Death | apoptosis of embryonic cells | | 2.56E-02 |
| Cell Death | cell death | TGFBR2, TIAM1, VDR | 6.61E-03 |
| Cell Death | apoptosis of embryonic stem cells | FAS, ITGA5 | 7.65E-03 |
| Cell Death | cell death of brain cells | BAK1, FAS, FRAT1, GRIA2 | 2.54E-02 |
| Cell Death | cell death of cortical neurons | BAK1, FAS, GRIA2 | |
| **Neurological Disease** (Kaminsky et al. (2006) Ann Med 38, 530-44) | delay in cell death of cortical neurons | BAK1 | 5.29E-03 |
| | killing of oligodendrocytes | MBP | 5.29E-03 |
| | neurodegeneration of CA3 neurons | 3RIA2 | 5.29E-03 |
| | cell death of microglia | FAS, IRF1 | 5.43E-03 |
| | disease of brain cells | GRIA2, TG | 1.02E-02 |
| | depletion of neurons | DLD | 1.05E-02 |
| | gracile axonal dystrophy | PLA2G6 | 1.05E-02 |
| | mobility of brain cancer cell lines | ITGA5 | 1.05E-02 |
| | multiple sclerosis of humans | MBP | 1.58E-02 |
| | Neurodegeneration | GRIA2, PLA2G6, TGFBR2 | 1.76E-02 |
| | apoptosis of granule cells | FAS, FRAT1 | |
| | cell death of brain cells, cortical neurons | BAK1, FAS, FRAT1, GRIA2 | 1.89E-02 2.54E-02 |
| | apoptosis of neural precursor cells | FAS | 2.62E-02 |
| | hypomyelination of axons | MBP | 2.62E-02 |
| **Nervous System Development and Function** (Beaudet, A. L. (2007) Nature Medicine 13, 534-536) | calcium permeability of neurons, innervation of Purkinje cells, | | |
| | retraction of glial cell projections | GRIA2 | 5.29E-03 |
| | formation of hippocampal commissure, presence/development of corpus | | |
| | callosum, formation of sling cells | NFIA | 5.29E-03 |
| | dedepression and depotentiation of CA1 neurons | GRIA2 | 1.05E-02 |
| | organization of axons, organization of central nervous system | MBP | 1.05E-02 |
| | quantity of satellite cells | PAX7 | 1.05E-02 |
| | dilation of cerebral ventricles | NFIA | 1.58E-02 |
| | function of brain | RORA | 2.10E-02 |
| | formation of neurite branches | TIAM1 | 2.10E-02 |
| | migration of neuroglia | NFIA, TIAM1 | 2.26E-02 |

In the following preferred embodiments of the invention are described.
1. An array comprising a plurality of different oligonucleotides with specificity for at least one of the genes contained in Table 1, Table 2, or Table 3, or a combination thereof.
2. The array of embodiment 1, wherein members of said plurality have specificity for at least one of the genes selected from retinoic acid receptor related orphan receptor (RORA) and B-cell CLL/Lymphoma 2 (BCL-2) genes.
3. The array of embodiments 1 or 2, comprising oligonucleotides having specificity for differentially methylated regions of the genes or for differentially methylated CpG islands of promoter(s) of the gene(s).
4. A method of screening a subject for a neurological disease or disorder comprising the steps of:
   (a) isolating nucleic acid from a subject;
   (b) measuring a level of DNA methylation of at least one gene in Table 1, Table 2, Table 3 or a combination thereof in the isolated nucleic acid of (a); and
   (c) diagnosing the subject as being at risk for or affected by a neurological disease or disorder if there is a statistically significant difference in the level of DNA methylation of at least one gene listed in Table 1, Table 2, Table 3 of (a) compared to the level of DNA methylation of the same genes from a sample from a healthy individual not having the neurological disease or disorder.
5. The method of Embodiment 4, wherein the neurological disease is an autism spectrum disorder.
6. The method of embodiment 5 wherein the autistic spectrum disorder is selected from the group consisting of pervasive developmental disorder-not otherwise specified (PDD-NOS), atypical autism, and Asperger's Disorder.
7. The method of Embodiment 4, wherein the at least one gene is selected from genes involved in nervous system development, axon guidance, synaptic transmission or plasticity, myelination, long-term potentiation, neuron toxicity, Purkinje cell differentiation, cerebellar development, embryonic development, regulation of actin networks, digestion, inflammation, oxidative stress, epilepsy, apoptosis, morphogenesis, cell survival, differentiation, the unfolded protein response, Type [Pi] diabetes and insulin signaling, digestion, liver toxicity (hepatic stellate cell activation, fibrosis, and cholestasis), endocrine function, circadian rhythm, cholesterol metabolism and the steroidogenesis pathway, or a combination thereof.
8. The method of Embodiment 4, wherein the healthy individual is a non-phenotypic discordant twin, sibling of the subject, or unrelated subject.
9. The method of Embodiment 4, wherein the neurological disease or disorder is an autism spectrum disorder characterized by (a) a lower severity score across all ADIR items, (b) an intermediate severity score across all ADIR items, (c) a higher severity scores on spoken language items on the ADIR, (d) a higher frequency of savant skills, (e) a severe language impairment, or a combination thereof.
10. The method of Embodiment 4, wherein DNA methylation is quantified with an assay comprising (a) bisulfite-sequencing, (b) methylation-specific PCR (MSP), (c) large scale CpG island microarray analysis using methylation-enriched and -unenriched samples, (d) promoter analysis using methylation-enriched and -unenriched samples, (e) combined bisulfite and restriction analysis (COBRA), (f) pyrosequncing, (g) Methyl-Light (Premier Biosoft International) or Methyl- Profiler (SABiosciences) methylation analysis, (h) Sequenom mass analyzer, (i) bisulfite treatment of DNA combined with selected amplification of the specific promoter regions of differentially methylated genes with the addition of a T7 promoter tag, followed by T7- mediated transcription and RNase TI cleavage of the transcript with methylation sites determined by MALDI-TOF mass spectrometry analysis in, or a combination thereof.
11. A method for determining a differential DNA methylation profile for at least one autism spectrum disorder,'comprising
   (a) obtaining samples of control and experimental DNA, wherein the experimental DNA is generated from a sample isolated from a subject afflicted with or suspected of being afflicted with the at least one autism spectrum disorder and the control DNA is generated from a sample isolated from a healthy individual;
   (b) applying the samples of (a) to a microassay comprising a plurality of different oligonucleotides having specificity for differentially-methylated genes associated with the at least one autism spectrum disorder to allow hybridization between the oligonucleotides and the control DNA and experimental DNA; and
   (c) identifying the oligonucleotides on the microarray that display differential hybridization to the experimental DNA relative to the control DNA, thereby determining a differential DNA methylation profile for the at least one autism spectrum disorder.
12. The method according to Embodiment 11, wherein the plurality of different oligonucleotides is specific for at least one differentially DNA-methylated gene in Table 1, Table 2, Table 3 or a combination thereof.
13. The method of Embodiment 12, wherein the at least one autism spectrum disorder is selected from autistic disorder, pervasive developmental disorder-not otherwise specified (PDD-NOS), atypical autism, and Asperger's Disorder.
14. A method for distinguishing between different phenotypes of an autism spectrum disorder comprising severely language impaired (L), mildly affected (M), or "savants" (S) comprising
   (a) obtaining samples of control and experimental DNA, wherein the experimental DNA is generated from a sample isolated from a subject suspected of being afflicted with at least one phenotype selected from the severely language impaired (L), mildly affected (M), or "savants" (S);
   (b) applying the samples of (a) to a microarray comprising a plurality of different oligonucleotides having specificity for differentially DNA-methylated genes associated with the at least one phenotype to allow hybridization between the oligonucleotides and the control and experimental DNA; and
   (c) identifying the oligonucleotides on the microarray which display differential hybridization to the experimental DNA relative to the control DNA thereby determining a DNA methylation profile for distinguishing among the different phenotypes of autism spectrum disorder.
15. The method according to Embodiment 14, wherein the plurality of different oligonucleotides is specific for at least one differentially methylated gene in Table 1, Table 2, Table 3 or a combination thereof.
16. The method of Embodiment 14, wherein the at least one autism spectrum disorder is selected from autistic disorder, pervasive developmental disorder-not otherwise specified (PDD-NOS), a typical autism, or Asperger's Disorder.
17. The method of embodiments 14 wherein the control DNA is from a healthy individual not having an autism spectrum disorder and wherein the difference in the DNA methylation profiles distinguishes the different phenotypes of autism spectrum disorder.
18. A method of assessing the efficacy of a treatment in a subject having at least one autism spectrum disorder comprising,
   (a) determining a level of DNA methylation of at least one gene in Table 1, Table 2, Table 3 or a combination thereof in a sample of a test subject, or in a plurality of samples of a selected tissue type of a test subject to generate a methylation profile for the sample;
   (b) determining a degree of similarity between (i) the level of DNA methylation of the profile of (a); and (ii) the level of DNA methylation of at least one differentially DNA- methylated gene in Table 1, Table 2, Table 3 or a combination thereof in a control profile, wherein the control profile is a methylation profile specific for the genes set out in listed in Table 1, Table 2, Table 3, or a combination thereof, produced by a therapy which has been shown to be efficacious in treatment of the at least one autism spectrum disorder; wherein a high degree of similarity of the differential methylation profile data is indicative that the treatment is effective.
19. The method of embodiment 18 wherein the high degree of similarity is log 2 ratio less than +/-0.3.
20. A method of assessing the efficacy of a treatment in a subject having at least one autism spectrum disorder comprising
   (a) determining DNA methylation profile data specific for at least one differentially DNA- methylated gene contained in Table 1, Table 2, Table 3 or a combination thereof, in a plurality of subject samples of a selected tissue type, wherein the subject has received a treatment, by measuring the level of DNA methylation of the at least one differentially DNA-methylated gene;
   (b) determining a degree of similarity between (i) the DNA methylation profile data in the subject samples of (a); and (ii) DNA methylation profile data specific for the genes contained in Table 1, Table 2, Table 3 or a combination thereof, produced by a therapy which has been shown to be efficacious in treatment of the at least one autism spectrum disorder; wherein a high degree of similarity of the DNA methylation profile data of (i) and (ii) is indicative that the treatment is effective.
21. The method of embodiment 20 wherein the high degree of similarity is a log2 ratio less than +/-0.3.
22. A method of identifying a DNA methylation profile responsive to administration of a therapeutic treatment to a subject with at least one autism spectrum disorder comprising,
   (a) obtaining samples of control and experimental nucleic acid, wherein the experimental nucleic acid is generated from a sample isolated from a test subject (i) prior to receiving a therapeutic treatment and (ii) after receiving the therapeutic treatment;
   (b) preparing one or more microarrays comprising a plurality of different oligonucleotides, wherein the oligonucleotides are specific for genes associated with an autism spectrum disorder;
   (c) applying the prepared samples to the one or more microarrays to allow hybridization between the oligonucleotides and the control and experimental nucleic acids;
   (d) identifying the oligonucleotides on the microarray that display differential hybridization to the experimental nucleic acid relative to the control nucleic acid to generate a differential DNA methylation profile for the samples, and
   (e) comparing the profiles of the samples, wherein increase in the similarity of the differential DNA-methylation of the test sample of (ii) and the control as compared to the similarity of the differential DNA-methylation profile of (i) and control identifies a differential DNA methylation profile responsive to the administration of the therapeutic treatment to the subject with at least one autism spectrum disorder.
23. The method of Embodiment 22 wherein the control sample is taken from the subject prior to the therapeutic treatment or from a healthy subject not having an autism spectrum disorder or a subject have a less severe form of autism spectrum disorder.
24. The method of Embodiment 21, wherein the plurality of different oligonucleotides is specific for at least one differentially DNA-methylated gene set out in Table 1, Table 2, Table 3 or a combination thereof.
25. The method of Embodiment 22, wherein the at least one autism spectrum disorder neurological condition comprises autistic disorder, pervasive developmental disorder-not otherwise specified (PDD-NOS), a typical autism, Asperger's Disorder, or a combination thereof.
26. A method for conducting drug discovery comprising,
   (a) generating a database of DNA methylation profile data representative of the genetic expression response of at least one selected neuronal tissue type from a subject that was subjected to at least one of a plurality of behavioral therapies and that has undergone a selected physiological change since commencement of the behavioral therapy;
   (b) administering a small molecule test agent(s) to untreated subjects to obtain DNA methylation profile data associated with administration of the agent(s) and comparing the obtained data with the one or more DNA methylation profiles of (a);
   (c) selecting the test agent(s) that induce DNA methylation profiles similar to the DNA methylation profiles obtained by administration of the behavioral therapy;
   (d) conducting therapeutic profiling of the selected test agent(s), or analogs thereof, for efficacy and toxicity in subjects; and
   (e) identifying a pharmaceutical preparation including one or more agent(s) identified in step (d) as having an acceptable therapeutic and/or toxicity profile.
27. The method of Embodiment 26, wherein the behavioral therapy comprises applied behavior analysis (ABA) intervention methods, dietary changes, exercise, massage therapy, group therapy, talk therapy, play therapy, conditioning, or alternative therapies such as sensory integration and auditory integration therapies.
28. The method of Embodiment 26, wherein the selected physiological change includes one or more improvements in social interaction, language abilities, restricted interests, repetitive behaviors, sleep disorders, seizures, gastrointestinal, hepatic, and mitochondrial function, neural inflammation, or a combination thereof.
29. The method of Embodiment 26, wherein, prior to administration of behavioral therapy, the subject showed at least one symptom of a psychological or physiological abnormality.
30. The method of Embodiment 26, wherein the neuronal tissue type is selected from the group consisting of olfactory bulb cells, cerebrospinal fluid, hypothalamus, amygdala, pituitary, nervous system, brainstem, cerebellum, cortex, frontal cortex, hippocampus, striatum, and thalamus
31. The method of any one of Embodiments 4, 11, 14, 18, 20, 23, or 26, wherein the gene comprises any one or more of RORA or BCL-2.
32. The method of any one of Embodiments 4, 11, 14, 18, 20, 23, or 26, wherein the sample is a blood sample.
33. The method of any one of Embodiments 4, 11, 14, 18, 20, 23, or 26, wherein the sample comprises lymphocytes.
34. The method of any one of Embodiments 4, 11, 14, 18, 20, 23, or 26, wherein the sample is an LCL derived from the subject.
35. A kit for identifying a compound for treating at least one autism spectrum disorder comprising (a) a database having information stored therein of one or more DNA methylation profiles specific for the genes set out in Table 1, Table 2, Table 3 or a combination thereof, of subjects that have been subjected to at least one of a plurality of selected autism spectrum disorder neurological therapies and wherein the subject has undergone a desired physiological change; and (b) a computer program for comparing DNA methylation profile data obtained from assays wherein a test compound is administered to a subject with the database of stored DNA methylation profiles and providing information representative of a measure of similarity between the DNA methylation profile data and one or more stored DNA methylation profiles.
36. A computer-readable medium on which is encoded programming code for analyzing autism spectrum disorder from a plurality of data points comprising a profile of differentially expressed DNA methylation genes, wherein said differential DNA methylation profile is specific for at least one differentially DNA methylated gene set out in Table 1, Table 2, Table 3, or a combination thereof.

## Claims

1. An in vitro method of aiding in assessing a subject's risk for having an autism spectrum disorder comprising steps of:
(a) obtaining nucleic acid sample from a subject;
(b) measuring a level of DNA methylation of retinoic acid related orphan receptor (RORA) in the nucleic acid sample of (a); and
(c) determining whether there is a difference in the level of DNA methylation of RORA of (b) compared to the level of DNA methylation of RORA in a sample from an individual not having autism spectrum disorder, wherein a difference is indicative of a subject being at increased risk of having an autism spectrum disorder.

2. An in vitro method of aiding in assessing a subject's risk for having an autism spectrum disorder comprising steps of:
(a) obtaining nucleic acid sample from a subject;
(b) measuring a level of DNA methylation of B-cell CLL/Lymphoma 2 (BCL-2) in the nucleic acid sample of (a); and
(c) determining whether there is a difference in the level of DNA methylation of BCL-2 of (b) compared to the level of DNA methylation of BCL-2 in a sample from an individual not having autism spectrum disorder, wherein a difference is indicative of a subject being at increased risk of having an autism spectrum disorder.

3. The method of any of claims 1 or 2 wherein the autism spectrum disorder is **characterized by** severe language impairment.

4. The method of any of claims 1-3 wherein the nucleic acid sample is obtained from blood cells.

5. The method of claim 4 wherein the blood cells comprise lymphocytes.

6. The method of claims 1-3 wherein the nucleic acid sample is obtained from cells of a lymphoblastoid cell line derived from the subject.


SEQUENCE LISTING

<110> THE GEORGE WASHINGTON UNIVERSITY

<120> COMPOSITIONS AND METHODS FOR IDENTIFYING AUTISM SPECTRUM
      DISORDERS

<130> 031778.63411WO

<140> PCT/US2011/031450
<141> 2011-04-06

<150> 61/321,172
<151> 2010-04-06

<160> 15

<170> PatentIn version 3.5

<210> 1
<211> 25
<212> DNA
<213> Artificial Sequence

<220>
<223> Description of Artificial Sequence: Synthetic
      oligo-long primer

<400> 1
gcggtgaccc gggagatctg aattc                                          25


<210> 2
<211> 18
<212> DNA
<213> Artificial Sequence

<220>
<223> Description of Artificial Sequence: Synthetic
      oligo-MseI primer

<400> 2
tagaattcag atctcccg                                                  18


<210> 3
<211> 20
<212> DNA
<213> Artificial Sequence

<220>
<223> Description of Artificial Sequence: Synthetic
      BCL2 forward primer

<400> 3
tattgtggct gcacttgctc                                                20


<210> 4
<211> 20
<212> DNA
<213> Artificial Sequence

<220>
<223> Description of Artificial Sequence: Synthetic
      BCL2 reverse primer

<400> 4
tgttgcccaa ctgcaaaata                                                   20


<210> 5
<211> 20
<212> DNA
<213> Artificial Sequence

<220>
<223> Description of Artificial Sequence: Synthetic
      RORA forward primer

<400> 5
acaccagcat caggcttctt                                                   20


<210> 6
<211> 20
<212> DNA
<213> Artificial Sequence

<220>
<223> Description of Artificial Sequence: Synthetic
      RORA reverse primer

<400> 6
ggtctgccac gttatctgct                                                   20


<210> 7
<211> 22
<212> DNA
<213> Artificial Sequence

<220>
<223> Description of Artificial Sequence: Synthetic
      BCL2 nested PCR - outer forward primer

<400> 7
ggtggtttag aggagggttt tt                                                22


<210> 8
<211> 26
<212> DNA
<213> Artificial Sequence

<220>
<223> Description of Artificial Sequence: Synthetic
      BCL2 nested PCR - outer reverse primer

<400> 8
acaaaaatcc tcttctaatt aaactc                                            26


<210> 9
<211> 23
<212> DNA

<213> Artificial Sequence

<220>
<223> Description of Artificial Sequence: Synthetic
      BCL2 nested PCR - inner forward primer

<400> 9
gagaatgaag taagaggata ggt                                              23


<210> 10
<211> 23
<212> DNA
<213> Artificial Sequence

<220>
<223> Description of Artificial Sequence: Synthetic
      BCL2 nested PCR - inner reverse primer

<400> 10
caccctttct cctcctccta atc                                              23


<210> 11
<211> 22
<212> DNA
<213> Artificial Sequence

<220>
<223> Description of Artificial Sequence: Synthetic
      RORA MSP: methylated forward primer

<400> 11
ggttggagaa gttttcgtta gc                                               22


<210> 12
<211> 22
<212> DNA
<213> Artificial Sequence

<220>
<223> Description of Artificial Sequence: Synthetic
      RORA MSP: methylated reverse primer

<400> 12
gacgaacgaa caaacaaaaa cg                                               22


<210> 13
<211> 24
<212> DNA
<213> Artificial Sequence

<220>
<223> Description of Artificial Sequence: Synthetic
      RORA MSP: unmethylated forward primer

<400> 13
ttggttggag aagtttttgt tagt                                             24


<210> 14

<211> 24
<212> DNA
<213> Artificial Sequence

<220>
<223> Description of Artificial Sequence: Synthetic
      RORA MSP: unmethylated reverse primer

<400> 14
caaacaaaaa cacaaaaaaa caca                                              24


<210> 15
<211> 6
<212> PRT
<213> Artificial Sequence

<220>
<223> Description of Artificial Sequence: Synthetic
      6xHis tag

<400> 15
His His His His His His
1               5